# EUROPEAN PATENT APPLICATION

(11) **EP 3 594 208 A1**
(43) Date of publication of application: **15.01.2020**
(21) Application number: 18757050.2
(22) Date of filing: 16.02.2018
(51) Int. Cl.: C07D 307/91, C07D 407/04, C09K 19/12, C09K 19/18, C09K 19/20, C09K 19/30, C09K 19/32, C09K 19/34, C09K 19/42, G02F 1/13

(54) **COMPOUND HAVING DIBENZOFURAN RING, LIQUID CRYSTAL COMPOSITION, AND LIQUID CRYSTAL DISPLAY ELEMENT**

(30) Priority: 27.02.2017 JP 2017034600
(71) Applicant: JNC Corporation, Chiyoda-ku Tokyo 100-8105 (JP); JNC Petrochemical Corporation, Tokyo 100-0004 (JP)
(72) Inventor: TAKATA Akihiro, Ichihara-shi Chiba 290-8551 (JP)
(74) Representative: Becker, Eberhard
(86) International application number: PCT/JP2018/005506
(87) International publication number: WO 2018/155340

(57) **Abstract**

The purpose of the present invention is to provide a liquid crystalline compound; a liquid crystal composition containing the compound; and a liquid crystal display element including the composition, the liquid crystalline compound satisfying at least one physical property among high stability against heat and light, high transparency, low minimum temperature of the liquid crystal phase, low viscosity, proper optical anisotropy, negatively large dielectric anisotropy, proper elastic constant, good compatibility with other liquid crystalline compounds at room temperature and low temperature, and the like. Provided is a compound represented by formula (1), or the like. In the formula, R¹ and R² are hydrogen, C1-10 alkyl, C2-9 alkoxy, or the like; ring A¹ is 1,3-cyclopentylene or the like; Y¹ and Y² are hydrogen, fluorine, or the like; Z¹ and Z² are a single bond, C1-6 alkylene, or the like; ring N¹ is 1,4-cyclohexylene, 1,4-phenylene, or the like; and n is 0, 1, or 2.

## Description

### [Technical Field]

The present invention relates to a liquid crystalline compound, a liquid crystal composition, and a liquid crystal display element, and more specifically, to a liquid crystalline compound including a dibenzofuran ring and having negative dielectric anisotropy, a liquid crystal composition including the same, and a liquid crystal display element including the composition.

### [Background Art]

Based on the operation mode of liquid crystal molecules, liquid crystal display elements are classified into a phase change (PC) mode, a twisted nematic (TN) mode, a super twisted nematic (STN) mode, an electrically controlled birefringence (ECB) mode, an optically compensated bend (OCB) mode, an in-plane switching (IPS) mode, a vertical alignment (VA) mode, a fringe field switching (FFS) mode, a field-induced photo-reactive alignment (FPA) mode and the like. Elements are classified into a passive matrix (PM) method and an active matrix (AM) method based on the drive method. PM methods are classified into a static method and a multiplex method, and AM methods are classified into a thin film transistor (TFT) method and a metal insulator metal (MIM) method.

A liquid crystal composition is included in the element. Physical properties of the composition relate to characteristics of the element. Examples of physical properties of the composition include stability with respect to heat and light, a temperature range of a nematic phase, a viscosity, optical anisotropy, dielectric anisotropy, a specific resistance, and an elastic constant. The composition is prepared by mixing many liquid crystalline compounds. Physical properties necessary for the compound include high stability with respect to water, air, heat, and a lighting environment, a wide temperature range of a liquid crystal phase, low viscosity, appropriate optical anisotropy, large dielectric anisotropy, an appropriate elastic constant, and favorable compatibility with other liquid crystalline compounds. A compound having a high upper limit temperature of a nematic phase is preferable. A compound having a low lower limit temperature in a liquid crystal phase such as a nematic phase and a smectic phase is preferable. A compound having a low viscosity contributes to a short response time of the element. An appropriate value of optical anisotropy differs according to a mode of the element. A compound having positive or large negative dielectric anisotropy is preferable in order to drive the element at a low voltage. In order to prepare a composition, a compound having favorable compatibility with other liquid crystalline compounds is preferable. A compound having favorable compatibility at a low temperature is preferable because then the element may be used at a temperature equal to or lower than the freezing point.

Many liquid crystalline compounds having large dielectric anisotropy have been synthesized so far. The development of new liquid crystalline compounds is still ongoing. This is because novel compounds are expected to have favorable physical properties which are not obtained in compounds of the related art. This is because novel compounds may provide an appropriate balance between at least two physical properties in the composition.

The following compound is disclosed in Table 1-2 in paragraph [0079] in Patent Literature 1 (Japanese Unexamined Patent Application Publication No. 2015-174864). While the compound has large dielectric anisotropy, the compatibility at a low temperature is not sufficient.

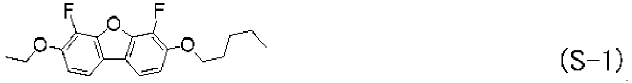

### [Citation List]

### [Patent Literature]

[Patent Literature 1]
   Published Japanese Translation No. 2004-529867 of the PCT International Publication
[Patent Literature 2]
   Japanese Unexamined Patent Application Publication No. 2015-174864

### [Summary of Invention]

### [Technical Problem]

A first objective is to provide a liquid crystalline compound that has at least one of physical properties such as high stability with respect to heat and light, a high clearing point (or a high upper limit temperature of a nematic phase), a low lower limit temperature of a liquid crystal phase, a low viscosity, appropriate optical anisotropy, large negative dielectric anisotropy, an appropriate elastic constant, and favorable compatibility with other liquid crystalline compounds. This objective is to provide a compound having larger dielectric anisotropy and more favorable compatibility with other liquid crystalline compounds than similar compounds. A second objective is to provide a liquid crystal composition which includes the compound and has at least one of physical properties such as high stability with respect to heat and light, a high upper limit temperature of a nematic phase, a low lower limit temperature of a nematic phase, a low viscosity, appropriate optical anisotropy, large negative dielectric anisotropy, a high specific resistance, and an appropriate elastic constant. This objective is to provide a liquid crystal composition having an appropriate balance regarding at least two physical properties. A third objective is to provide a liquid crystal display element which includes the composition and has a wide temperature range in which the element can be used, a short response time, a high voltage holding ratio, a low threshold voltage, a large contrast ratio, a low flicker rate, and a prolonged lifespan.

### [Solution to Problem]

The present invention relates to a compound represented by Formula (1), a liquid crystal composition including the compound, and a liquid crystal display element including the composition.

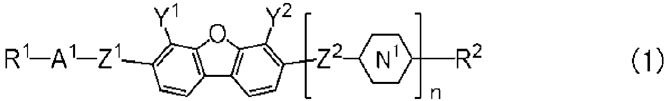

In Formula (1),
R¹ and R² independently represent a hydrogen atom or an alkyl group having 1 to 10 carbon atoms, and in the alkyl group, at least one -CH₂- is optionally substituted with -O-, -S-, -CO-, or -SiH₂-, at least one -CH₂CH₂- is optionally substituted with -CH=CH- or -C≡C-, and in these groups, at least one hydrogen atom is optionally substituted with a fluorine atom;
the ring A¹ represents 1,2-cyclopropylene, 1,3-cyclobutylene, 1,3-cyclopentylene, 1,3-cyclopentenylene, or 1,4-cyclopentenylene, and in these groups, at least one -CH₂- is optionally substituted with -O-;
the ring N¹ represents 1,4-cyclohexylene, decahydronaphthalene-2,6-diyl, or 1,4-phenylene, and in these groups, at least one -CH₂- is optionally substituted with -O-, -S-, -CO-, or -SiH₂-, at least one -CH₂CH₂- is optionally substituted with -CH=CH- or -CH=N-, and in these divalent groups, at least one hydrogen atom is optionally substituted with a fluorine atom, a chlorine atom, -C≡N, -CF₃, -CHF₂, -CH₂F, -OCF₃, -OCHF₂, or -OCH₂F;
Y¹ and Y² independently represent a hydrogen atom, a fluorine atom, a chlorine atom, -CF₃, -CHF₂, -CH₂F, -OCF₃, -OCHF₂, or -OCH₂F;
n is 0, 1 or 2;
Z¹ and Z² represent a single bond or an alkylene group having 1 to 6 carbon atoms, and in the alkylene group, at least one -CH₂- is optionally substituted with -O-, -S-, or -CO-, one or two -CH₂CH₂-'s are optionally substituted with -CH=CH- or -C≡C-, and in these divalent groups, at least one hydrogen atom is optionally substituted with a fluorine atom.

### [Advantageous Effects of Invention]

A first effect is to provide a liquid crystalline compound that has at least one of physical properties such as high stability with respect to heat and light, a high clearing point (or a high upper limit temperature of a nematic phase), a low lower limit temperature of a liquid crystal phase, a low viscosity, appropriate optical anisotropy, large negative dielectric anisotropy, an appropriate elastic constant, and favorable compatibility with other liquid crystalline compounds. There is provided a compound having more favorable compatibility with other liquid crystalline compounds than similar compounds (Comparative Example 1). A second effect is to provide a liquid crystal composition which includes the compound and has at least one of physical properties such as high stability with respect to heat and light, a high upper limit temperature of a nematic phase, a low lower limit temperature of a nematic phase, a low viscosity, appropriate optical anisotropy, large negative dielectric anisotropy, a high specific resistance, and an appropriate elastic constant This effect is to provide a liquid crystal composition having an appropriate balance regarding at least two physical properties. A third effect is to provide a liquid crystal display element which includes the composition and has a wide temperature range in which the element can be used, a short response time, a high voltage holding ratio, a low threshold voltage, a large contrast ratio, a low flicker rate, and a prolonged lifespan.

### [Description of Embodiments]

The terms used herein are used as follows. The terms "liquid crystalline compound," "liquid crystal composition," and "liquid crystal display element" may be abbreviated as a "compound," a "composition," and an "element." A "liquid crystalline compound" generally refers to a compound having a liquid crystal phase such as a nematic phase or a smectic phase and a compound which does not have a liquid crystal phase and is added to adjust physical properties of a composition such as an upper limit temperature, a lower limit temperature, a viscosity, and dielectric anisotropy. The compound has a rod-like molecular structure. A "liquid crystal display element" generally refers to a liquid crystal display panel and a liquid crystal display module. A "polymerizable compound" is a compound which is added to form a polymer in the composition.

A liquid crystal composition is prepared by mixing a plurality of liquid crystalline compounds. Additives are added to the composition in order to additionally adjust physical properties. Additives such as a polymerizable compound, a polymerization initiator, a polymerization inhibitor, an optically active compound, an antioxidant, a UV absorber, a light stabilizer, a heat stabilizer, a dye, and an antifoaming agent are added as necessary. A liquid crystalline compound and additives are mixed in such a procedure. Even when additives are added, a proportion (content) of a liquid crystalline compound is expressed as a weight percentage (weight%) based on a weight of a liquid crystal composition including no additives. A proportion (addition amount) of additives is expressed as a weight percentage (weight%) based on a weight of a liquid crystal composition including no additives. Parts per million (ppm) by weight may be used. Proportions of a polymerization initiator and a polymerization inhibitor are exceptionally expressed based on a weight of the polymerizable compound.

The "clearing point" is a transition temperature of a liquid crystal phase-isotropic phase in a liquid crystalline compound. A "lower limit temperature of a liquid crystal phase" is a transition temperature of a solid-liquid crystal phase (such as a smectic phase and a nematic phase) in a liquid crystalline compound. An "upper limit temperature of a nematic phase" is a transition temperature of a nematic phase-isotropic phase in a mixture of a liquid crystalline compound and a mother liquid crystal or a liquid crystal composition, and may be abbreviated as an "upper limit temperature." A "lower limit temperature of a nematic phase" may be abbreviated as a "lower limit temperature." The expression "increasing dielectric anisotropy" means that a value increases positively when a composition has positive dielectric anisotropy and means that a value increases negatively when a composition has negative dielectric anisotropy. The expression "a voltage holding ratio is high" means that an element has a high voltage holding ratio not only at room temperature in an initial stage but also at a temperature close to an upper limit temperature, and even after it is used for a long time, it has a high voltage holding ratio not only at room temperature but also at a temperature close to an upper limit temperature. Characteristics of compositions and elements may be examined before and after an aging test (including an accelerated deterioration test).

The compound represented by Formula (1) may be abbreviated as Compound (1). At least one compound selected from the group consisting of compounds represented by Formula (1) may be abbreviated as Compound (1). "Compound (1)" refers to one compound represented by Formula (1), a mixture of two compounds, or a mixture of three or more compounds. These rules also apply to compounds represented by other formulae. In Formulae (1) to (15) and sub-formulae thereof, symbols such as A¹, B¹, and C¹ surrounded by a hexagon correspond to rings such as a ring A¹, a ring B¹, and a ring C¹. A hexagon refers to a six-membered ring such as cyclohexane or benzene. A hexagon may refer to a condensed ring such as naphthalene or a cross-linked ring such as adamantane.

In a chemical formula of a component compound, the symbol of a terminal group R¹¹ is used for a plurality of compounds. In these compounds, two groups represented by any two R¹¹'s may be the same as or different from each other. For example, there is a case in which R¹¹ of Compound (2) is an ethyl group and R¹¹ of Compound (3) is an ethyl group. There is also a case in which R¹¹ of Compound (2) is an ethyl group and R¹¹ of Compound (3) is a propyl group. These rules also apply to symbols such as R¹², R¹³, and Z¹¹. In Compound (15), when i is 2, there are two rings E¹. In this compound, two groups represented by two rings E¹ may be the same as or different from each other. When i is greater than 2, this rule also applies to any two rings E¹. These rules also apply to other symbols.

The expression "at least one 'A'" means that the number of 'A's' is arbitrary. The expression "at least one 'A' is optionally substituted with 'B'" means that, when the number of 'A's' is 1, the position on 'A' is arbitrary and when the number of 'A's is 2 or more, the positions thereon can be selected without limitation. These rules also apply to the expression "at least one 'A' is substituted with 'B'." The expression "at least one 'A' is optionally substituted with 'B,' 'C,' or 'D'" means a case in which any 'A' is substituted with 'B,' a case in which any 'A' is substituted with 'C,' and a case in which any 'A' is substituted with 'D,' and also a case in which a plurality of 'A's are substituted with at least two of 'B,' 'C,' and/or 'D.' For example, "an alkyl group in which at least one -CH₂- is optionally substituted with -O- or -CH=CH-" includes an alkyl group, an alkoxy group, an alkoxyalkyl group, an alkenyl group, an alkoxyalkenyl group, and an alkenyloxyalkyl group. Here, it is not preferable for two consecutive -CH₂-'s to be substituted with -O-, and -O-O- formed. In an alkyl group and the like, it is not preferable for -CH₂- in a methyl moiety (-CH₂-H) to be substituted with -O- and -O-H formed.

The expression "R¹¹ and R¹² independently represent an alkyl group having 1 to 10 carbon atoms or an alkenyl group having 2 to 10 carbon atoms, and in the alkyl and alkenyl groups, at least one -CH₂- is optionally substituted with -O-, and in these groups, at least one hydrogen atom is optionally substituted with a fluorine atom" may be used. In this expression, "in these groups" may be interpreted literally. In this expression, "these groups" refers to an alkyl group, an alkenyl group, an alkoxy group, and an alkenyloxy group. That is, "these groups" indicate all of groups shown before the expression "in these groups." This common interpretation also applies to the expressions "in these monovalent groups" and "in these divalent groups." For example, "these monovalent groups" indicate all of groups shown before the expression "in these monovalent groups."

A halogen includes fluorine, chlorine, bromine, and iodine. A preferable halogen is fluorine or chlorine. A more preferable halogen is fluorine. In a liquid crystalline compound, an alkyl group is linear or branched and does not include a cyclic alkyl group. Generally, a linear alkyl group is more preferable than a branched alkyl group. This also applies to a terminal group such as an alkoxy group and an alkenyl group. Regarding the configuration of 1,4-cyclohexylene, the trans type is preferable to the cis type in order to increase the upper limit temperature. 2-Fluoro-1,4-phenylene refers to the following two divalent groups. In the chemical formula, fluorine may be leftward (L) or rightward (R). These rules also apply to an asymmetric divalent group that is formed by removing two hydrogen atoms from a ring such as tetrahydropyran-2,5-diyl.

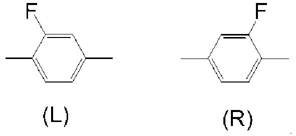

The present invention includes the following items.
Item 1. A compound represented by Formula (1): 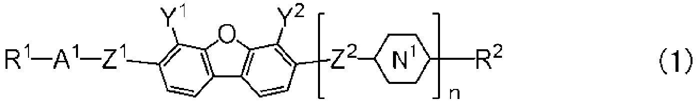 in Formula (1),
   R¹ and R² independently represent a hydrogen atom or an alkyl group having 1 to 10 carbon atoms, and in the alkyl group, at least one -CH₂- is optionally substituted with -O-, -S-, -CO-, or -SiH₂-, at least one -CH₂CH₂- is optionally substituted with -CH=CH- or -C≡C-, and in these groups, at least one hydrogen atom is optionally substituted with a fluorine atom;
   the ring A¹ represents 1,2-cyclopropylene, 1,3-cyclobutylene, 1,3-cyclopentylene, 1,3-cyclopentenylene, or 1,4-cyclopentenylene, and in these groups, at least one -CH₂- is optionally substituted with -O-;
   the ring N¹ represents 1,4-cyclohexylene, decahydronaphthalene-2,6-diyl, or 1,4-phenylene, and in these groups, at least one -CH₂- is optionally substituted with -O-, -S-, -CO-, or -SiH₂-, at least one -CH₂CH₂- is optionally substituted with -CH=CH- or -CH=N-, and in these divalent groups, at least one hydrogen atom is optionally substituted with a fluorine atom, a chlorine atom, -C≡N, -CF₃, -CHF₂, -CH₂F, -OCF₃, -OCHF₂, or -OCH₂F;
   Y¹ and Y² independently represent a hydrogen atom, a fluorine atom, a chlorine atom, -CF₃, -CHF₂, -CH₂F, -OCF₃, -OCHF₂, or -OCH₂F;
   n is 0, 1 or 2; and
   Z¹ and Z² independently represent a single bond or an alkylene group having 1 to 6 carbon atoms, and in the alkylene group, at least one -CH₂- is optionally substituted with -O-, -S-, or -CO-, one or two -CH₂CH₂-'s are optionally substituted with -CH=CH- or -C≡C-, and in these divalent groups, at least one hydrogen atom is optionally substituted with a fluorine atom.
Item 2. The compound according to Item 1,
   wherein, in Formula (1),
   R¹ and R² independently represent a hydrogen atom, an alkyl group having 1 to 10 carbon atoms, an alkoxy group having 1 to 9 carbon atoms, an alkoxyalkyl group having 2 to 9 carbon atoms, an alkenyl group having 2 to 10 carbon atoms, or an alkenyloxy group having 2 to 9 carbon atoms;
   the ring N¹ represents 1,4-cyclohexylene or 1,4-phenylene, and in these groups, at least one -CH₂- is optionally substituted with -O-, at least one -CH₂CH₂- is optionally substituted with -CH=CH-, and in these divalent groups, at least one hydrogen atom is optionally substituted with a fluorine atom;
   Y¹ and Y² independently represent a hydrogen atom or a fluorine atom; and
   Z¹ and Z² independently represent a single bond or an alkylene group having 1 to 6 carbon atoms, and at least one -CH₂- is optionally substituted with -O-.
Item 3. The compound according to Item 1 represented by Formulae (1-1) to (1-3): 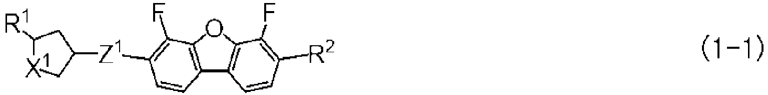 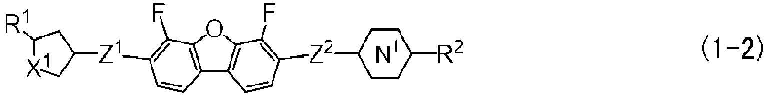 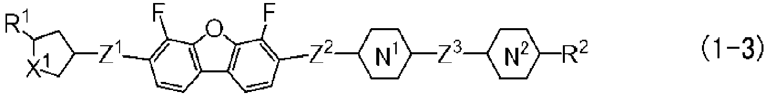 in Formulae (1-1) to (1-3),
   R¹ and R² independently represent a hydrogen atom, an alkyl group having 1 to 10 carbon atoms, an alkoxy group having 1 to 9 carbon atoms, an alkoxyalkylgroup having 2 to 9 carbon atoms, an alkenyl group having 2 to 10 carbon atoms, or an alkenyloxy group having 2 to 9 carbon atoms;
   X¹ represents -CH₂- or -O-;
   the ring N¹ and the ring N² represent 1,4-cyclohexylene, 1,4-cyclohexenylene, 1,4-phenylene, 1,4-phenylene in which at least one hydrogen atom is substituted with a halogen atom, or tetrahydropyran-2,5-diyl; and
   Z¹, Z² and Z³ represent a single bond or an alkylene group having 1 to 6 carbon atoms, and at least one -CH₂- is optionally substituted with -O-.
Item 4. The compound according to Item 3,
   wherein, in Formulae (1-1) to (1-3),
   Z¹ represents a single bond, -O-, -CH₂-, -CH₂O-, -OCH₂- or -CH₂CH₂-;
   X¹ represents -CH₂-; and
   Z² and Z³ represent a single bond.
Item 5. The compound according to Item 1 represented by any one of Formulae (1-4) to (1-14): 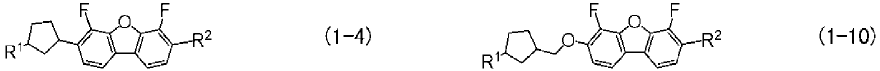 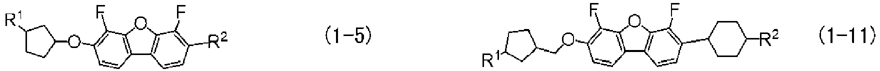 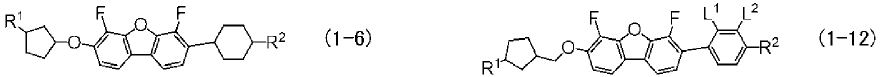 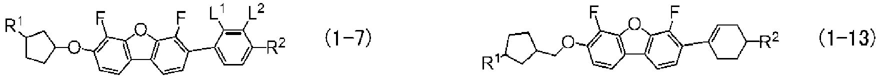 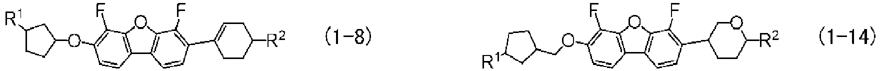 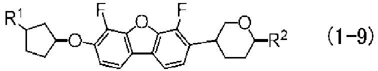 in Formulae (1-4) to (1-14),
   R¹ and R² independently represent a hydrogen atom, an alkyl group having 1 to 10 carbon atoms, an alkoxy group having 1 to 9 carbon atoms, an alkoxyalkyl group having 2 to 9 carbon atoms, an alkenyl group having 2 to 10 carbon atoms, or an alkenyloxy group having 2 to 9 carbon atoms; and L¹ and L² independently represent a hydrogen atom or a fluorine atom.
Item 6. The compound according to Item 5,
   wherein, in Formulae (1-4) to (1-14),
   R¹ represents a hydrogen atom, and R² represents an alkyl group having 1 to 10 carbon atoms, an alkoxy group having 2 to 9 carbon atoms, or an alkenyl group having 2 to 10 carbon atoms.
Item 7. The compound according to Item 1 represented by Formula (1-15) or (1-16): 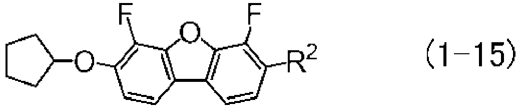 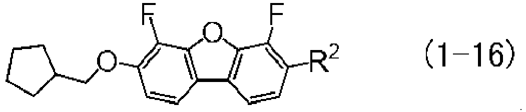 in Formulae (1-15) and (1-16), R² represents an alkoxy group having 1 to 9 carbon atoms.
Item 8. A liquid crystal composition including at least one compound selected from the group consisting of compounds represented by Formula (1) and at least one compound selected from the group consisting of compounds represented by Formulae (2) to (4): 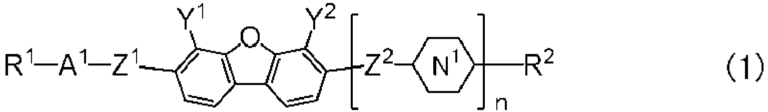 in Formula (1),
   R¹ and R² independently represent a hydrogen atom or an alkyl group having 1 to 10 carbon atoms, and in the alkyl group, at least one -CH₂- is optionally substituted with -O-, -S-, -CO-, or -SiH₂-, at least one -CH₂CH₂- is optionally substituted with -CH=CH- or -C≡C-, and in these groups, at least one hydrogen atom is optionally substituted with a fluorine atom;
   the ring A¹ represents 1,2-cyclopropylene, 1,3-cyclobutylene, 1,3-cyclopentylene, 1,3-cyclopentenylene, or 1,4-cyclopentenylene, and in these groups, at least one -CH₂- is optionally substituted with -O-;
   the ring N¹ represents 1,4-cyclohexylene or 1,4-phenylene, and in these groups, at least one -CH₂- is optionally substituted with -O-, -S-, -CO-, or -SiH₂-, at least one -CH₂CH₂- is optionally substituted with -CH=CH- or -CH=N-, and in these divalent groups, at least one hydrogen atom is optionally substituted with a fluorine atom, a chlorine atom, -C≡N, -CF₃, -CHF₂, -CH₂F, -OCF₃, -OCHF₂, or -OCH₂F;
   Y¹ and Y² independently represent a hydrogen atom, a fluorine atom, a chlorine atom, -CF₃, -CHF₂, -CH₂F, -OCF₃, -OCHF₂, or -OCH₂F;
   n is 0, 1 or 2; and
   Z¹ and Z² independently represent a single bond or an alkylene group having 1 to 6 carbon atoms, and in the alkylene group, at least one -CH₂- is optionally substituted with -O-, -S-, or -CO-, one or two -CH₂CH₂-'s are optionally substituted with -CH=CH- or -C≡C-, and in these divalent groups, at least one hydrogen atom is optionally substituted with a fluorine atom; and 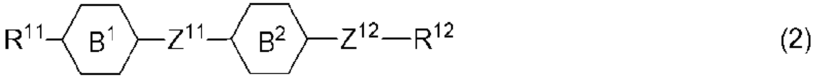 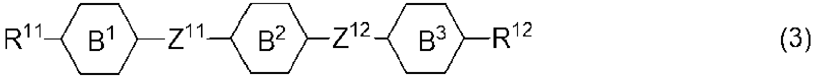 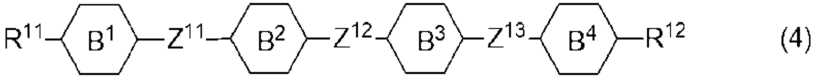
   in Formulae (2) to (4),
   R¹¹ and R¹² independently represent an alkyl group having 1 to 10 carbon atoms or an alkenyl group having 2 to 10 carbon atoms, and in the alkyl and alkenyl groups, at least one -CH₂- is optionally substituted with -O-, and in these groups, at least one hydrogen atom is optionally substituted with a fluorine atom;
   the ring B¹, the ring B², the ring B³, and the ring B⁴ independently represent 1,4-cyclohexylene, 1,4-phenylene, 2-fluoro-1,4-phenylene, 2,5-difluoro-1,4-phenylene, or pyrimidin-2,5-diyl; and
   Z¹¹, Z¹², and Z¹³ independently represent a single bond, -COO-, -CH₂CH₂-, -CH=CH-, or -C≡C-.
Item 9. The liquid crystal composition according to Item 8, further including at least one compound selected from the group consisting of compounds represented by Formulae (5) to (11): 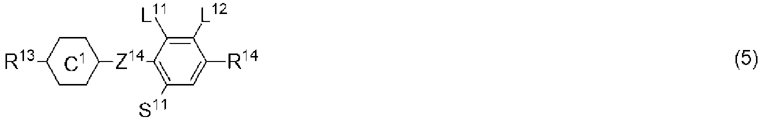 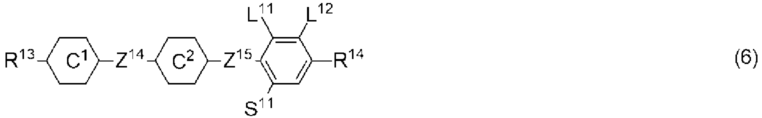 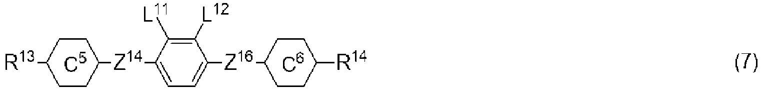 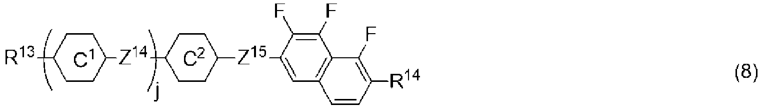 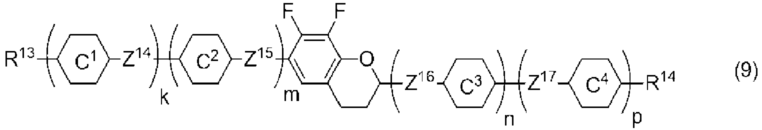 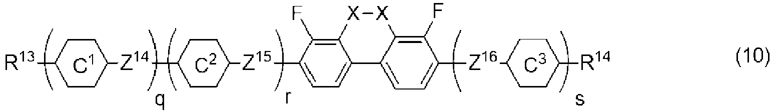 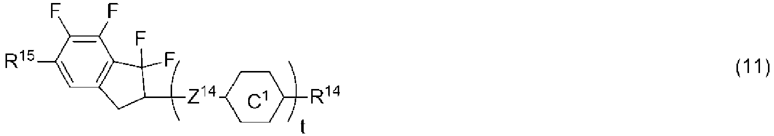 in Formulae (5) to (11),
   R¹³, R¹⁴ and R¹⁵ independently represent an alkyl group having 1 to 10 carbon atoms or an alkenyl group having 2 to 10 carbon atoms, and in the alkyl and alkenyl groups, at least one -CH₂- is optionally substituted with -O-, and in these groups, at least one hydrogen atom is optionally substituted with a fluorine atom, and R¹⁵ may be a hydrogen atom or a fluorine atom;
   the ring C¹, the ring C², the ring C³, and the ring C⁴ independently represent 1,4-cyclohexylene, 1,4-cyclohexenylene, 1,4-phenylene in which at least one hydrogen atom is optionally substituted with a fluorine atom, tetrahydropyran-2,5-diyl, or decahydronaphthalene-2,6-diyl;
   the ring C⁵ and the ring C⁶ independently represent 1,4-cyclohexylene, 1,4-cyclohexenylene, 1,4-phenylene, tetrahydropyran-2,5-diyl, or decahydronaphthalene-2,6-diyl;
   Z¹⁴, Z¹⁵, Z¹⁶, and Z¹⁷ independently represent a single bond, -COO-, -CH₂O-, -OCF₂-, -CH₂CH₂-, or -OCF₂CH₂CH₂-;
   L¹¹ and L¹² independently represent a fluorine atom or a chlorine atom;
   S¹¹ represents a hydrogen atom or a methyl group;
   X represents -CHF- or -CF₂-; and
   j, k, m, n, p, q, r, and s independently represent 0 or 1, a sum of k, m, n, and p is 1 or 2, a sum of q, r, and s is 0, 1, 2, or 3, and t is 1, 2, or 3.
Item 10. The liquid crystal composition according to Item 8 or 9, further including at least one compound selected from the group consisting of compounds represented by Formulae (12) to (14): 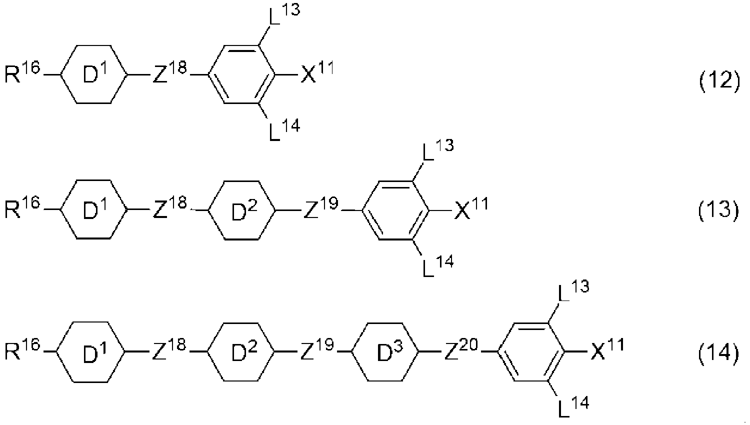 in Formulae (12) to (14),
   R¹⁶ represents an alkyl group having 1 to 10 carbon atoms or an alkenyl group having 2 to 10 carbon atoms, and in the alkyl and alkenyl groups, at least one -CH₂- is optionally substituted with -O-, and in these groups, at least one hydrogen atom is optionally substituted with a fluorine atom;
   X¹¹ represents a fluorine atom, a chlorine atom, -CF₃, -CHF₂, -CH₂F, -OCF₃, -OCHF₂, -OCF₂CHF₂, or -OCF₂CHFCF₃;
   the ring D¹, the ring D², and the ring D³ independently represent 1,4-cyclohexylene, 1,4-phenylene in which at least one hydrogen atom is optionally substituted with a fluorine atom, tetrahydropyran-2,5-diyl, 1,3-dioxane-2,5-diyl, or pyrimidin-2,5-diyl;
   Z¹⁸, Z¹⁹, and Z²⁰ independently represent a single bond, -COO-, -CH₂O-, -CF₂O-, -OCF₂-, -CH₂CH₂-, -CH=CH-, -C≡C-, or -(CH₂)₄-; and
   L¹³ and L¹⁴ independently represent a hydrogen atom or a fluorine atom.
Item 11. The liquid crystal composition according to any one of Items 8 to 10, further including at least one compound selected from the group consisting of compounds represented by Formula (15): 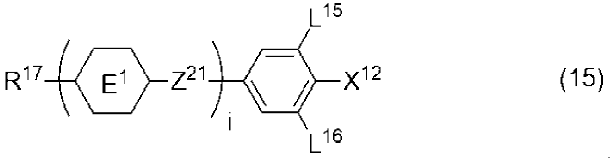 in Formula (15),
   R¹⁷ represents an alkyl group having 1 to 10 carbon atoms or an alkenyl group having 2 to 10 carbon atoms, and in the alkyl and alkenyl groups, at least one -CH₂- is optionally substituted with -O-, and in these groups, at least one hydrogen atom is optionally substituted with a fluorine atom;
   X¹² represents -C≡N or -C≡C-C≡N;
   the ring E¹ represents 1,4-cyclohexylene, 1,4-phenylene in which at least one hydrogen atom is optionally substituted with a fluorine atom, tetrahydropyran-2,5-diyl, 1,3-dioxane-2,5-diyl, or pyrimidin-2,5-diyl;
   Z²¹ represents a single bond, -COO-, -CH₂O-, -CF₂O-, -OCF₂-, -CH₂CH₂-, or -C≡C-;
   L¹⁵ and L¹⁶ independently represent a hydrogen atom or a fluorine atom; and
   i is 1, 2, 3, or 4.
Item 12. A liquid crystal display element including the liquid crystal composition according to any one of Items 8 to 11.

The present invention also includes the following items. (a) A composition that further includes at least one optically active compound and/or a polymerizable compound. (b) A composition that further includes at least one antioxidant and/or a UV absorber.

The present invention also includes the following items. (c) A composition that further includes one, two, or at least three additives selected from among the group consisting of a polymerizable compound, a polymerization initiator, a polymerization inhibitor, an optically active compound, an antioxidant, a UV absorber, a light stabilizer, a heat stabilizer, a dye, and an antifoaming agent. (d) A composition in which an upper limit temperature of a nematic phase is 70 °C or higher, an optical anisotropy (measured at 25 °C) at a wavelength of 589 nm is 0.08 or more and a dielectric anisotropy (measured at 25 °C) at a frequency of 1 kHz is -2 or lower.

The present invention also includes the following items. (e) An element which includes the composition and has a PC, TN, STN, ECB, OCB, IPS, VA, FFS, FPA, or PSA mode. (f) An AM element which includes the composition. (g) A transmission type element which includes the composition. (h) A use of the composition as a composition having a nematic phase. (i) A use as an optically active composition by adding an optically active compound to the composition.

Forms of Compound (1), synthesis of Compound (1), a liquid crystal composition, and a liquid crystal display element will be described in order.

### 1. Forms of Compound (1)

Compound (1) of the present invention has a dibenzofuran ring and a 3-membered ring, 4-membered ring, or 5-membered ring structure.

The compound is physically and chemically very stable under conditions in which the element is generally used, and has large dielectric anisotropy and favorable compatibility with other liquid crystalline compounds. A composition including the compound is stable under conditions in which the element is generally used. When the composition is stored at a low temperature, the compound is less likely to precipitate as crystals (or a smectic phase). The compound has general physical properties necessary for components of the composition, appropriate optical anisotropy, and appropriate dielectric anisotropy.

Preferable examples of the terminal groups R¹ and R², the ring A¹, the ring N¹, the linking groups Z¹ and Z², and the side groups Y¹ and Y² in Compound (1) are as follows. These examples also apply to sub-formulae of Compound (1). In Compound (1), when these groups are appropriately combined, physical properties can be arbitrarily adjusted. Compound (1) may include a larger amount of isotopes such as ²H (deuterium) and ¹³C than a natural abundance of these isotopes, since there are no large differences in the physical properties of such a compound. Here, definitions of symbols of Compound (1) are those described in Item 1.

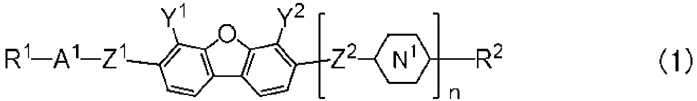

In Formula (1), R¹ and R² independently represent a hydrogen atom or an alkyl group having 1 to 10 carbon atoms, and in the alkyl group, at least one -CH₂- is optionally substituted with -O-, -S-, -CO-, or -SiH₂-, at least one -CH₂CH₂- is optionally substituted with -CH=CH- or -C≡C-, and in these groups, at least one hydrogen atom is optionally substituted with a fluorine atom.

Preferable examples of R¹ and R² include a hydrogen atom, an alkyl group, an alkoxy group, an alkoxyalkyl group, an alkoxyalkoxy group, an alkylthio group, an alkylthioalkoxy group, an acyl group, an acylalkyl group, an acyloxy group, an acyloxyalkyl group, an alkoxycarbonyl group, an alkoxycarbonylalkyl group, an alkenyl group, an alkenyloxy group, an alkenyloxyalkyl group, an alkoxyalkenyl group, an alkynyl group, an alkynyloxy group, a silaalkyl group, and a disilaalkyl group. In these groups, at least one hydrogen atom is optionally substituted with a fluorine atom or a chlorine atom. These examples include groups in which at least two hydrogen atoms are substituted with both fluorine atoms and chlorine atoms. Groups in which at least one hydrogen atom is substituted with only fluorine atoms are more preferable. In these groups, a linear chain is preferable to a branched chain. It is preferable for R¹ or R² to have a branched chain, and be optically active. More preferably, R¹ or R² is an alkyl group, an alkoxy group, an alkoxyalkyl group, an alkenyl group, a monofluoroalkyl group or a monofluoroalkoxy group.

A preferable configuration of -CH=CH- in the alkenyl group depends on the position of a double bond. A trans configuration is preferable in an alkenyl group such as 1-propenyl, 1-butenyl, 1-pentenyl, 1-hexenyl, 3-pentenyl, and 3-hexenyl. A cis configuration is preferable in an alkenyl group such as 2-butenyl, 2-pentenyl, and 2-hexenyl.

Specific examples of R¹ or R² include a hydrogen atom, a methyl group, an ethyl group, a propyl group, a butyl group, a pentyl group, a hexyl group, a heptyl group, an octyl group, a methoxy group, an ethoxy group, a propoxy group, a butoxy group, a pentyloxy group, a hexyloxy group, a heptyloxy group, a methoxymethyl group, a methoxyethyl group, a methoxypropyl group, an ethoxymethyl group, an ethoxyethyl group, an ethoxypropyl group, a propoxymethyl group, a butoxymethyl group, a pentoxymethyl group, a vinyl group, a 1-propenyl group, a 2-propenyl group, a 1-butenyl group, a 2-butenyl group, a 3-butenyl group, a 1-pentenyl group, a 2-pentenyl group, a 3-pentenyl group, a 4-pentenyl group, a 2-propenyloxy group, a 2-butenyloxy group, a 2-pentenyloxy group, and a 1-propynyl group.

Preferable examples of R¹ or R² include a hydrogen atom, a methyl group, an ethyl group, a propyl group, a butyl group, a pentyl group, a hexyl group, a methoxy group, an ethoxy group, a propoxy group, a butoxy group, a pentyloxy group, a methoxymethyl group, an ethoxymethyl group, a propoxymethyl group, a vinyl group, a 1-propenyl group, a 2-propenyl group, a 1-butenyl group, a 2-butenyl group, a 3-butenyl group, a 1-pentenyl group, a 2-pentenyl group, a 3-pentenyl group, a 4-pentenyl group, a 2-propenyloxy group, a 2-butenyloxy group and a 2-pentenyloxy group. Most preferably, R¹ is a hydrogen atom, and most preferably, R² is a methyl, ethyl, propyl, butyl, pentyl, hexyl, methoxy, ethoxy, propoxy, butoxy, pentyloxy or hexyloxy group.

In Formula (1), the ring A¹ represents 1,2-cyclopropylene, 1,3-cyclobutylene, 1,3-cyclopentylene, 1,3-cyclopentenylene, or 1,4-cyclopentenylene, and in these groups, at least one -CH₂- is optionally substituted with -O-.

Preferably, the ring A¹ represents 1,2-cyclopropylene, 1,3-cyclopentylene, 1,3-cyclopentenylene, 1,4-cyclopentenylene, 2,4-tetrahydrofuranyl, or 2,5-tetrahydrofuranyl. Particularly preferably, the ring A¹ represents 1,3-cyclopentylene.

In Formula (1), the ring N¹ represents 1,4-cyclohexylene, decahydronaphthalene-2,6-diyl, or 1,4-phenylene, and in these groups, at least one -CH₂-is optionally substituted with -O-, -S-, -CO-, or -SiH₂-, at least one -CH₂CH₂- is optionally substituted with -CH=CH- or -CH=N-, and in these divalent groups, at least one hydrogen atom is optionally substituted with a fluorine atom, a chlorine atom, -C≡N, -CF₃, -CHF₂, -CH₂F, -OCF₃, -OCHF₂, or -OCH₂F.

Preferable examples of "in these groups, at least one -CH₂- is optionally substituted with -O-, -S-, -CO-, or -SiH₂-, at least one -CH₂CH₂- is optionally substituted with -CH=CH- or -CH=N-" include divalent groups represented by the following Formulae (16-1) to (16-50). More preferable examples include divalent groups represented by Formulae (16-1) to (16-4), Formula (16-15), Formula (16-23), Formulae (16-27) to (16-29), Formula (16-36), Formula (16-39), and Formula (16-45).

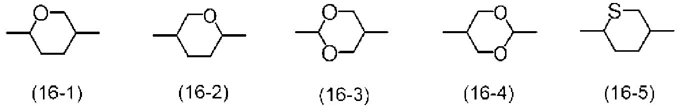

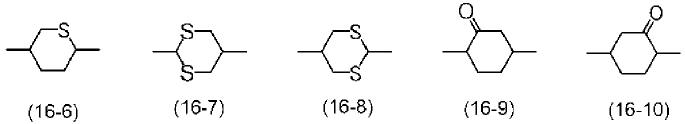

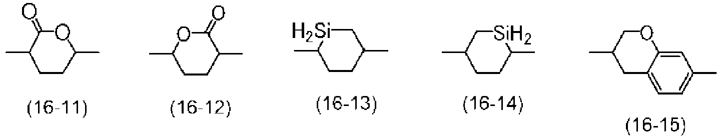

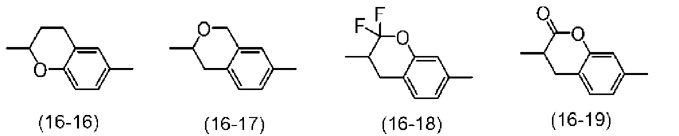

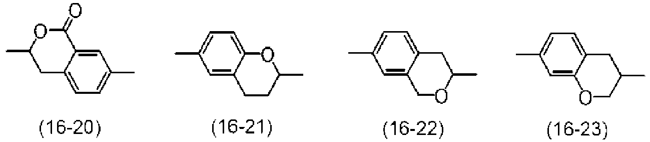

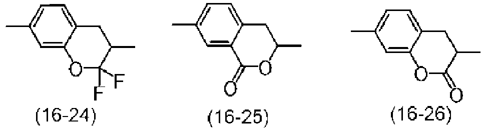

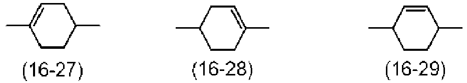

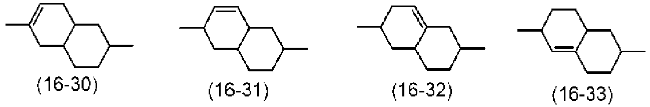

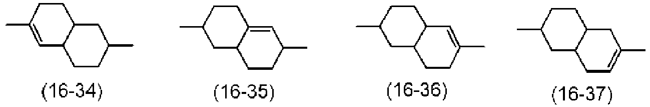

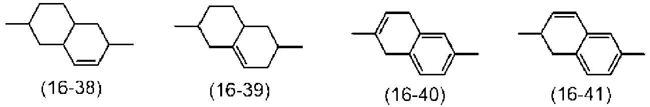

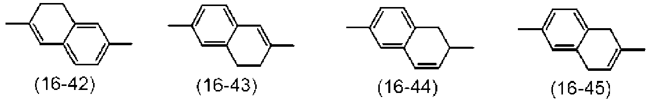

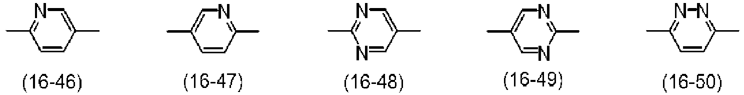

Preferable examples of "in these divalent groups, at least one hydrogen atom is optionally substituted with a fluorine atom, a chlorine atom, -C≡N, -CF₃, -CHF₂, -CH₂F, -OCF₃, -OCHF₂, or -OCH₂F" include divalent groups represented by the following Formulae (17-1) to (17-71). More preferable examples include divalent groups represented by Formulae (17-1) to (17-4), Formula (17-6), Formulae (17-10) to (17-15), and Formulae (17-54) to (17-59).

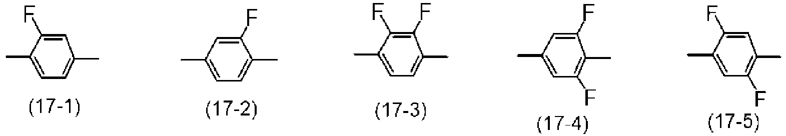

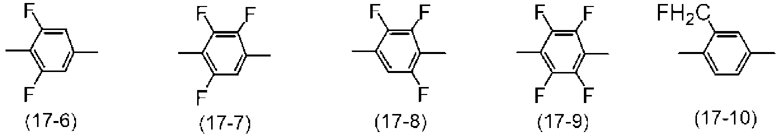

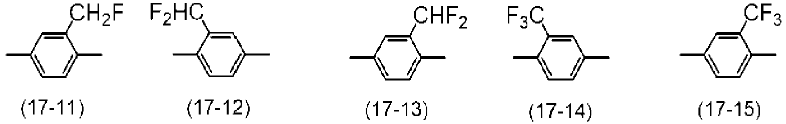

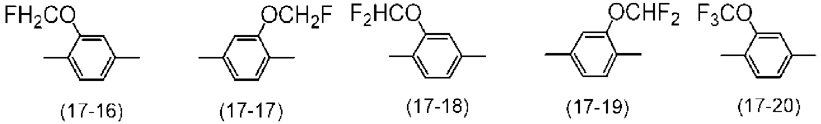

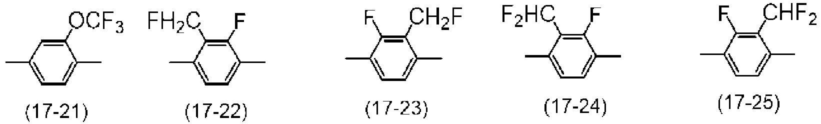

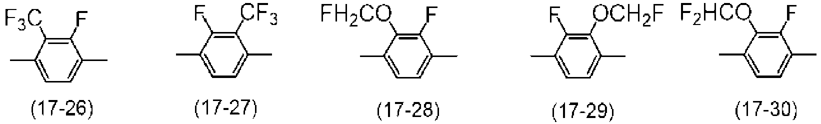

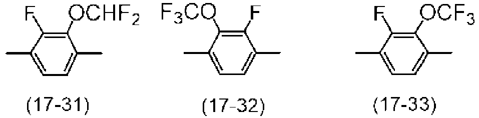

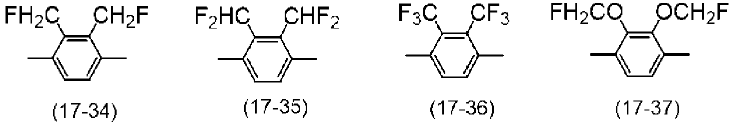

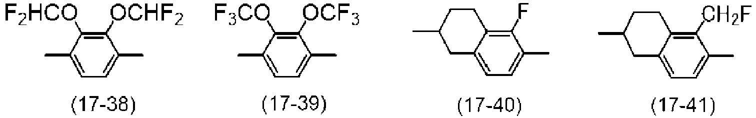

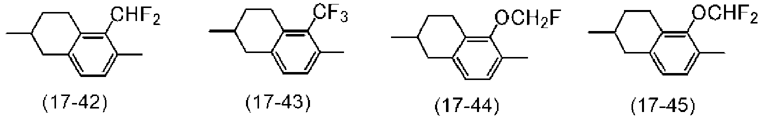

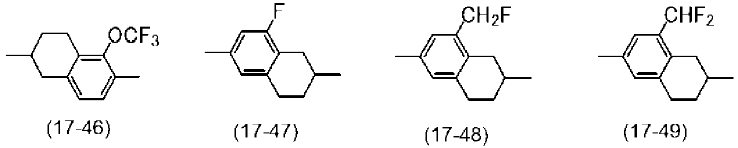

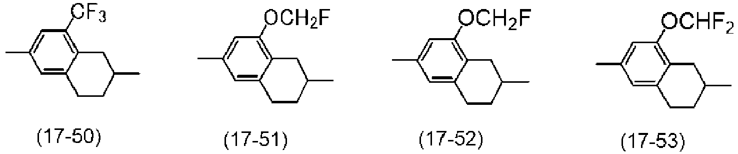

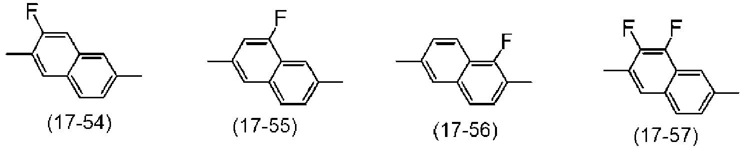

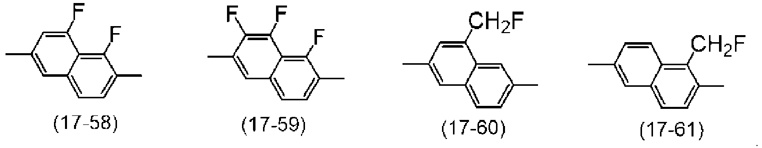

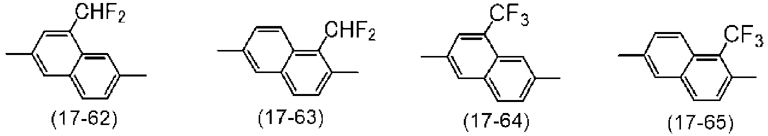

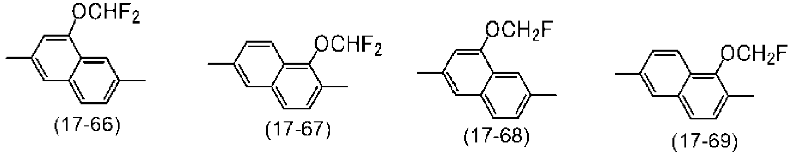

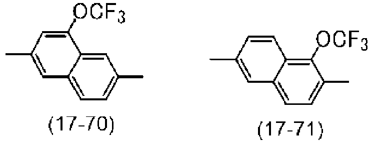

More preferable examples of the ring N¹ include 1,4-cyclohexylene, 1,4-cyclohexenylene, 1,3-dioxane-2,5-diyl, 1,4-phenylene, 2-fluoro-1,4-phenylene, 2,3-difluoro-1,4-phenylene, 2,5-difluoro-1,4-phenylene, 2,6-difluoro-1,4-phenylene, 2,3,5-trifluoro-1,4-phenylene, pyridine-2,5-diyl, 3-fluoropyridine-2,5-diyl, pyrimidin-2,5-diyl, pyridazine-2,5-diyl, decahydronaphthalene-2,6-diyl, 1,2,3,4-tetrahydronaphthalene-2,6-diyl, and naphthalene-2,6-diyl. Regarding the configuration of 1,4-cyclohexylene and 1,3-dioxane-2,5-diyl, the trans type is preferable to the cis type.

Particularly preferable examples of the ring N¹ include 1,4-cyclohexylene, 1,3-dioxane-2,5-diyl, 1,4-phenylene, 2-fluoro-1,4-phenylene, 2,3-difluoro-1,4-phenylene, 2,5-difluoro-1,4-phenylene, 2,6-difluoro-1,4-phenylene, pyridine-2,5-diyl, and pyrimidin-2,5-diyl. Most preferable examples of the ring N¹ include 1,4-cyclohexylene and 1,4-phenylene.

In Formula (1), Z¹ and Z² represent a single bond or an alkylene group having 1 to 6 carbon atoms, and in the alkylene group, at least one -CH₂- is optionally substituted with -O-, -S-, or -CO-, one or two -CH₂CH₂-'s are optionally substituted with -CH=CH- or -C≡C-, and in these divalent groups, at least one hydrogen atom is optionally substituted with a fluorine atom.

Specific examples of Z¹ and Z² include a single bond, -O-, -COO-, -OCO-, -CH₂O-, -OCH₂-, -CF₂O-, -OCF₂-, -CH₂-, -CH₂CH₂-, -CH=CH-, -CF=CH-, -CH=CF-, -CF=CF-, -C≡C-, -CH₂CO-, -COCH₂-, -(CH₂)₄-, -(CH₂)₂COO-, -(CH₂)₂OCO-, -OCO(CH₂)₂-, -COO(CH₂)₂-, -(CH₂)₂CF₂O-, -(CH₂)₂OCF₂-, -OCF₂(CH₂)₂-, -CF₂O(CH₂)₂-, -(CH₂)₃O-, and -O(CH₂)₃-. Regarding the configuration of a double bond of a linking group such as -CH=CH-, -CF=CF-, -CH=CH-CH₂O-, and -OCH₂-CH=CH-, the trans type is preferable to the cis type.

Preferable examples of Z¹ and Z² include a single bond, -O-, -COO-, -OCO-, -CH₂O-, -OCH₂-, -CF₂O-, -OCF₂-, -CH₂-, -CH₂CH₂-, -CH=CH-, -CF=CF-, -C≡C-, and -(CH₂)₄-. More preferable examples of Z¹ include a single bond, -O-, -CH₂O-, -CH₂-, and -CH₂CH₂-. Most preferable examples of Z¹ include -O- and -CH₂O-, and most preferable examples of Z² include a single bond, -CH₂O-, and -OCH₂-.

Y¹ and Y² independently represent a hydrogen atom, a fluorine atom, a chlorine atom, -CF₃, -CHF₂, -CH₂F, -OCF₃, -OCHF₂, or -OCH₂F. Preferably, Y¹ and Y² represent a hydrogen atom or a fluorine atom. Particularly preferably, Y¹ and Y² represent a fluorine atom.

In Formula (1), n is 0, 1 or 2. Compound (1) has one ring to three rings, not counting the 5-membered ring A¹. The type of the ring includes a condensed ring and a cross-linked six-membered ring in addition to a general six-membered ring. When Compound (1) has one ring, the compatibility with other liquid crystalline compounds is very favorable. When Compound (1) has one ring or two rings, the viscosity is low. When Compound (1) has two rings or three rings, the upper limit temperature is high.

When the terminal group, ring and linking group of Compound (1) are appropriately selected, physical properties such as optical anisotropy and dielectric anisotropy can be arbitrarily adjusted. The effects of the types of the terminal groups R¹ and R², the ring A¹, the ring N¹, the side groups Y¹ and Y², and the linking group Z¹ on physical properties of Compound (1) will be described below.

In Compound (1), when R¹ or R² is linear, a temperature range of a liquid crystal phase is wide and the viscosity is low. When R¹ or R² has a branched chain, the compatibility with other liquid crystalline compounds is favorable. A compound in which R¹ or R² is an optically active group is useful as a chiral dopant. When the compound is added to the composition, it is possible to prevent a reverse twisted domain that is generated in the element. A compound in which R¹ or R² is not an optically active group is useful as a component of the composition. When R¹ or R² is an alkenyl group, a preferable configuration depends on the position of a double bond. An alkenyl compound having a preferable configuration has a high upper limit temperature or a wide temperature range of a liquid crystal phase. Details are described in Mol. Cryst. Liq. Cryst., 1985, 131, 109 and Mol. Cryst. Liq. Cryst., 1985, 131, 327.

When the ring A¹ is 1,3-cyclopentylene, the viscosity is low.

When the ring N¹ is 1,4-phenylene in which at least one hydrogen atom is optionally substituted with a fluorine atom or a chlorine atom, pyridine-2,5-diyl, pyrimidin-2,5-diyl, or pyridazine-3,6-diyl, the optical anisotropy is large. When the ring is 1,4-cyclohexylene, 1,4-cyclohexenylene or 1,3-dioxane-2,5-diyl, the optical anisotropy is small.

When the linking groups Z¹ and Z² are a single bond, -CH₂O-, -CF₂O-, -OCF₂-, -CH₂CH₂-, -CH=CH-, -CF=CF-, or -(CH₂)₄-, the viscosity is low. When the linking group is a single bond, -OCF₂-, -CF₂O-, -CH₂CH₂-, or -CH=CH-, the viscosity is lower. When the linking group is -O- or -CH₂O-, the dielectric anisotropy is large.

When the side groups Y¹ and Y² are F, Compound (1) has large dielectric anisotropy.

When Compound (1) has one ring or two rings, the viscosity is low. When Compound (1) has two rings or three rings, the upper limit temperature is high. As described above, it is possible to obtain a compound having required physical properties by appropriately selecting the types of the terminal group, ring, side group, and linking group. Therefore, Compound (1) is useful as a component of the composition used for elements having a mode such as PC, TN, STN, ECB, OCB, IPS, and VA.

Preferable examples of Compound (1) include Compound (1-1) described in Item 3. More preferable examples include compounds represented by sub-formulae in Item 4 and the like. Compound (1) is suitable for elements having a mode such as VA, IPS, and PSA.

### 2. Synthesis of Compound (1)

A synthesis method of Compound (1) will be described. Compound (1) can be synthesized by appropriately combining methods in synthetic organic chemistry. Methods of introducing required terminal groups, rings and linking groups into a starting material are described in books such as "Organic Syntheses" (John Wiley & Sons, Inc.), "Organic Reactions" (John Wiley & Sons, Inc.), "Comprehensive Organic Synthesis" (Pergamon Press), and "New Course of Experimental Chemistry" (Maruzen).

### 2-1. Generation of linking groups Z¹ and Z²

First, a scheme of a method of generating linking groups Z¹ and Z² is shown. Next, the reaction described in the scheme in Methods (1) to (11) will be described. In this scheme, one of MSG¹'s (or MSG²'s) is a monovalent organic group having a six-membered ring or a condensed ring, and the other is a monovalent organic group having one of a 3-membered ring, a 4-membered ring, and a 5-membered ring. Monovalent organic groups represented by a plurality of MSG¹'s (or MSG²'s) used in the scheme may be the same as or different from each other. Compounds (1A) to (1J) correspond to Compound (1).

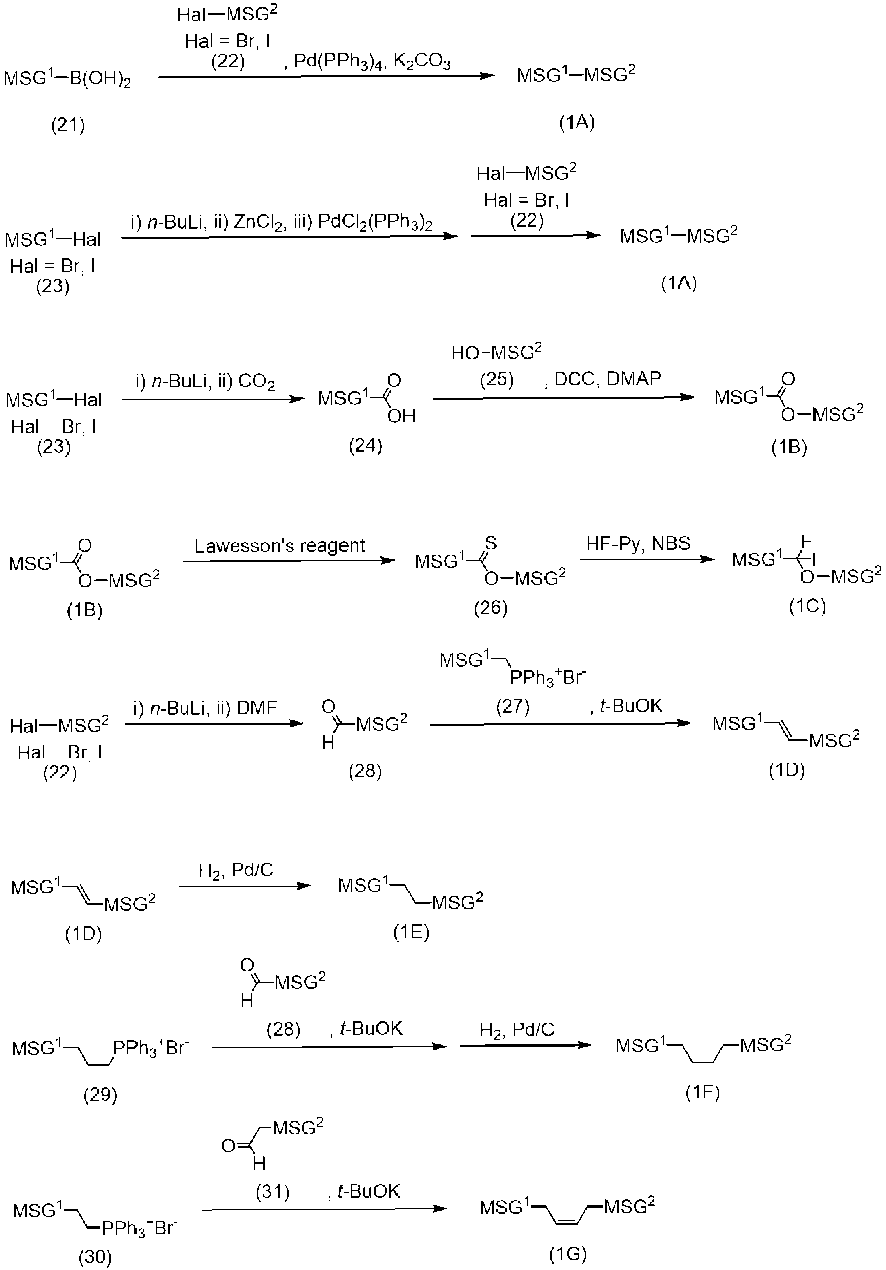

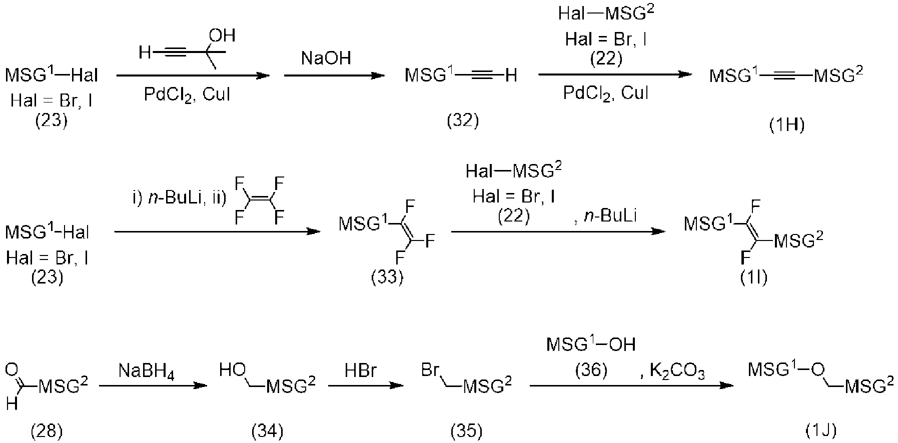

### (1) Generation of single bond

An arylboronic acid (21) and a halide (22) synthesized by a known method are reacted in the presence of a catalyst such as a carbonate and tetrakis(triphenylphosphine)palladium to synthesize Compound (1A). Compound (1A) is also synthesized by reacting a halide (23) synthesized by a known method with n-butyl lithium, and then with zinc chloride, and reacting a halide (22) in the presence of a catalyst such as dichlorobis(triphenylphosphine)palladium.

### (2) Generation of -COO-

A halide (23) is reacted with n-butyl lithium and subsequently with carbon dioxide to obtain a carboxylic acid (24). Compound (25) and carboxylic acid (24) synthesized by a known method are dehydrated in the presence of DCC (1,3-dicyclohexylcarbodiimide) and DMAP (4-dimethylaminopyridine) to synthesize Compound (1B).

### (3) Generation of -CF₂O-

Compound (1B) is treated with a sulfurizing agent such as Lawesson's reagent to obtain a thionoester (26). The thionoester (26) is fluorinated with a hydrogen fluoride pyridine complex and NBS (N-bromosuccinimide) to synthesize Compound (1C). Refer to M. Kuroboshi et al., Chem. Lett., 1992, 827. Compound (1C) is also synthesized by fluorinating a thionoester (26) with DAST ((diethylamino)sulfur trifluoride). Refer to W. H. Bunnelle et al., J. Org. Chem. 1990, 55, 768. It is also possible to generate a linking group according to the method described in Peer. Kirsch et al., Angew. Chem. Int. Ed. 2001, 40, 1480.

### (4) Generation of -CH=CH-

A halide (22) is treated with n-butyl lithium and then reacted with DMF (N,N-dimethylformamide) to obtain an aldehyde (28). A phosphonium salt (27) synthesized by a known method is treated with a base such as potassium t-butoxide to generate a phosphorus ylide. The phosphorus ylide is reacted with the aldehyde (28) to synthesize Compound (1D). Since a cis form is generated depending on reaction conditions, the cis form is isomerized to a trans form as necessary by a known method.

### (5) Generation of -CH₂CH₂-

Compound (1D) is hydrogenated in the presence of a catalyst such as palladium carbon to synthesize Compound (1E).

### (6) Generation of -(CH₂)₄-

A compound having -(CH₂)₂-CH=CH- is obtained by a method (4) using a phosphonium salt (29) in place of a phosphonium salt (27). This is catalytically hydrogenated to synthesize Compound (1F).

### (7) Generation of -CH₂CH=CHCH₂-

Compound (1G) is synthesized by a method (4) using a phosphonium salt (30) in place of a phosphonium salt (27) and an aldehyde (31) in place of an aldehyde (28). Since a trans form is generated depending on reaction conditions, the trans form is isomerized to a cis form as necessary by a known method.

### (8) Generation of -C≡C-

A halide (23) is reacted with 2-methyl-3-butyn-2-ol in the presence of a catalyst such as dichloropalladium and a copper halide and then deprotected under basic conditions to obtain Compound (32). In the presence of a catalyst such as dichloropalladium and a copper halide, Compound (32) is reacted with a halide (22) to synthesize Compound (1H).

### (9) Generation of -CF=CF-

A halide (23) is treated with n-butyl lithium and then reacted with tetrafluoroethylene to obtain Compound (33). A halide (22) is treated with n-butyl lithium and then reacted with Compound (33) to synthesize Compound (1I).

### (10) Generation of -OCH₂-

An aldehyde (28) is reduced with a reducing agent such as sodium borohydride to obtain Compound (34). Compound (34) is brominated with hydrobromic acid or the like to obtain a bromide (35). In the presence of a base such as potassium carbonate, the bromide (35) is reacted with Compound (36) to synthesize Compound (1J).

### (11) Generation of -(CF₂)₂-

According to the method described in J. Am. Chem. Soc., 2001, 123, 5414, a diketone (-COCO-) is fluorinated with sulfur tetrafluoride in the presence of a hydrogen fluoride catalyst to obtain a compound having -(CF₂)₂-.

### 2-2. Generation of ring N¹

Next, a method of generating a ring N¹ will be described. For rings such as 1,4-cyclohexylene, 1,3-dioxane-2,5-diyl, 1,4-phenylene, 2-fluoro-1,4-phenylene, 2,3-difluoro-1,4-phenylene, pyridine-2,5-diyl, and pyrimidin-2,5-diyl, starting materials are commercially available and generation methods thereof are well known. Therefore, Compounds (38), (41), and (45) shown below will be described.

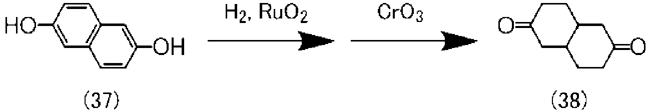

Decahydronaphthalene-2,6-dione (38) is a starting material for a compound including decahydronaphthalene-2,6-diyl. Compound (38) is obtained by catalytic hydrogen reduction of a diol (37) in the presence of ruthenium oxide and additionally performing oxidation with chromium oxide according to the method described in Japanese Unexamined Patent Application Publication No. 2000-239564. The compound is converted into Compound (1) by a general method.

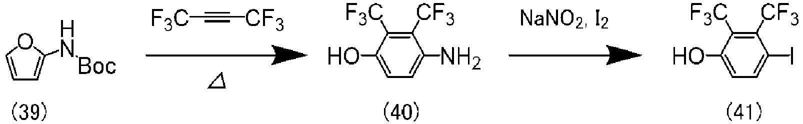

A structural unit of 2,3-(bistrifluoromethyl)phenylene is synthesized by the method described in Org. Lett., 2000, 2 (21), 3345. An aniline (40) is synthesized by the Diels-Alder reaction of a furan (39) and 1,1,1,4,4,4-hexafluoro-2-butyne at a high temperature. The compound is subjected to the Sandmeyer reaction according to the method described in Org. Synth. Coll., Vol. 2, 1943, 355 to obtain an iodide (41). The compound is converted into Compound (1) by a general method.

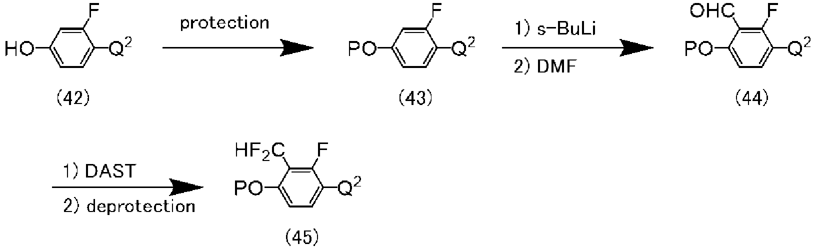

A structural unit of 2-difluoromethyl-3-fluorophenylene is synthesized by the following method. A hydroxyl group of Compound (42) is protected with an appropriate protecting group to obtain Compound (43). P indicates a protecting group. Compound (43) is reacted with s-butyl lithium and subsequently with N,N-dimethylformamide (DMF) to obtain an aldehyde (44). The compound is fluorinated with diethylaminosulfur trifluoride (DAST) and subsequently deprotected to obtain a phenol (45). The compound is converted into Compound (1) by a general method.

### 2-4. Introduction of ring A¹

Next, a method of generating a ring A¹ will be described. A starting material such as cyclopropanemethanol, cyclopentanone, cyclopentanol, bromocyclopentane, 4-bromocyclopentene, 3-bromocyclopentene, 1-bromocyclopentene, 2-bromotetrahydrofuran, and 3-bromotetrahydrofuran is commercially available or a generation method thereof is well known, and it can be used to synthesize Compound (1) by a general method.

### 2-5. Generation of dibenzofuran ring

A method of generating a dibenzofuran ring is well known and can be synthesized with reference to the method described in Japanese Unexamined Patent Application Publication No. 2015-174864.

### 2-6. Synthesis method of Compound (1)

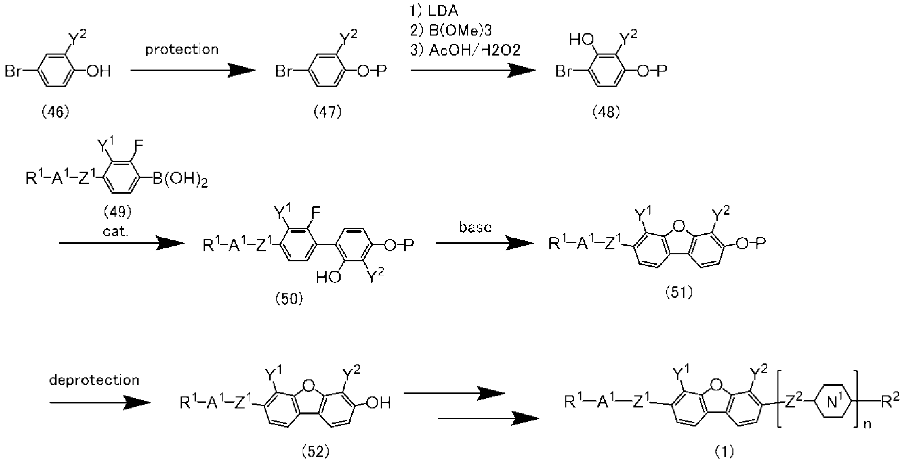

A hydroxyl group of Compound (46) is protected with an appropriate protecting group to obtain Compound (47). P indicates a protecting group. Compound (47) is reacted with LDA and reacted with trimethylborate and hydrogen peroxide to obtain Compound (48). Compound (49) and Compound (48) synthesized by a general method are coupled using a metal catalyst to synthesize Compound (50). Compound (50) is heated in the presence of a base and formed into a ring to obtain Compound (51). Subsequently, deprotection is performed to obtain Compound (52). The compound is converted into Compound (1) by a general method. In these compounds, the definitions of R¹, R², A¹, N¹, Y¹, Y², Z¹, Z² and n are the same as the definitions of the symbols described in Item 1.

### 3. Liquid crystal composition

### 3-1. Component compound

A liquid crystal composition of the present invention will be described. The composition includes at least one Compound (1) as Component (a). The composition may include two, three or more Compounds (1). A component of the composition may be only Compound (1). The composition preferably includes at least one of Compound (1) in a range of 1 weight% to 99 weight% in order to exhibit favorable physical properties. In a composition having negative dielectric anisotropy, a preferable content of Compound (1) is in a range of 5 weight% to 60 weight%. In a composition having positive dielectric anisotropy, a preferable content of Compound (1) is 30 weight% or less.

**Table 1. Component compounds of composition**

| Components | Component compounds | Dielectric anisotropy |
|---|---|---|
| Component (a) | Compound (1) | Large negative |
| Component (b) | Compound (2) to Compound (4) | Small |
| Component (c) | Compound (5) to Compound (11) | Large negative |
| Component (d) | Compound (12) to Compound (14) | Large positive |
| Component (e) | Compound (15) | Large positive |

The composition includes Compound (1) as Component (a). Preferably, the composition further includes a liquid crystalline compound selected from among Components (b) to (e) shown in Table 1. When the composition is prepared, it is preferable to select Components (b) to (e) in consideration of a positive or negative sign or a magnitude of dielectric anisotropy. The composition may include a liquid crystalline compound different from Compounds (1) to (15). The composition may not include such a liquid crystalline compound.

Component (b) is a compound in which two terminal groups are an alkyl group and the like. Preferable examples of Component (b) include Compounds (2-1) to (2-11), Compounds (3-1) to (3-19), and Compounds (4-1) to (4-7). In these compounds, R¹¹ and R¹² independently represent an alkyl group having 1 to 10 carbon atoms or an alkenyl group having 2 to 10 carbon atoms, and in the alkyl and alkenyl groups, at least one -CH₂- is optionally substituted with -O-, and in these groups, at least one hydrogen atom is optionally substituted with a fluorine atom.

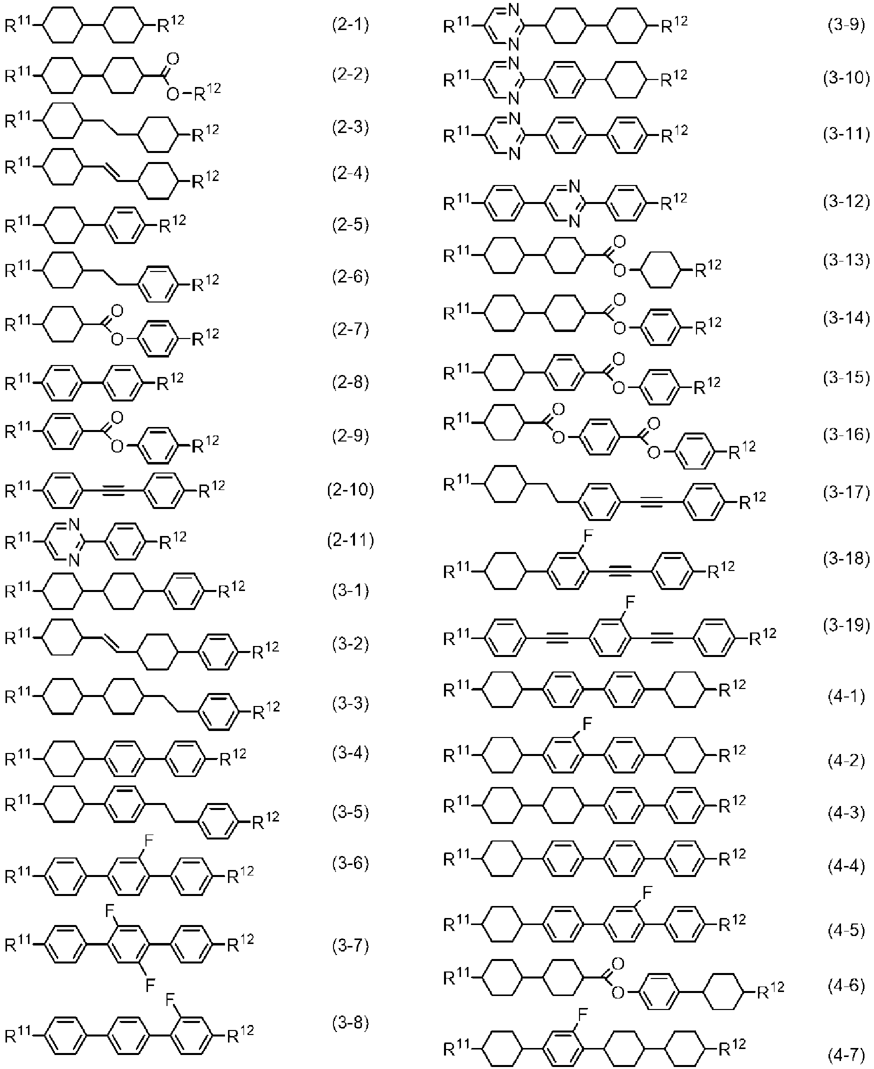

Component (b) has small dielectric anisotropy. Component (b) is close to being neutral. Compound (2) has an effect of lowering the viscosity or adjusting the optical anisotropy. Compounds (3) and (4) have an effect of increasing an upper limit temperature and thus widening a temperature range of a nematic phase or adjusting the optical anisotropy.

As the content of Component (b) increases, the viscosity of the composition decreases, but the dielectric anisotropy decreases. Therefore, a higher content is preferable as long as a required value of a threshold voltage of the element is satisfied. When a composition for a mode such as IPS and VA is prepared, the content of Component (b) is preferably 30 weight% or more, and more preferably 40 weight% or more on the basis of the weight of the liquid crystal composition.

Component (c) includes Compounds (5) to (11). These compounds include phenylene in which the lateral positions are substituted with two halogen atoms as in 2,3-difluoro-1,4-phenylene. Preferable examples of Component (c) include Compounds (5-1) to (5-8), Compounds (6-1) to (6-18), Compound (7-1), Compounds (8-1) to (8-3), Compounds (9-1) to (9-11), Compounds (10-1) to (10-3), and Compounds (11-1) to (11-3). In these compounds, R¹³, R¹⁴, and R¹⁵ independently represent an alkyl group having 1 to 10 carbon atoms or an alkenyl group having 2 to 10 carbon atoms, and in the alkyl and alkenyl groups, at least one -CH₂- is optionally substituted with -O-, and in these groups, at least one hydrogen atom is optionally substituted with a fluorine atom, and R¹⁵ may be a hydrogen atom or a fluorine atom.

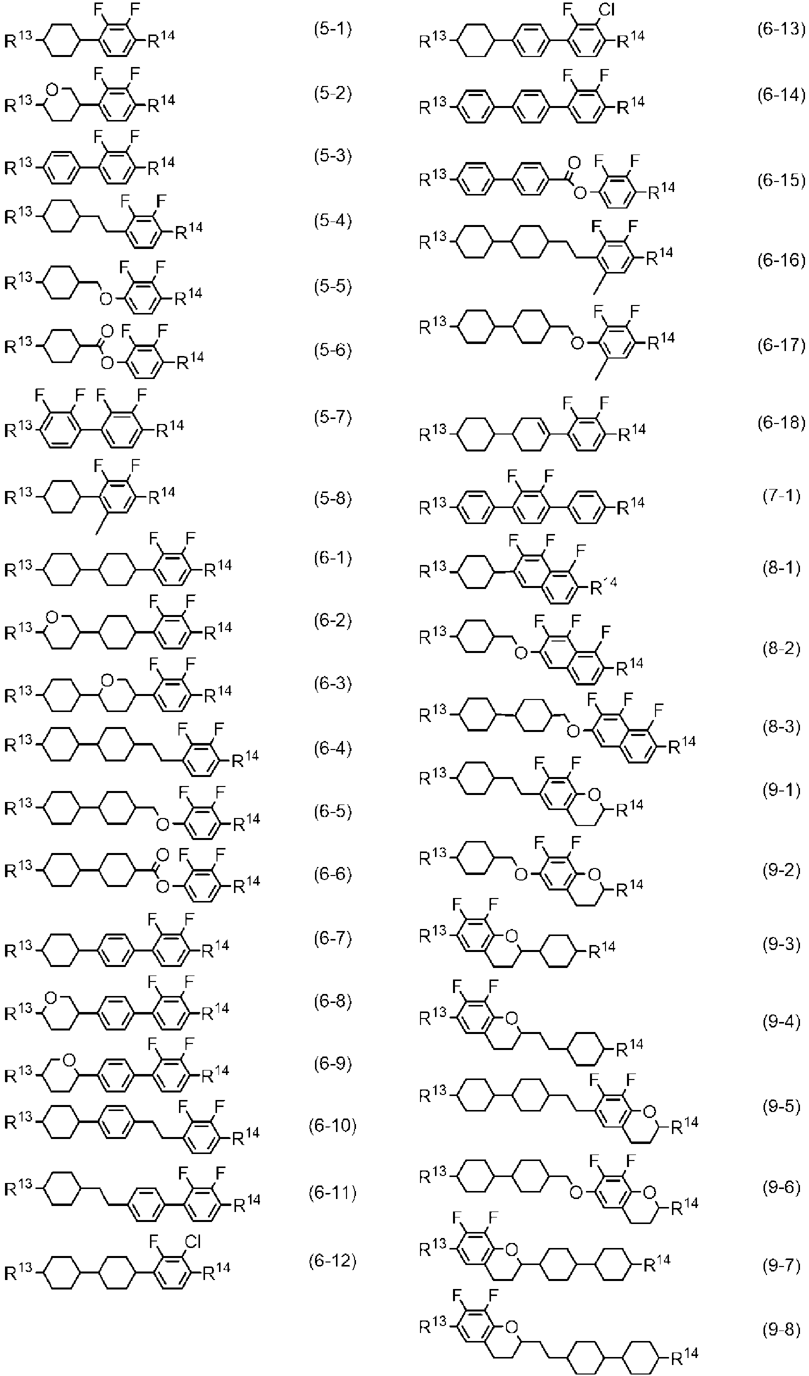

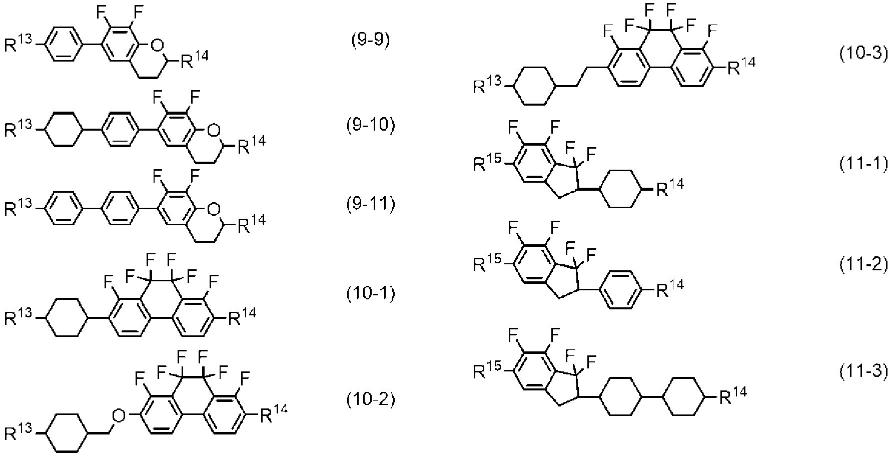

Component (c) has large negative dielectric anisotropy. Component (c) is used when a composition for a mode such as IPS, VA, and PSA is prepared. As the content of Component (c) increases, the dielectric anisotropy of the composition becomes negatively larger, but the viscosity increases. Therefore, the content is preferably as small as possible as long as a required value of a threshold voltage of the element is satisfied. In consideration of the fact that the dielectric anisotropy is about -5, in order for driving at a sufficient voltage, the content is preferably 40 weight% or more.

Among Components (c), since Compound (5) is a bicyclic compound, it has an effect of lowering the viscosity, adjusting the optical anisotropy, or increasing the dielectric anisotropy. Since Compounds (5) and (6) are tricyclic compounds, they have an effect of increasing the upper limit temperature, increasing the optical anisotropy, or increasing the dielectric anisotropy. Compounds (8) to (11) have an effect of increasing the dielectric anisotropy.

When a composition for a mode such as IPS, VA, and PSA is prepared, the content of Component (c) is preferably 40 weight% or more and more preferably in a range of 50 weight% to 95 weight% on the basis of the weight of the liquid crystal composition. When Component (c) is added to a composition having positive dielectric anisotropy, the content of Component (c) is preferably 30 weight% or less. When Component (c) is added, it is possible to adjust an elastic constant of the composition and adjust a voltage-transmittance curve of the element.

Component (d) is a compound having a halogen atom or a fluorine-containing group at the right terminal. Preferable examples of Component (d) include Compounds (12-1) to (12-16), Compounds (13-1) to (13-113), and Compounds (14-1) to (14-58). In these compounds, R¹⁶ represents an alkyl group having 1 to 10 carbon atoms or an alkenyl group having 2 to 10 carbon atoms, and in the alkyl and alkenyl groups, at least one -CH₂- is optionally substituted with -O-, and in these groups, at least one hydrogen atom is optionally substituted with a fluorine atom. X¹¹ represents a fluorine atom, a chlorine atom, -OCF₃, -OCHF₂, -CF₃, -CHF₂, -CH₂F, -OCF₂CHF₂, or -OCF₂CHFCF₃.

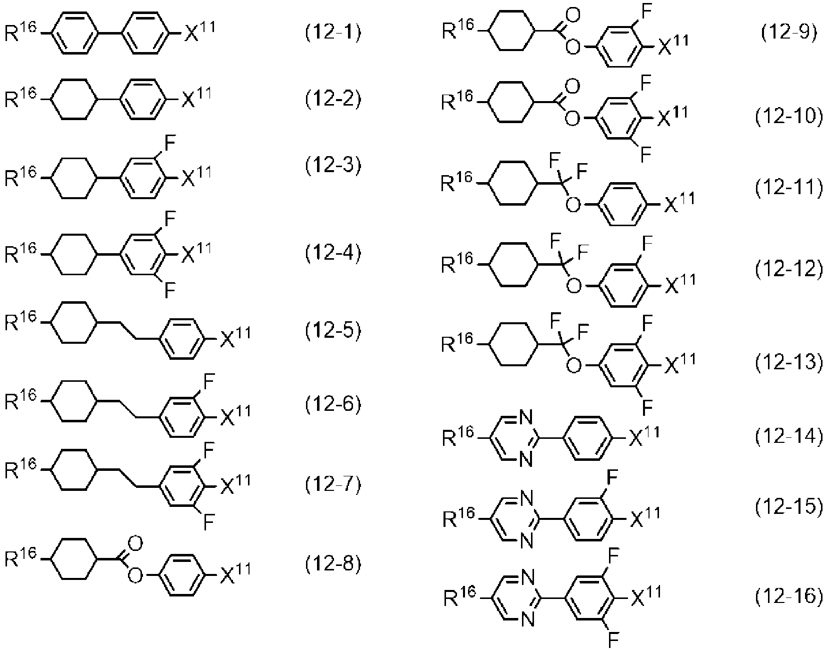

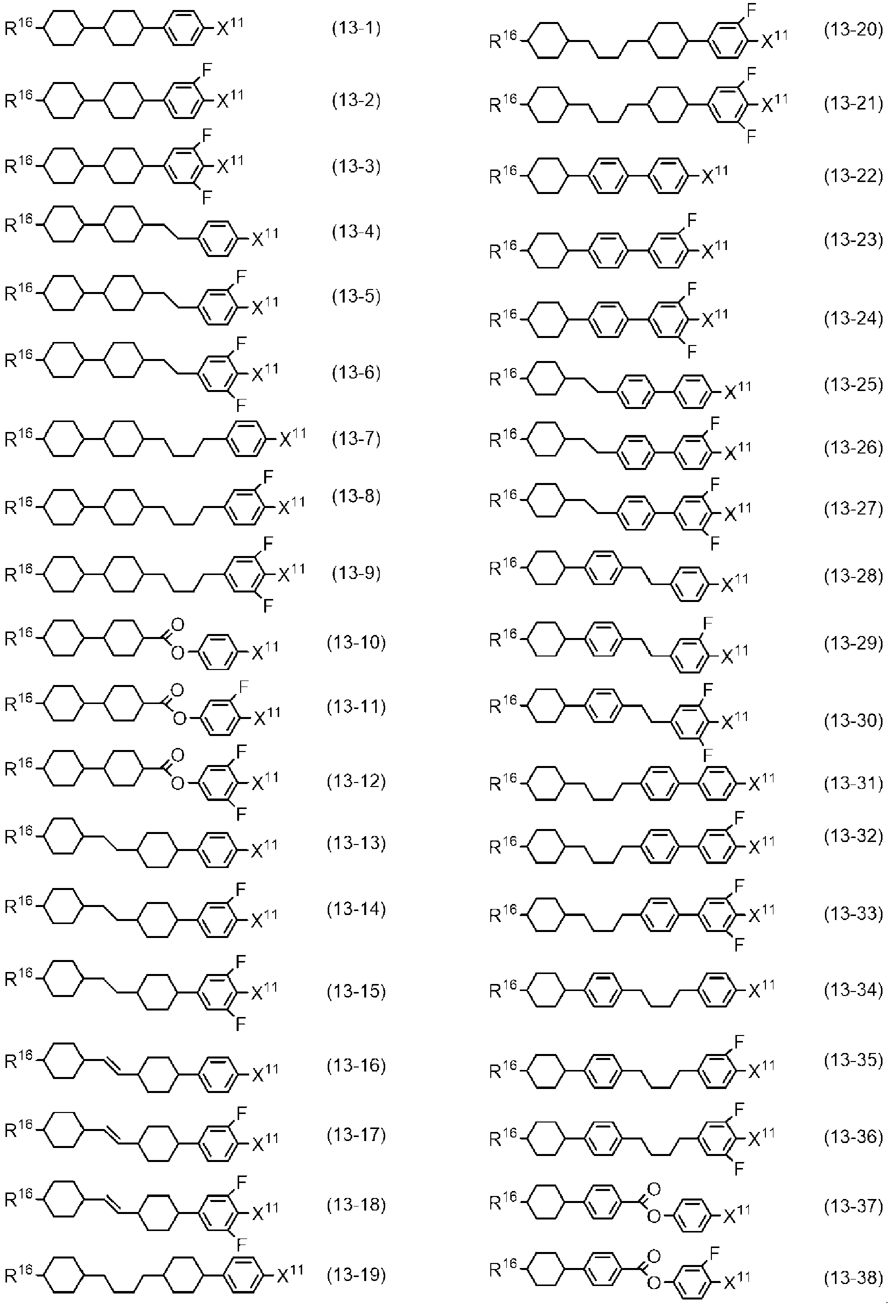

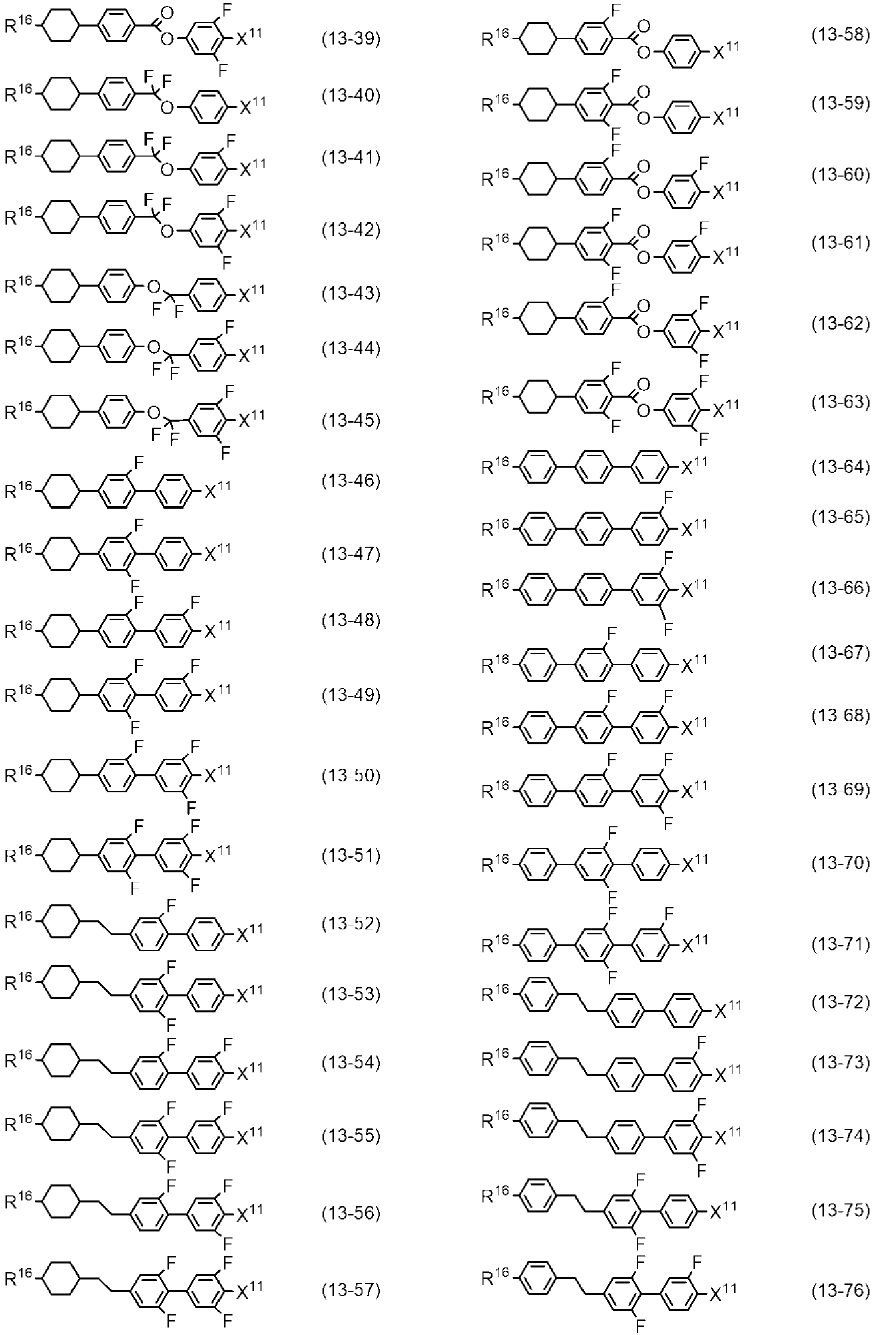

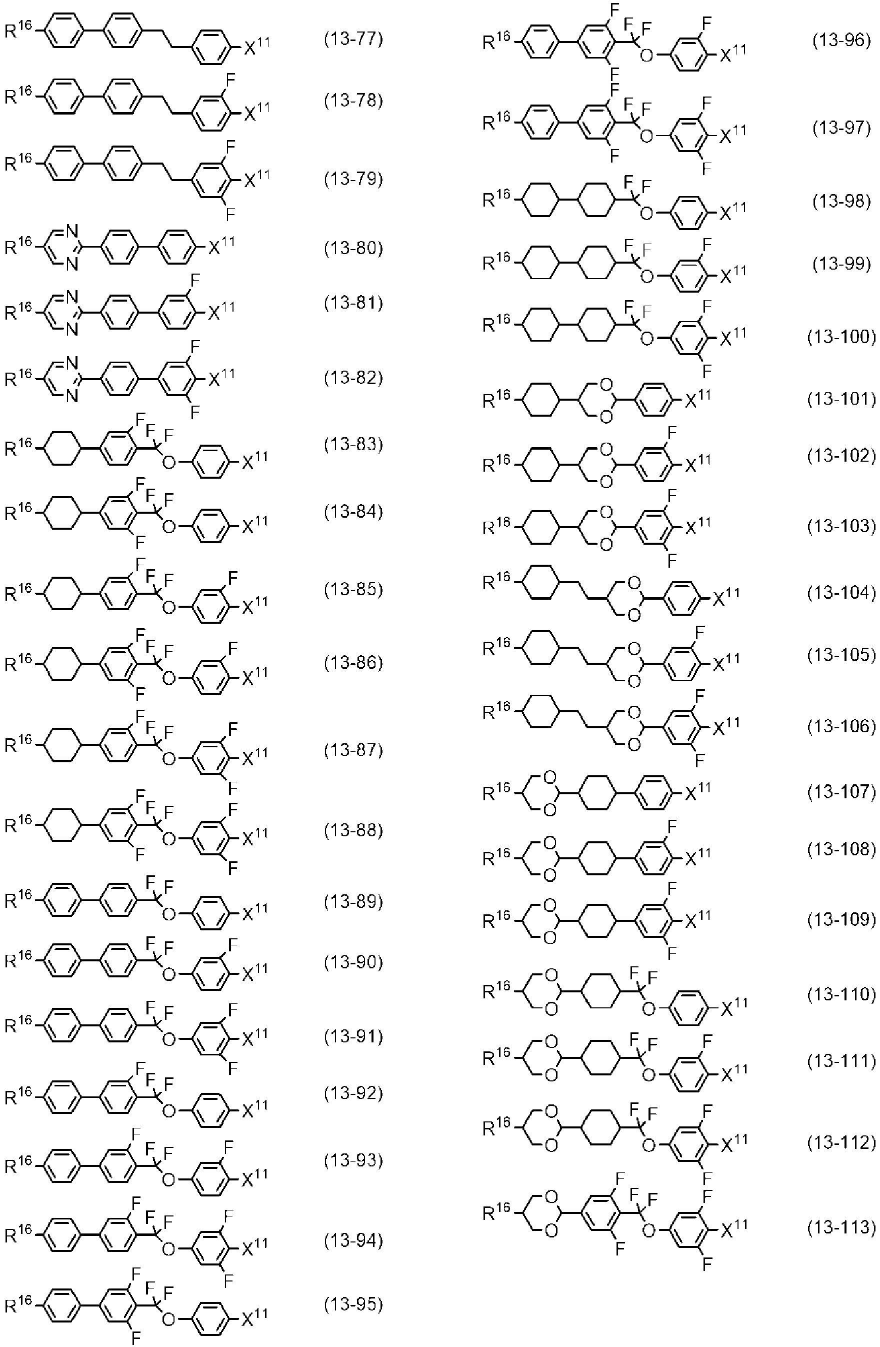

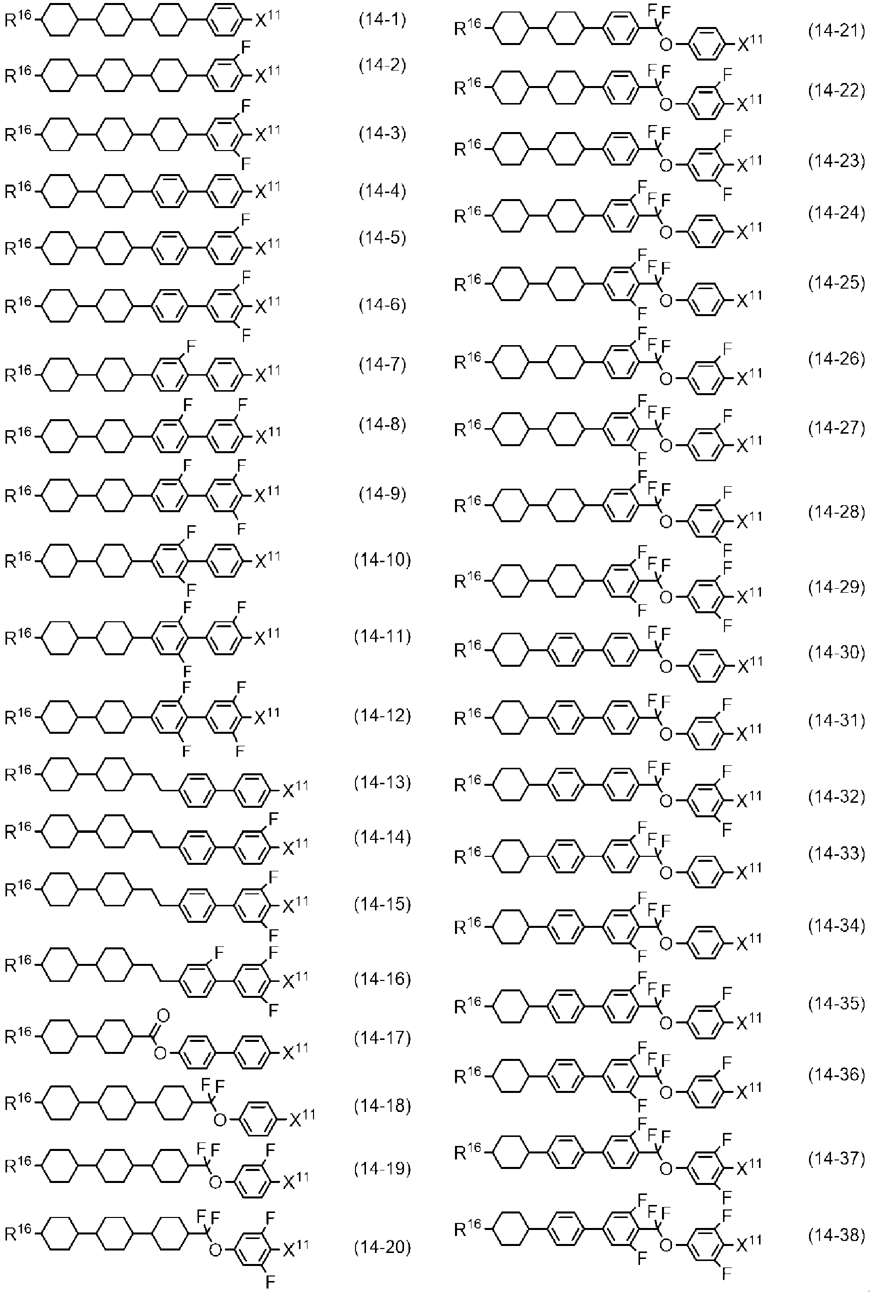

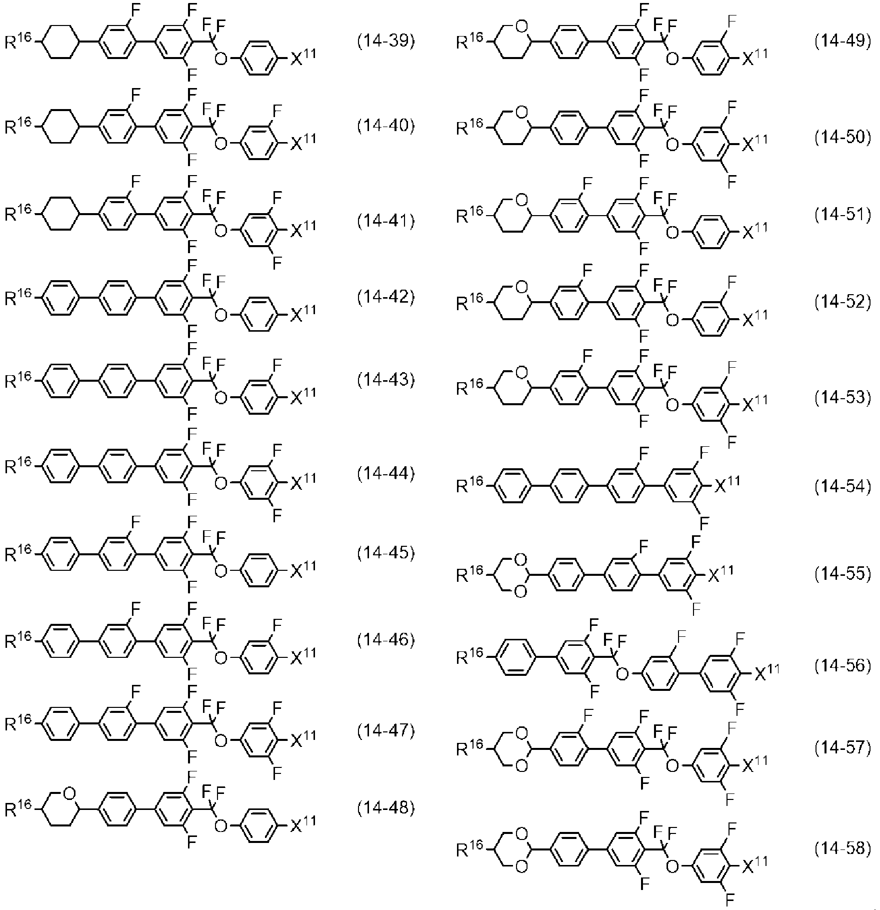

Since Component (d) has positive dielectric anisotropy and very favorable stability with respect to heat and light, it is used to prepare a composition for a mode such as IPS, FFS, and OCB. The content of Component (d) is suitably in a range of 1 weight% to 99 weight%, preferably in a range of 10 weight% to 97 weight%, and more preferably in a range of 40 weight% to 95 weight% on the basis of the weight of the liquid crystal composition. When Component (d) is added to a composition having negative dielectric anisotropy, the content of Component (d) is preferably 30 weight% or less. When Component (d) is added, it is possible to adjust an elastic constant of the composition and adjust a voltage-transmittance curve of the element

Component (e) is Compound (15) in which the right terminal group is -C≡N or -C≡C-C≡N. Preferable examples of Component (e) include Compounds (15-1) to (15-64). In these compounds, R¹⁷ is an alkyl group having 1 to 10 carbon atoms or an alkenyl group having 2 to 10 carbon atoms, and in these alkyl and alkenyl groups, at least one -CH₂- is optionally substituted with -O-, and in these groups, at least one hydrogen atom is optionally substituted with fluorine. X¹² is -C≡N or -C≡C-C≡N.

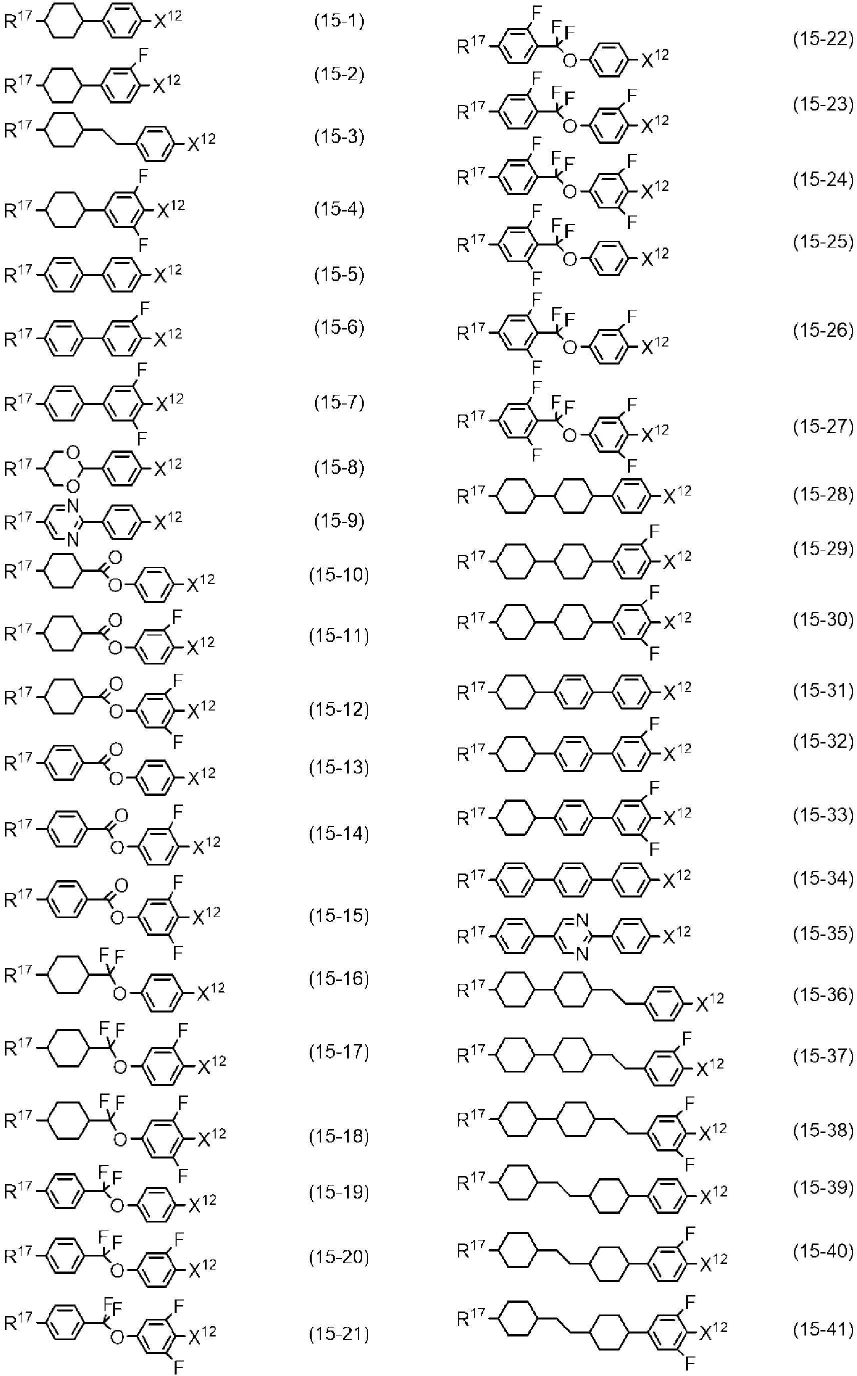

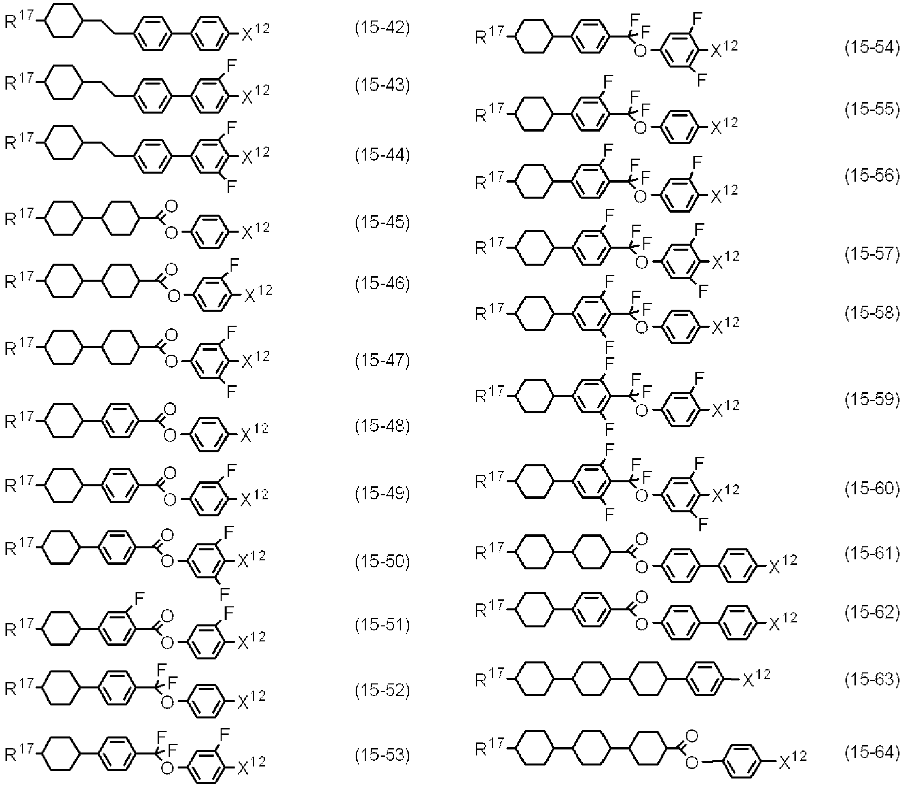

Since Component (e) has positive dielectric anisotropy and its value is large, it is used to prepare a composition for a mode such as TN. When Component (e) is added, it is possible to increase the dielectric anisotropy of the composition. Component (e) has an effect of widening a temperature range of the liquid crystal phase, adjusting the viscosity, or adjusting the optical anisotropy. Component (e) is also beneficial for adjusting a voltage-transmittance curve of the element.

When a composition for a mode such as TN is prepared, the content of Component (e) is suitably in a range of 1 weight% to 99 weight%, preferably in a range of 10 weight% to 97 weight%, and more preferably in a range of 40 weight% to 95 weight% on the basis of the weight of the liquid crystal composition. When Component (e) is added to a composition having negative dielectric anisotropy, the content of Component (e) is preferably 30 weight% or less. When Component (e) is added, it is possible to adjust an elastic constant of the composition and adjust a voltage-transmittance curve of the element.

When compounds that are appropriately selected from among the above Components (b) to (e) and Compound (1) are combined, it is possible to prepare a liquid crystal composition that has at least one of physical properties such as high stability with respect to heat and light, a high upper limit temperature, a low lower limit temperature, a low viscosity, suitable optical anisotropy (that is, large optical anisotropy or small optical anisotropy), large positive or negative dielectric anisotropy, high specific resistance, and a suitable elastic constant (that is, a large elastic constant or a small elastic constant). An element including such a composition has a wide temperature range in which the element can be used, a short response time, a high voltage holding ratio, a low threshold voltage, a large contrast ratio, a low flicker rate, and a prolonged lifespan.

When the element is used for a long time, flicker may occur on a display screen. The flicker rate (%) can be represented as (Iluminance when positive voltage is applied-luminance when negative voltage is applied|/average luminance) ×100. An element having a flicker rate in a range of 0% to 1% is less likely to cause flicker on the display screen even if the element is used for a long time. This flicker is related to image burning, and is assumed to be caused by a potential difference between a positive frame and a negative frame when the element is driven by an alternating current. A composition including Compound (1) is also beneficial for reducing the occurrence of flicker.

### 3-2. Additives

A liquid crystal composition is prepared by a known method. For example, component compounds are mixed, heated and dissolved together. According to applications, an additive may be added to the composition. Examples of the additive include a polymerizable compound, a polymerization initiator, a polymerization inhibitor, an optically active compound, an antioxidant, a UV absorber, a light stabilizer, a heat stabilizer, a dye, and an antifoaming agent. Such additives are well-known to those skilled in the art and described in documents.

In a liquid crystal display element having a polymer sustained alignment (PSA) mode, the composition includes a polymer. The polymerizable compound is added in order to form a polymer in the composition. While a voltage is applied between electrodes, when ultraviolet rays are emitted and the polymerizable compound is polymerized, the polymer is formed in the composition. According to this method, since a suitable pre-tilt is achieved, an element in which a response time is shortened and image burning is ameliorated is produced.

Preferable examples of the polymerizable compound include an acrylate, a methacrylate, a vinyl compound, a vinyloxy compound, propenyl ether, an epoxy compound (oxirane, oxetane), and vinyl ketone. More preferable examples include a compound having at least one acryloyloxy group and a compound having at least one methacryloyloxy group. Still more preferable examples include a compound having both an acryloyloxy group and a methacryloyloxy group.

More preferable examples include Compounds (M-1) to (M-18). In these compounds, R²⁵ to R³¹ are independently hydrogen or a methyl group; R³², R³³, and R³⁴ are independently hydrogen or an alkyl group having 1 to 5 carbon atoms, at least one of R³², R³³, and R³⁴ is an alkyl group having 1 to 5 carbon atoms; v, w, and x are independently 0 or 1; u and y are independently an integer of 1 to 10. L²¹ to L²⁶ are independently hydrogen or fluorine; L²⁷ and L²⁸ are independently hydrogen, fluorine, or a methyl group.

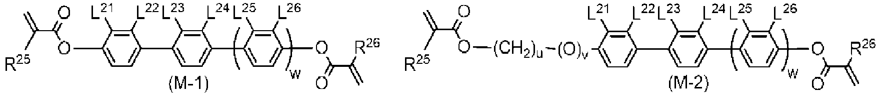

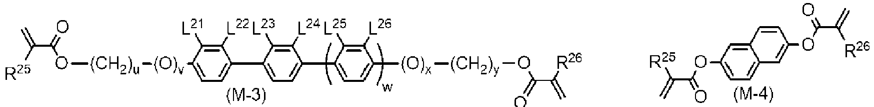

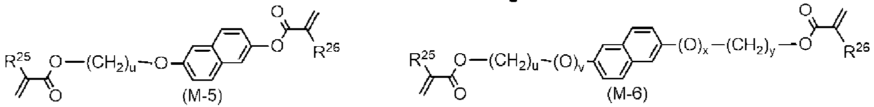

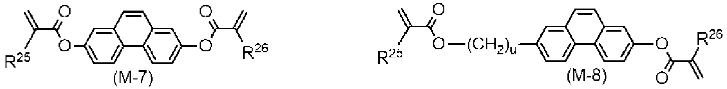

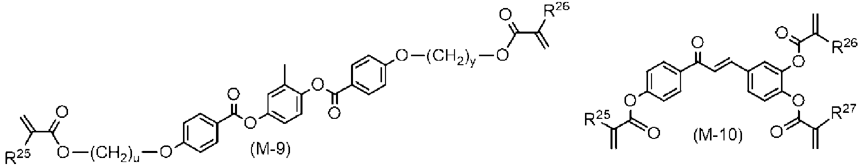

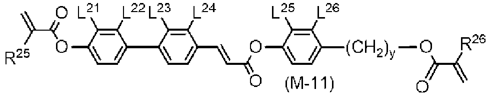

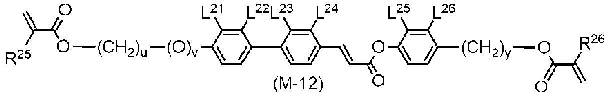

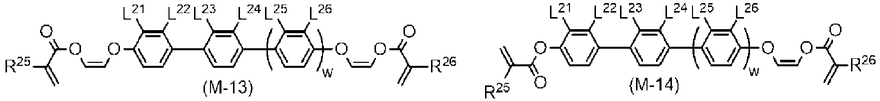

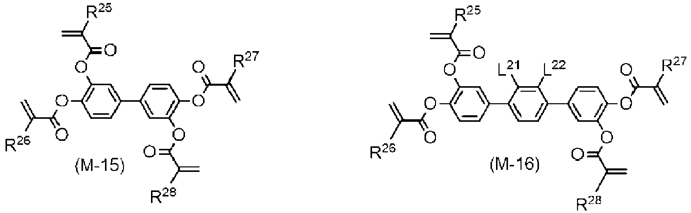

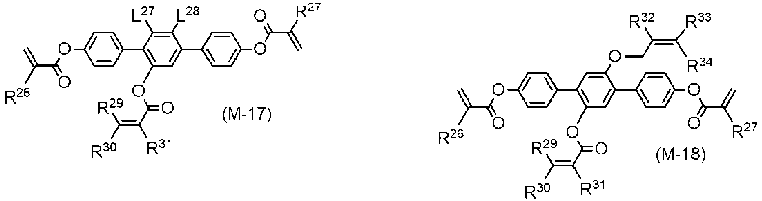

The polymerizable compound can be polymerized rapidly by adding a polymerization initiator. When reaction conditions are optimized, it is possible to reduce an amount of the polymerizable compound remaining. Examples of a photoradical polymerization initiator include Darocur series TPO, 1173, and 4265, and Irgacure series 184, 369, 500, 651, 784, 819, 907, 1300, 1700, 1800, 1850, and 2959 which are commercially available from BASF.

Additional examples of the photoradical polymerization initiator include 4-methoxyphenyl-2,4-bis(trichloromethyl)triazine, 2-(4-butoxystyryl)-5-trichloromethyl-1,3,4-oxadiazole, 9-phenylacridine, 9,10-benzphenazine, a benzophenone/Michler's ketone mixture, a hexaarylbiimidazole/mercaptobenzimidazole mixture, 1-(4-isopropylphenyl)-2-hydroxy-2-methylpropan-1-one, benzyldimethylketal, 2-methyl-1-[4-(methylthio)phenyl]-2-morpholinopropan-1-one, 2,4-diethyl xanthone/methyl p-dimethylaminobenzoate mixture, and a benzophenone/methyltriethanolamine mixture.

After a photoradical polymerization initiator is added to the liquid crystal composition, ultraviolet rays are emitted while an electric field is applied, and thus polymerization can be performed. However, unreacted polymerization initiator or decomposition products of a polymerization initiator may cause display defects such as image burning in the element. In order to prevent this, photopolymerization may be performed without adding a polymerization initiator. A preferable wavelength of light to be emitted is in a range of 150 nm to 500 nm. A more preferable wavelength is in a range of 250 nm to 450 nm, and a most preferable wavelength is in a range of 300 nm to 400 nm.

When the polymerizable compound is stored, a polymerization inhibitor may be added in order to prevent polymerization. Generally, the polymerizable compound is added to the composition when the polymerization inhibitor has not been removed. Examples of the polymerization inhibitor include hydroquinone derivatives such as hydroquinone and methyl hydroquinone, 4-t-butyl catechol, 4-methoxyphenol, and phenothiazine.

The optically active compound has an effect of inducing a helical structure in liquid crystal molecules, imparting a required helix angle, and thus preventing reverse twist. When the optically active compound is added, it is possible to adjust a helical pitch. Two or more optically active compounds may be added in order to adjust temperature dependence of the helical pitch. Preferable examples of the optically active compound include the following Compounds (Op-1) to (Op-18). In Compound (Op-18), a ring J is 1,4-cyclohexylene or 1,4-phenylene, and R²⁸ is an alkyl group having 1 to 10 carbon atoms. The symbol * indicates an asymmetric carbon atom.

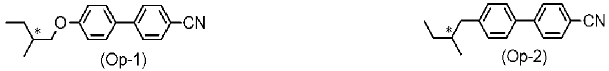

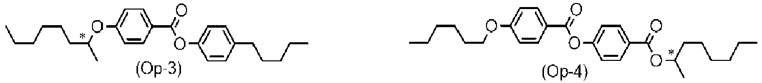

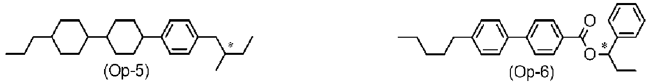

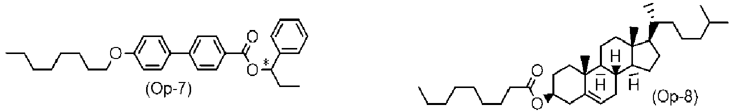

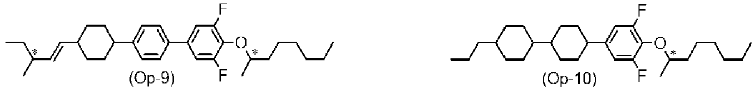

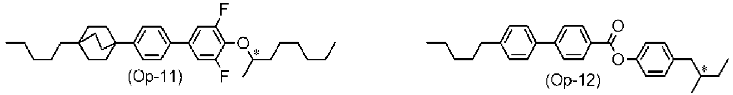

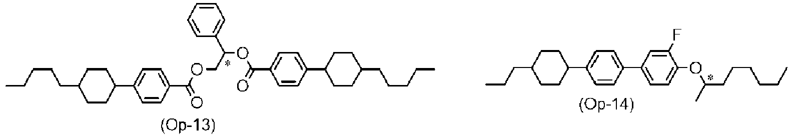

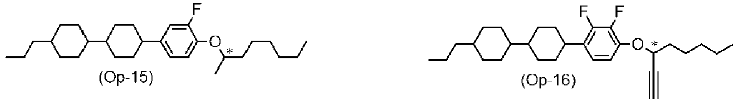

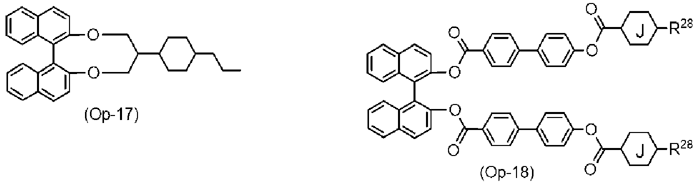

The antioxidant is effective for maintaining a high voltage holding ratio. Preferable examples of the antioxidant include the following Compounds (AO-1) and (AO-2); Irganox 415, Irganox 565, Irganox 1010, Irganox 1035, Irganox 3114, and Irganox 1098 (product name; commercially available from BASF). The UV absorber is effective for preventing the upper limit temperature from decreasing. Preferable examples of the UV absorber include benzophenone derivatives, benzoate derivatives, and triazole derivatives, and specific examples include the following Compounds (AO-3) and (AO-4); Tinuvin 329, Tinuvin P, Tinuvin 326, Tinuvin 234, Tinuvin 213, Tinuvin 400, Tinuvin 328, and Tinuvin 99-2 (product name; commercially available from BASF); and 1,4-diazabicyclo[2.2.2]octane (DABCO).

The light stabilizer such as a sterically hindered amine is preferably used to maintain a high voltage holding ratio. Preferable examples of the light stabilizer include the following Compounds (AO-5), (AO-6), and (AO-7); Tinuvin 144, Tinuvin 765, and Tinuvin 770DF (product name; commercially available from BASF); LA-77Y and LA-77G (product name; commercially available from ADEKA). The heat stabilizer is also effective for maintaining a high voltage holding ratio, and preferable examples thereof include Irgafos 168 (product name; commercially available from BASF). In order to adapt the composition to an element in a guest host (GH) mode, a dichroic dye such as an azo type dye or an anthraquinone type dye is added to the composition. The antifoaming agent is effective for preventing foaming. Preferable examples of the antifoaming agent include dimethyl silicone oil and methylphenyl silicone oil.

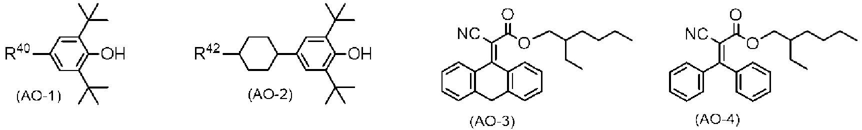

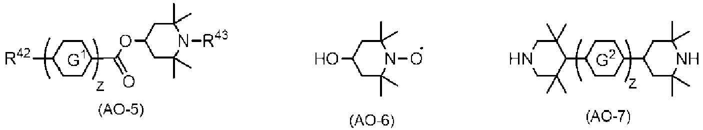

In Compound (AO-1), R⁴⁰ is an alkyl group having 1 to 20 carbon atoms, an alkoxy group having 1 to 20 carbon atoms, -COOR⁴¹, or -CH₂CH₂COOR⁴¹, here R⁴¹ is an alkyl group having 1 to 20 carbon atoms. In Compounds (AO-2) and (AO-5), R⁴² is an alkyl group having 1 to 20 carbon atoms. In Compound (AO-5), R⁴³ is hydrogen, a methyl group or O· (oxygen radical); a ring G¹ is 1,4-cyclohexylene or 1,4-phenylene; in Compound (AO-7), a ring G² is 1,4-cyclohexylene, 1,4-phenylene, or 1,4-phenylene in which at least one hydrogen atom is substituted with fluorine; in Compounds (AO-5) and (AO-7), z is 1, 2, or 3.

### 4. Liquid crystal display element

The liquid crystal composition has an operation mode such as PC, TN, STN, OCB, and PSA, and can be used for a liquid crystal display element that is driven by an active matrix method. The composition has an operation mode such as PC, TN, STN, OCB, VA, and IPS, and can also be used for a liquid crystal display element that is driven by a passive matrix method. These elements can be applied to any type of a reflective type, a transmission type, and a semi-transmission type.

The composition is also suitable for a nematic curvilinear aligned phase (NCAP) element, and here, the composition is microencapsulated. The composition can also be used for a polymer dispersion type liquid crystal display element (PDLCD) and a polymer network liquid crystal display element (PNLCD). In these compositions, a large amount of the polymerizable compound is added. On the other hand, when a proportion of the polymerizable compound is 10 weight% or less on the basis of the weight of the liquid crystal composition, a liquid crystal display element in a PSA mode is produced. A preferable proportion is in a range of 0.1 weight% to 2 weight%. A more preferable proportion is in a range of 0.2 weight% to 1.0 weight%. An element in a PSA mode can be driven by a driving method such as an active matrix method and a passive matrix method. Such an element can be applied to any type of a reflective type, a transmission type, and a semi-transmission type.

### Examples

### 1. Examples of Compound (1)

The present disclosure will be described in further detail with reference to examples. Since the examples are typical examples, the present disclosure is not limited by the examples. Compound (1) was synthesized by the following procedures. The synthesized compounds were identified by a method such as NMR analysis. Physical properties of compounds and compositions and characteristics of elements were measured by the following methods.

NMR analysis: a DRX-500 (commercially available from Bruker BioSpin) was used for measurement. In ¹H-NMR measurement, a sample was dissolved in a deuterated solvent such as CDCl₃ and measurement was performed under conditions of room temperature and 500 MHz with a cumulative number of 16 measurements. Tetramethylsilane was used as an internal reference. In ¹⁹F-NMR measurement, CFCl₃ was used as an internal reference and a cumulative number of measurements was 24. In the explanation of nuclear magnetic resonance spectrums, s is singlet, d is doublet, t is triplet, q is quartet, quin is quintet, sex is sextet, m is multiplet, and br is broad.

Gas chromatographic analysis: A GC-2010 type gas chromatography instrument (commercially available from Shimadzu Corporation) was used for measurement. As a column, a capillary column DB-1 (commercially available from Agilent Technologies Inc, with a length of 60 m, an inner diameter of 0.25 mm, and a film thickness of 0.25 µm) was used. Helium (1 mL/min) was used as a carrier gas. A temperature of a sample vaporization chamber was set to 300 °C, and a temperature of a detector (FID) was set to 300 °C. A sample was dissolved in acetone to prepare a 1 weight% solution, and 1 µl of the obtained solution was injected into the sample vaporization chamber. As a recorder, a GCSolution system (commercially available from Shimadzu Corporation) was used.

Gas chromatograph mass spectrometry: A QP-2010Ultra type gas chromatography mass spectrometer (commercially available from Shimadzu Corporation) was used for measurement. As a column, a capillary column DB-1 (commercially available from Agilent Technologies Inc, with a length of 60 m, an inner diameter of 0.25 mm, and a film thickness of 0.25 µm) was used. Helium (1 ml / min) was used as a carrier gas. A temperature of a sample vaporization chamber was set to 300 °C, a temperature of an ion source was set to 200 °C, an ionization voltage was set to 70 eV, and an emission current was set to 150 uA. A sample was dissolved in acetone to prepare a 1 weight% solution, and 1 µl of the obtained solution was injected into the sample vaporization chamber. As a recorder, a GCMS solution system (commercially available from Shimadzu Corporation) was used.

HPLC analysis: a Prominence (LC-20AD; SPD-20A, commercially available from Shimadzu Corporation) was used for measurement. As a column, a YMC-Pack ODS-A (with a length of 150 mm, an inner diameter of 4.6 mm, and a particle size of 5 µm, commercially available from YMC Co., Ltd.) was used. As an eluent, acetonitrile and water were appropriately mixed and used. As a detector, a UV detector, an RI detector, a CORONA detector, or the like was appropriately used. When a UV detector was used, a detection wavelength was set to 254 nm. A sample was dissolved in acetonitrile to prepare a 0.1 weight% solution and 1 µL of the solution was introduced into a sample chamber. As a recorder, a C-R7Aplus (commercially available from Shimadzu Corporation) was used.

UV-visible spectroscopic analysis: a PharmaSpec UV-1700 (commercially available from Shimadzu Corporation) was used for measurement. A detection wavelength of 190 nm to 700 nm was set. A sample was dissolved in acetonitrile to prepare a 0.01 mmol/L solution, and put into a quartz cell (optical path length of 1 cm) for measurement.

Measurement sample: When a phase structure and a transition temperature (a clearing point, a melting point, a polymerization initiation temperature, etc.) were measured, a compound itself was used as a sample. When physical properties such as an upper limit temperature of a nematic phase, a viscosity, optical anisotropy, and dielectric anisotropy were measured, a mixture of a compound and a mother liquid crystal was used as a sample.

When a sample in which a compound was mixed with a mother liquid crystal was used, measurement was performed as follows. 15 weight% of a compound and 85 weight% of a mother liquid crystal were mixed to prepare a sample. An extrapolation value was computed from measured values of this sample according to the following equation and this value was stated.

### <Extrapolation value>=(100×<measured value of sample>-<weight% of mother liquid crystal>×<measured value of mother liquid crystal>)/<weight% of compound>

When a crystal (or a smectic phase) was precipitated at 25 °C at this proportion, a ratio between the compound and the mother liquid crystal was changed to 10 weight%:90 weight%, 5 weight%:95 weight%, and 3 weight%:97 weight% in that order, and physical properties of the sample at a proportion at which no crystals (or a smectic phase) precipitated at 25 °C were measured. Here, unless otherwise specified, a ratio between the compound and the mother liquid crystal was 15 weight%:85 weight%.

When the dielectric anisotropy of the compound was zero or positive, the following mother liquid crystal (A) was used. Proportions of components are expressed as weight%.

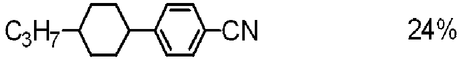

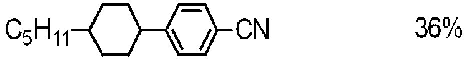

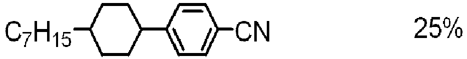

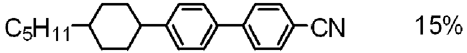

When the dielectric anisotropy of the compound was zero or negative, the following mother liquid crystal (B) was used. Proportions of components are expressed as weight%.

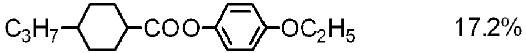

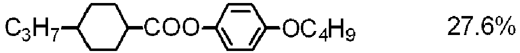

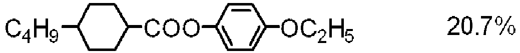

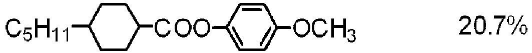

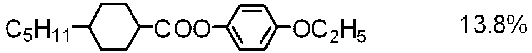

Measurement method: Physical properties were measured by the following methods. Most of these are described in JEITA standards (JEITA·ED-2521B) discussed and established by the Japan Electronics and Information Technology Industries Association (JEITA). Modified methods were also used. No thin film transistor (TFT) was attached to a TN element used for measurement.
(1) Phase structure: A sample was placed on a hot plate (FP-52 type hot stage commercially available from Mettler) of a melting point measuring device including a polarization microscope. While this sample was heated at a speed of 3 °C/min, a phase state and a change thereof were observed under a polarization microscope, and a type of the phase was identified.
(2) Transition temperature (°C): A scanning calorimeter (commercially available from PerkinElmer) and a Diamond DSC system or a high sensitivity differential scanning calorimeter X-DSC7000 (commercially available from SII NanoTechnology Inc.) were used for measurement. The temperature of the sample was raised or lowered at a speed of 3 °C/min, a starting point of an endothermic peak or an exothermic peak according to a phase change in the sample was obtained by extrapolation, and a transition temperature was determined. A melting point and a polymerization initiation temperature of the compound were measured using this device. A temperature at which the compound transitions from a solid phase to a liquid crystal phase such as a smectic phase or a nematic phase may be abbreviated as a "lower limit temperature of a liquid crystal phase." A temperature at which the compound transitions from a liquid crystal phase to a liquid may be abbreviated as a "clearing point."
   A crystal is represented as C. When two types of crystal are distinguished, they are denoted as C₁ and C₂. The smectic phase is represented by S and the nematic phase is represented as N. When phases are distinguished as a smectic A phase, a smectic B phase, a smectic C phase, and a smectic F phase, they are represented as S_{A}, S_{B}, S_{C}, and S_{F}, respectively. A liquid (isotropic) is represented as I. The transition temperature is expressed as, for example, "C 50.0 N 100.0 I." This indicates that a transition temperature from a crystal to a nematic phase is 50.0 °C, and a transition temperature from a nematic phase to a liquid is 100.0 °C.
(3) Compatibility of compound: A mother liquid crystal and a compound were mixed such that a proportion of the compound was 20 weight%, 15 weight%, 10 weight%, 5 weight%, 3 weight%, or 1 weight% and thereby a sample was prepared. The sample was put into a glass bottle, and stored in a freezing chamber at -10 °C or -20 °C for a certain time. It was observed whether a nematic phase of the sample was maintained or a crystal (or a smectic phase) precipitated. Conditions in which maintain nematic phase were used as a measure of compatibility. A proportion of the compound and the temperature of the freezing chamber were changed as necessary.
(4) Upper limit temperature (T_{NI} or NI; °C) of nematic phase: A sample was placed on a hot plate of a melting point measuring device including a polarization microscope and heated at a speed of 1 °C/min. A temperature at which a part of the sample changed from a nematic phase to an isotropic liquid was measured. When a sample was a mixture of Compound (1) and a mother liquid crystal, the sample is indicated by a symbol T_{NI}. When a sample is a mixture of Compound (1) and a compound selected from among Compounds (2) to (15), the sample is indicated by a symbol NI. An upper limit temperature of the nematic phase may be abbreviated as an "upper limit temperature."
(5) Lower limit temperature (T_{C};°C) of nematic phase: A sample having a nematic phase was put into a glass bottle and stored in a freezer at 0 °C, -10 °C, -20 °C, -30 °C, and -40 °C for 10 days, and then a liquid crystal phase was observed. For example, when the sample remained in a nematic phase at -20 °C, and changed to a crystal or a smectic phase at -30 °C, T_{C} is described as <-20 °C. A lower limit temperature of the nematic phase may be abbreviated as a "lower limit temperature."
(6) Viscosity (bulk viscosity; η; measured at 20 °C; mPa·s): An E type rotational viscometer (commercially available from Tokyo Keiki) was used for measurement.
(7) Optical anisotropy (refractive index anisotropy; measured at 25 °C; Δn): Measurement was performed by an Abbe refractometer in which a polarizing plate was attached to an eyepiece using light with a wavelength of 589 nm. A surface of a main prism was rubbed in one direction and the sample was then added dropwise onto a main prism. A refractive index (n∥) was measured when a direction of polarized light was parallel to a rubbing direction. A refractive index (n⊥) was measured when a direction of polarized light was perpendicular to a rubbing direction. A value of optical anisotropy (Δn) was calculated from the equation Δn=n∥-n⊥.
(8) Specific resistance (ρ; measured at 25 °C; Ωcm): 1.0 mL of a sample was injected into a container including an electrode. A DC voltage (10 V) was applied to this container and a direct current was measured after 10 seconds. A specific resistance was computed from the following equation. (Specific resistance)={(voltage)×(electric capacitance of container)}/{(direct current)×(dielectric constant of vacuum)}.
(9) Voltage holding ratio (VHR-1; measured at 25 °C; %): A TN element used for measurement included a polyimide alignment film, and an interval (cell gap) between two glass substrates was 5 µm. This element was sealed with an adhesive that was cured with ultraviolet rays after the sample was inserted. A pulse voltage (at 5 V for 60 microseconds) was applied to this element for charging. An attenuating voltage was measured for 16.7 milliseconds by a high-speed voltmeter, and an area A between a voltage curve in a unit cycle and the horizontal axis was obtained. An area B was an area when the voltage was not attenuated. A voltage holding ratio was expressed as a percentage of the area A with respect to the area B.
(10) Voltage holding ratio (VHR-2; measured at 80 °C; %): A voltage holding ratio was measured in the same manner as in the above method except that the voltage holding ratio was measured at 80 °C instead of 25 °C. The obtained result was indicated by a symbol VHR-2.
(11) Flicker rate (measured at 25 °C; %): A multimedia display tester 3298F (commercially available from Yokogawa Electric Corporation) was used for measurement. A light source was an LED. An interval (cell gap) between two glass substrates was 3.5 µm, and a sample was inserted into an FFS element in a normally black mode in which a rubbing direction was antiparallel. This element was sealed using an adhesive that was cured with ultraviolet rays. A voltage was applied to this element, and a voltage at which a quantity of light that had passed through the element was a maximum was measured. A sensor part was brought close to the element while the voltage was applied to the element, and a displayed flicker rate was read.
   A physical property measurement method was different between a sample having positive dielectric anisotropy and a sample having negative dielectric anisotropy. A measurement method for positive dielectric anisotropy will be described from measurement (12a) to measurement (16a). A measurement method in negative dielectric anisotropy will be described from measurement (12b) to measurement (16b).
(12a) Viscosity (rotational viscosity; γ1; measured at 25 °C; mPa·s; sample with positive dielectric anisotropy): Measurement was performed according to a method described in M. Imai et al., Molecular Crystals and Liquid Crystals, Vol. 259, 37 (1995). A sample was inserted into a TN element in which a twist angle was 0 degrees and an interval (cell gap) between two glass substrates was 5 µm. Voltages from 16 V to 19.5 V were gradually applied at 0.5 V intervals to this element. After no voltage was applied for 0.2 seconds, application was repeated under conditions of one square wave (square pulse; 0.2 seconds) and no voltage application (2 seconds). A peak current and a peak time of a transient current generated by this application were measured. A value of the rotational viscosity was obtained from these measured values and Equation (8) on page 40 in the paper (M. Imai). A value of the dielectric anisotropy necessary for this calculation was obtained by the method described below using an element for which the rotational viscosity was measured.
(12b) Viscosity (rotational viscosity; γ1; measured at 25 °C; mPa·s; sample with negative dielectric anisotropy): Measurement was performed according to a method described in M. Imai et al., Molecular Crystals and Liquid Crystals, Vol. 259, 37 (1995). A sample was inserted into a VA element in which an interval (cell gap) between two glass substrates was 20 µm. Voltages from 39 V to 50 V were gradually applied at 1 V intervals to this element. After no voltage was applied for 0.2 seconds, application was repeated under conditions of one square wave (square pulse; 0.2 seconds) and no voltage application (2 seconds). A peak current and a peak time of a transient current generated by this application were measured. A value of the rotational viscosity was obtained from these measured values and Equation (8) on page 40 in the paper (M. Imai). The dielectric anisotropy required for this calculation was measured in the following dielectric anisotropy section.
(13a) Dielectric anisotropy (Δε; measured at 25 °C; sample with positive dielectric anisotropy): A sample was inserted into a TN element in which an interval (cell gap) between two glass substrates was 9 µm and a twist angle was 80 degrees. A sine wave (10 V, 1 kHz) was applied to this element, and after 2 seconds, a dielectric constant (ε∥) in a long axis direction of liquid crystal molecules was measured. A sine wave (0.5 V, 1 kHz) was applied to this element, and after 2 seconds, a dielectric constant (ε⊥) in a short axis direction of liquid crystal molecules was measured. A value of the dielectric anisotropy was calculated from the equation Δε=ε∥-ε⊥.
(13b) Dielectric anisotropy (Δε; measured at 25 °C; sample with negative dielectric anisotropy): A value of the dielectric anisotropy was calculated from the equation Δε=ε∥-ε⊥. A dielectric constant (ε∥ and ε⊥) was measured as follows.
   1) Measurement of dielectric constant (ε∥): An ethanol (20 mL) solution containing octadecyltriethoxysilane (0.16 mL) was applied to a well-washed glass substrate. The glass substrate was rotated by a spinner and then heated at 150 °C for 1 hour. A sample was inserted into a VA element in which an interval (cell gap) between two glass substrates was 4 µm, and this element was sealed using an adhesive that was cured with ultraviolet rays. A sine wave (0.5 V, 1 kHz) was applied to this element and after 2 seconds, a dielectric constant (ε∥) in a long axis direction of liquid crystal molecules was measured.
   2) Measurement of dielectric constant (ε⊥): A polyimide solution was applied to a well-washed glass substrate. The glass substrate was fired and a rubbing treatment was then performed on the obtained alignment film. A sample was inserted into a TN element in which an interval (cell gap) between two glass substrates was 9 µm and a twist angle was 80 degrees. A sine wave (0.5 V, 1 kHz) was applied to this element, and after 2 seconds, a dielectric constant (ε⊥) in a short axis direction of liquid crystal molecules was measured.
(14a) Elastic constant (K; measured at 25 °C; pN; sample with positive dielectric anisotropy): A HP4284A type LCR meter (commercially available from Yokogawa-Hewlett-Packard Company) was used for measurement. A sample was inserted into a horizontally aligned element in which an interval (cell gap) between two glass substrates was 20 µm. A charge of 0 V to 20 V was applied to this element, and an electrostatic capacitance (C) and an applied voltage (V) were measured. These measured values were fitted using Equation (2.98) and Equation (2.101) on page 75 in "Liquid Crystal Device Handbook" (commercially available from Nikkan Kogyo Shimbun, Ltd.), and values of K₁₁ and K₃₃ were obtained from Equation (2.99). Next, in Equation (3.18) on page 171, K₂₂ was computed using the values of K₁₁ and K₃₃ obtained above. An elastic constant K was expressed as an average value of K₁₁, K₂₂, and K₃₃ obtained in this manner.
(14b) Elastic constant (K₁₁ and K₃₃; measured at 25 °C; pN; sample with negative dielectric anisotropy): An EC-1 type elastic constant measuring instrument (commercially available from TOYO Corporation) was used for measurement. A sample was inserted into a vertically aligned element in which an interval (cell gap) between two glass substrates was 20 µm. A charge of 20 V to 0 V was applied to this element, and an electrostatic capacitance (C) and an applied voltage (V) were measured. These values were fitted using Equation (2.98), and Equation (2.101) on page 75 in "Liquid Crystal Device Handbook" (commercially available from Nikkan Kogyo Shimbun, Ltd.) and a value of the elastic constant was obtained from Equation (2.100).
(15a) Threshold voltage (Vth; measured at 25 °C; V; sample with positive dielectric anisotropy): An LCD5100 type luminance meter (commercially available from Otsuka Electronics) was used for measurement. A light source was a halogen lamp. A sample was inserted into a TN element in a normally white mode in which an interval (cell gap) between two glass substrates was 0.45/Δn (µm) and a twist angle was 80 degrees. A voltage (32 Hz, square wave) applied to this element was gradually increased by 0.02 V from 0 V to 10 V. In this case, light was emitted to the element in a vertical direction and a quantity of light that had passed through the element was measured. A voltage-transmittance curve in which the transmittance was 100% when the quantity of light was a maximum and the transmittance was 0% when the quantity of light was a minimum was created. A threshold voltage was a voltage when the transmittance was 90%.
(15b) Threshold voltage (Vth; measured at 25 °C; V; sample with negative dielectric anisotropy): An LCD5100 type luminance meter (commercially available from Otsuka Electronics) was used for measurement. A light source was a halogen lamp. A sample was inserted into a VA element in a normally black mode in which an interval (cell gap) between two glass substrates was 4 µm and a rubbing direction was antiparallel, and this element was sealed using an adhesive that was cured with ultraviolet rays. A voltage (60 Hz, square wave) applied to this element was gradually increased by 0.02 V from 0 V to 20 V. In this case, light was emitted to the element in a vertical direction and a quantity of light that had passed through the element was measured. A voltage-transmittance curve in which the transmittance was 100% when the quantity of light was a maximum and the transmittance was 0% when the quantity of light was a minimum was created. A threshold voltage was a voltage when the transmittance was 10%.
(16a) Response time (τ; measured at 25 °C; ms; sample with positive dielectric anisotropy): An LCD5100 type luminance meter (commercially available from Otsuka Electronics) was used for measurement. A light source was a halogen lamp. A low-pass filter was set at 5 kHz. A sample was inserted into a TN element in a normally white mode in which an interval (cell gap) between two glass substrates was 5.0 µm and a twist angle was 80 degrees. A square wave (60 Hz, 5 V, 0.5 seconds) was applied to this element. In this case, light was emitted to the element in a vertical direction and a quantity of light that had passed through the element was measured. The transmittance was 100% when the quantity of light was a maximum, and the transmittance was 0% when the quantity of light was a minimum. A rise time (τr; millisecond) was a time required for the transmittance to change from 90% to 10%. A fall time (τf; millisecond) was a time for the transmittance to change from 10% to 90%. A response time was a sum of the rise time and the fall time obtained in this manner.
(16b) Response time (τ; measured at 25 °C; ms; sample with negative dielectric anisotropy): An LCD5100 type luminance meter (commercially available from Otsuka Electronics) was used for measurement. A light source was a halogen lamp. A low-pass filter was set at 5 kHz. A sample was inserted into a VA element in a normally black mode in which an interval (cell gap) between two glass substrates was 3.2 µm and a rubbing direction was antiparallel. This element was sealed using an adhesive that was cured with ultraviolet rays. A voltage that was slightly higher than a threshold voltage was applied to this element for 1 minute, and next ultraviolet rays of 23.5 mW/cm² were emitted for 8 minutes while a voltage of 5.6 V was applied. A square wave (60 Hz, 10 V, 0.5 seconds) was applied to this element. In this case, light was emitted to the element in a vertical direction and a quantity of light that had passed through the element was measured. The transmittance was 100% when the quantity of light was a maximum, and the transmittance was 0% when the quantity of light was a minimum. A response time was a time (fall time; milliseconds) required for the transmittance to change from 90% to 10%.

### [Synthesis Example 1]

### Synthesis of Compound (No. 26)

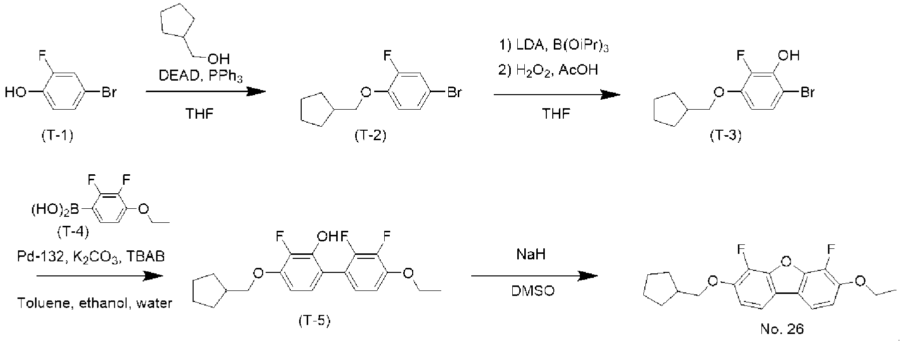

### First process: Synthesis of Compound (T-2)

Under a nitrogen atmosphere, Compound (T-1) (16.0 g), cyclopentyl methanol (8.39 g), triphenyl phosphine (26.4 g), and tetrahydrofuran (THF, 150 mL) were put into a reactor and cooled on an ice bath. Diethyl azodicarboxylate (DEAD, 2.2 M; toluene solution;45.7 ml) was added thereto and the mixture was stirred at room temperature for 8 hours. After the reaction was completed, the reaction mixture was poured into water and an aqueous layer was extracted in toluene. The combined organic layer was washed with a saline and then dried with anhydrous magnesium sulfate and concentrated under a reduced pressure. The residue was purified through silica gel chromatography (volume ratio, toluene: heptane=1:4), and thereby Compound (T-2) (19.8 g; 86%) was obtained.

### Second process: Synthesis of Compound (T-3)

Under a nitrogen atmosphere, Compound (T-2) (19.8 g) and tetrahydrofuran (THF, 200 mL) were put into a reactor and cooled to -70 °C. Lithium diisopropylamide (LDA, 1.1 M; n-hexane-tetrahydrofuransolution;73.0 mL) was added thereto and the mixture was stirred for 1 hour. Triisopropyl borate (8.9 ml, 79.5 mmol) was added thereto, the temperature returned to room temperature, and the mixture was stirred for 12 hours. Acetic acid (8.3 ml) was added thereto at room temperature, the mixture was stirred for 30 minutes, and a hydrogen peroxide solution (50 wt%; 8.9 ml) was then added thereto, and the mixture was stirred for 1 hour. After the reaction was completed, the reaction mixture was poured into water, and an aqueous layer was extracted in toluene. The combined organic layer was washed with water, saturated sodium thiosulfate aqueous solution water and a saline and then dried with anhydrous magnesium sulfate, and concentrated under a reduced pressure. The residue was purified through silica gel chromatography (volume ratio, ethyl acetate:heptane=1:4), and thereby Compound (T-3) (12.5 g; 59%) was obtained.

### Third process: Synthesis of Compound (T-5)

Under a nitrogen atmosphere, Compound (T-3) (12.5 g), Compound (T-4) (9.1 g), toluene (125 ml), ethanol (125 ml), water (125 ml), PdCl₂(Amphos)₂(Pd-132, 0.03 g), potassium carbonate (11.9 g), and tetrabutylammonium bromide (TBAB, 3.5 g) were put into a reactor and stirred at 72 °C for 4 hours. The reaction mixture was poured into water, extracted in toluene, and washed with water and a saline and then dried with anhydrous magnesium sulfate and concentrated under a reduced pressure. The residue was purified through silica gel chromatography (volume ratio, ethyl acetate:heptane=1:2), and thereby Compound (T-5) (13.0 g; 78%) was obtained.

### Fourth process: Synthesis of Compound (No. 26)

Under a nitrogen atmosphere, Compound (T-5) (13.0 g), sodium hydride (1.7 g), and dimethyl sulfoxide (DMSO, 130 mL) were put into a reactor and stirred at 120 °C for 4 hours. The reaction mixture was poured into water, extracted in toluene, and washed with a saline, and then dried with anhydrous magnesium sulfate and concentrated under a reduced pressure. The residue was purified through silica gel chromatography (volume ratio, toluene:heptane=2:1). In addition, purification was performed by re-crystallization from a solvent in which SOLMIX (registered trademark) A-11 and heptane were mixed (volume ratio, 1:1), and thereby Compound (No. 26) (6.0 g; 48%) was obtained.

¹H-NMR (CDCl₃; δppm): 7.48-7.45(m, 2H), 6.99-6.96(m, 2H), 4.20(q, J=7.0 Hz, 2H), 4.00(d, J=7.0 Hz, 2H), 2.48-2.39(m, 1H), 1.91-1.85(m, 2H), 1.70-1.58(m, 4H), 1.49(t, J=7.0 Hz, 3H), 1.45-1.36(m, 2H). ¹⁹F-NMR (δ ppm; CDCl₃): -157.91(d, J=7.3 Hz, IF), -158.10(d, J=7.0 Hz, 1F).

Phase transition temperature: C 103.9 I. upper limit temperature (NI)=26.3 °C; dielectric anisotropy (Δε) =-9.4; optical anisotropy (Δn) =0.200; viscosity (η)=69.6 mPa·s.; measured concentration: 5 weight%.

### [Synthesis Example 2]

### Synthesis of Compound (No. 3)

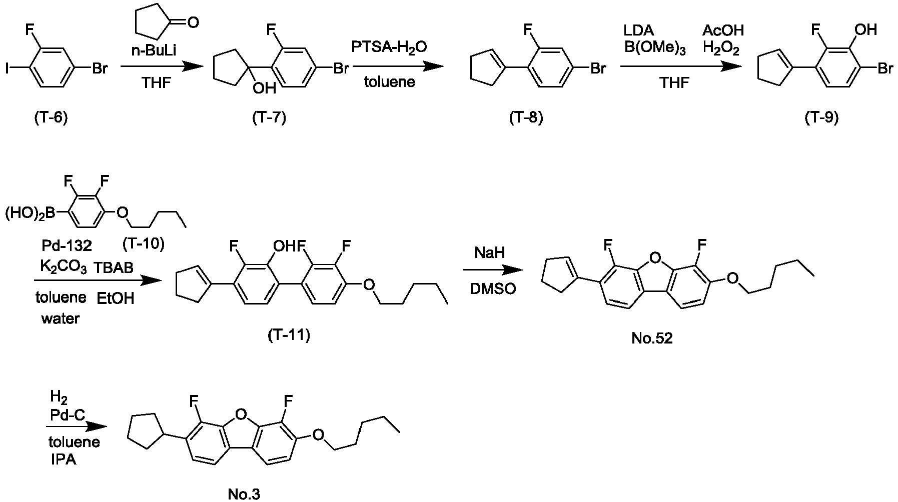

### First process: Synthesis of Compound (T-7)

Under a nitrogen atmosphere, Compound (T-6) (60.0 g), and tetrahydrofuran (500 mL) were put into a reactor and cooled to -70 °C. n-Butyl lithium (1.64 M; n-hexane solution; 127.7 ml) was slowly added thereto and the mixture was stirred for 1 hour. Next, a tetrahydrofuran (50.0 mL) solution containing cyclopentanone (17.6 g) was slowly added thereto, and the mixture was stirred for 12 hours while the temperature returned to room temperature. The reaction mixture was poured into water and an aqueous layer was extracted in toluene. The combined organic layer was washed with a saline and dried with anhydrous magnesium sulfate. The solution was concentrated under a reduced pressure and purified through silica gel chromatography (volume ratio, ethyl acetate:heptane =1:8), and thereby Compound (T-7) (31.9 g; 57%) was obtained.

### Second process: Synthesis of Compound (T-8)

Under a nitrogen atmosphere, Compound (T-7) (31.9 g), PTSA (2.3 g) and toluene (320 mL) were put into a reactor and heated and refluxed for 5 hours. The temperature returned to room temperature, the reaction mixture was poured into water and an aqueous layer was extracted in toluene. The combined organic layer was washed with a saline and dried with anhydrous magnesium sulfate. The solution was concentrated under a reduced pressure and purified through silica gel chromatography (volume ratio, toluene:heptane=1:8). In addition, purification was performed by re-crystallization from a solvent in which ethanol and heptane were mixed (volume ratio, 3: 1), and thereby Compound (T-8) (22.9 g; 76%) was obtained.

### Third process: Synthesis of Compound (T-9)

Compound (T-9) (17.6 g; 71%) was synthesized using Compound (T-8) (22.9 g) as a material and in the same method as in the second process of Synthesis Example 1.

### Fourth process: Synthesis of Compound (T-11)

Compound (T-11) (13.4 g; 84%) was synthesized using Compound (T-9) (10.0 g) and Compound (T-10) (10.0 g) as materials and in the same method as in the third process of Synthesis Example 1.

### Fifth process: Synthesis of Compound (No. 3)

Compound (No. 52) was synthesized using Compound (T-11) (13.4 g) as material and in the same method as in the fourth process of Synthesis Example 1. A crude product of the obtained Compound (No. 52), 5% palladium carbon (0.31 g), toluene (100 mL), and IPA (100 mL) were put into a reactor and stirred under a hydrogen atmosphere for 12 hours. A catalyst was removed by filtration, and the mixture was concentrated under a reduced pressure. Purification was performed through silica gel column chromatography (volume ratio, ethyl acetate:heptane=1:10), and additionally, purification was performed by re-crystallization from SOLMIX (registered trademark) A-ll, and thereby Compound (No. 3) (6.95 g, 54%) was obtained.

¹H-NMR (CDCl₃; δppm): 7.53-7.51(m, 2H), 7.21-7.18(m, 1H), 7.01-6.98(m, 1H), 4.13(t, J=6.7 Hz, 2H), 3.45-3.39(m, 1H), 2.14-2.10(m, 2H), 1.89-1.80(m, 4H), 1.78-1.65(m, 4H), 1.53-1.37(m, 4H), 0.95(t, J=7.2 Hz, 3H). ¹⁹F-NMR(δppm; CDCl₃): -142.00(d, J=6.3 Hz, 1F), -158.17(d, J=7.1 Hz, 1F).

Phase transition temperature: C 73.1 I. upper limit temperature (NI)=12.3 °C; dielectric anisotropy (Δε)=-7.3; optical anisotropy (Δn) =0.170; viscosity(η)=76.8 mPa·s.

### [Synthesis Example 3]

### Synthesis of Compound (No. 20)

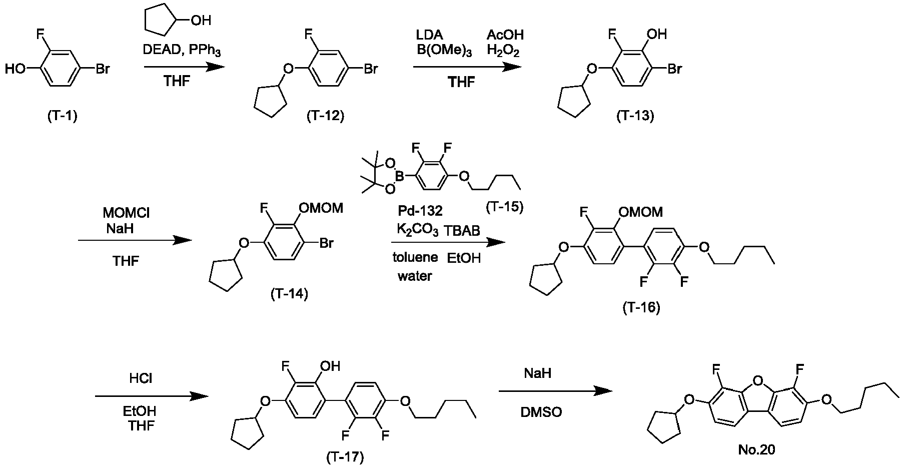

### First process: Synthesis of Compound (T-12)

Compound (T-12) (39.3 g; 96%) was obtained using Compound (T-1) (30.0 g) and cyclopentanol (13.5 g) as materials and in the same method as in the first process of Synthesis Example 1.

### Second process: Synthesis of Compound (T-13)

Compound (T-13) (33.8 g; 93%) was synthesized using Compound (T-12) (39.3 g) as a material and in the same method as in the second process of Synthesis Example 1.

### Third process: Synthesis of Compound (T-14)

Under a nitrogen atmosphere, Compound (T-13) (31.0 g) and tetrahydrofuran (310 mL) were put into a reactor and cooled to 0 °C. Sodium hydride (5.9 g) was slowly added thereto and the mixture was stirred for 1 hour. Methoxy methyl chloride (10.9 g) was added thereto and the mixture was stirred for 12 hours. The reaction mixture was poured into water and an aqueous layer was extracted in toluene. The combined organic layer was washed with a saline and dried with anhydrous magnesium sulfate. The solution was concentrated under a reduced pressure and purified through silica gel chromatography (volume ratio, toluene:heptane=1:3), and thereby Compound (T-14) (32.0 g; 88.2%) was obtained.

### Fourth process: Synthesis of Compound (T-16)

Compound (T-16) (16.5 g; 99%) was obtained using Compound (T-14) (12.0 g) and Compound (T-15) (12.9 g) as materials and in the same method as in the third process of Synthesis Example 1.

### Fifth process: Synthesis of Compound (T-17)

Compound (T-16) (16.5 g), hydrochloric acid (6 M, 25.0 mL), ethanol (40 mL) and tetrahydrofuran (40 mL) were put into a reactor and heated and refluxed for 3 hours. The reaction mixture cooled to room temperature was poured into water and an aqueous layer was extracted in toluene. The combined organic layer was washed with a saline and dried with anhydrous magnesium sulfate. The solution was concentrated under a reduced pressure and purified through silica gel chromatography (volume ratio, ethyl acetate:heptane=1: 3), and thereby Compound (T-17) (14.6 g; 95%) was obtained.

### Sixth process: Synthesis of Compound (No. 20)

Compound (No. 20) (8.46 g; 61%) was obtained using Compound (T-17) (14.6 g) as a material and in the same method as in the fourth process of Synthesis Example 1.

¹H-NMR (CDCl₃; δppm): 7.48-7.45(m, 2H), 6.99-6.96(m, 2H), 4.90-4.88(m, 1H), 4.12(t, J=6.8 Hz, 2H), 1.99-1.83(m, 8H), 1.69-1.60(m, 2H), 1.53-1.37(m, 4H), 0.95(t, J=7.1 Hz, 3H). ¹⁹F-NMR (δ ppm; CDCl₃): -157.22(d, J=7.2 Hz, 1F), -158.09(d, J=7.5 Hz, 1F).

Phase transition temperature: C 65.8 I. upper limit temperature (NI)=-5.0 °C; dielectric anisotropy (Δε)=-10.6; optical anisotropy (Δn)= 0.160; viscosity (η)=85.6 mPa·s.

### [Synthesis Example 4]

### Synthesis of Compound (No. 45)

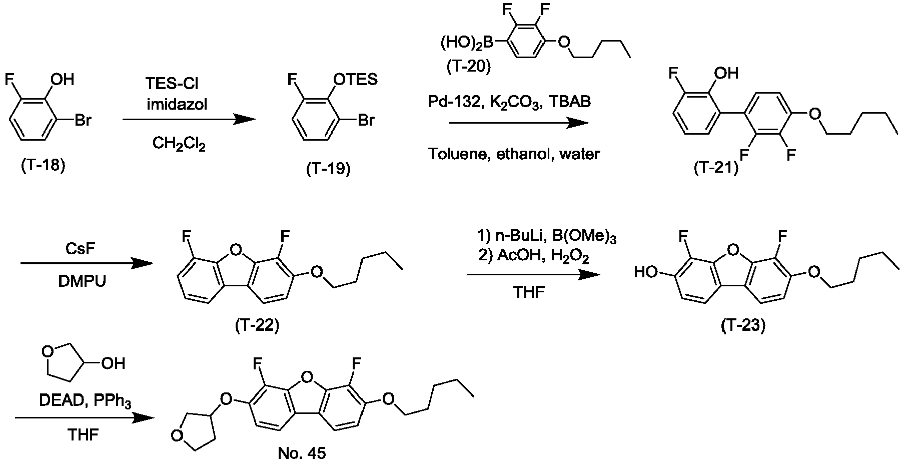

### First process: Synthesis of Compound (T-19)

Under a nitrogen atmosphere, Compound (T-18) (28.8 g), imidazole (22.6 g), and dichloromethane (250 mL) were put into a reactor and cooled on an ice bath. Chlorotriethylsilane (25.0 g) was added thereto little by little and the temperature returned to room temperature, and stirring was performed for 1 hour. After the reaction was completed, the reaction mixture was poured into water, and an aqueous layer was extracted in dichloromethane. The combined organic layer was washed with a saline and then dried with anhydrous magnesium sulfate and concentrated under a reduced pressure. The residue was purified through silica gel chromatography (heptane) and thereby Compound (T-19) (42.6 g; 91%) was obtained.

### Second process: Synthesis of Compound (T-21)

Under a nitrogen atmosphere, Compound (T-19) (15.0 g), Compound (T-20) (12.6 g), toluene (60 mL), ethanol (60 mL), water (60 mL), PdCl₂ (Amphos)₂(Pd-132, 0.035 g), potassium carbonate (15.6 g), and tetrabutylammonium bromide (TBAB, 3.96 g) were put into a reactor and stirred at 72 °C for 12 hours. The reaction mixture was poured into water and extracted in toluene, washed with water and a saline, and then dried with anhydrous magnesium sulfate and concentrated under a reduced pressure. The residue was purified through silica gel chromatography (volume ratio, ethyl acetate:heptane=1:2), and thereby Compound (T-21) (11.4 g; 75%) was obtained.

### Third process: Synthesis of Compound (T-22)

Under a nitrogen atmosphere, Compound (T-21) (10.9 g), cesium fluoride (7.81 g), and N,N'-dimethylpropylene urea (DMPU, 150 mL) were put into a reactor and stirred at 120 °C for 6 hours. After the reaction was completed, the reaction mixture cooled to room temperature and then poured into water, and extracted in diethyl ether. After washing with a saline, the reaction mixture was dried with anhydrous magnesium sulfate and concentrated under a reduced pressure. The residue was purified through silica gel chromatography (volume ratio, toluene:heptane=1:2). In addition, purification was performed by re-crystallization from a solvent in which SOLMIX (registered trademark) A-11 and heptane were mixed (volume ratio, 1:1), and thereby Compound (T-22) (9.04 g; 88%) was obtained.

### Fourth process: Synthesis of Compound (T-23)

Under a nitrogen atmosphere, Compound (T-22) (9.04 g) and THF (200 mL) were put into a reactor and cooled to -70 °C. n-Butyl lithium (1.6 M; n-hexane solution; 23.2 ml) was added thereto and the mixture was stirred for 2 hours. Trimethylborate (3.88 g) was added thereto, the temperature returned to room temperature, and stirring was performed for 12 hours. Acetic acid (3.6 ml) was added thereto at room temperature, the mixture was stirred for 30 minutes, and a hydrogen peroxide solution (50 wt%; 3.8 ml) was then added thereto and the mixture was stirred for 1 hour. After the reaction was completed, the reaction mixture was poured into water, and an aqueous layer was extracted in toluene. The combined organic layer was washed with water, a sodium bisulfite aqueous solution, and a saline and then dried with anhydrous magnesium sulfate and concentrated under a reduced pressure. The residue was purified through silica gel chromatography (volume ratio, ethyl acetate:toluene=1:2), and thereby Compound (T-23) (8.72 g; 95%) was obtained.

### Fifth process: Synthesis of Compound (No. 45)

Under a nitrogen atmosphere, Compound (T-23) (4.00 g), 3-hydroxytetrahydrofuran (1.15 g), triphenyl phosphine (4.11 g), and THF (40 mL) were put into a reactor and cooled on an ice bath. Diethyl azodicarboxylate (DEAD, 2.2 M; toluene solution; 7.1 ml) was added thereto and the mixture was stirred at room temperature for 12 hours. After the reaction was completed, the reaction mixture was poured into water and an aqueous layer was extracted in toluene. The combined organic layer was washed with a saline and then dried with anhydrous magnesium sulfate and concentrated under a reduced pressure. The residue was purified through silica gel chromatography (volume ratio, toluene:heptane=2:1), and additionally purification was performed by re-crystallization from heptane, and thereby Compound (No. 45) (4.11 g, 84%) was obtained.

¹H-NMR (CDCl₃; δppm): 7.50-7.47(m, 2H), 7.00-6.93(m, 2H), 5.08-5.06(m, 1H), 4.17(t, J=6.7 Hz, 1H), 4.09-4.01(m, 3H), 3.95(dt, J=4.0 Hz, J=8.3 Hz, 1H), 2.28-2.17(m, 2H), 1.89-1.84(m, 2H), 1.52-1.37(m, 4H), 0.95(t, J=7.3 Hz, 3H). ¹⁹F-NMR (δppm; CDCl₃): -156.11(d, J=7.1 Hz, 1F), -157.99(d, J=7.1 Hz, 1F).

Phase transition temperature: C 104.6 I. upper limit temperature (NI)=14.3 °C; dielectric anisotropy (Δε)=-9.71; optical anisotropy (Δn)=0.220; viscosity (η)=85.6 mPa·s.; measured concentration: 3 weight%.

It is possible to synthesize the following compounds with reference to the methods described in the synthesis examples and the section "2. Synthesis of Compound (1)."

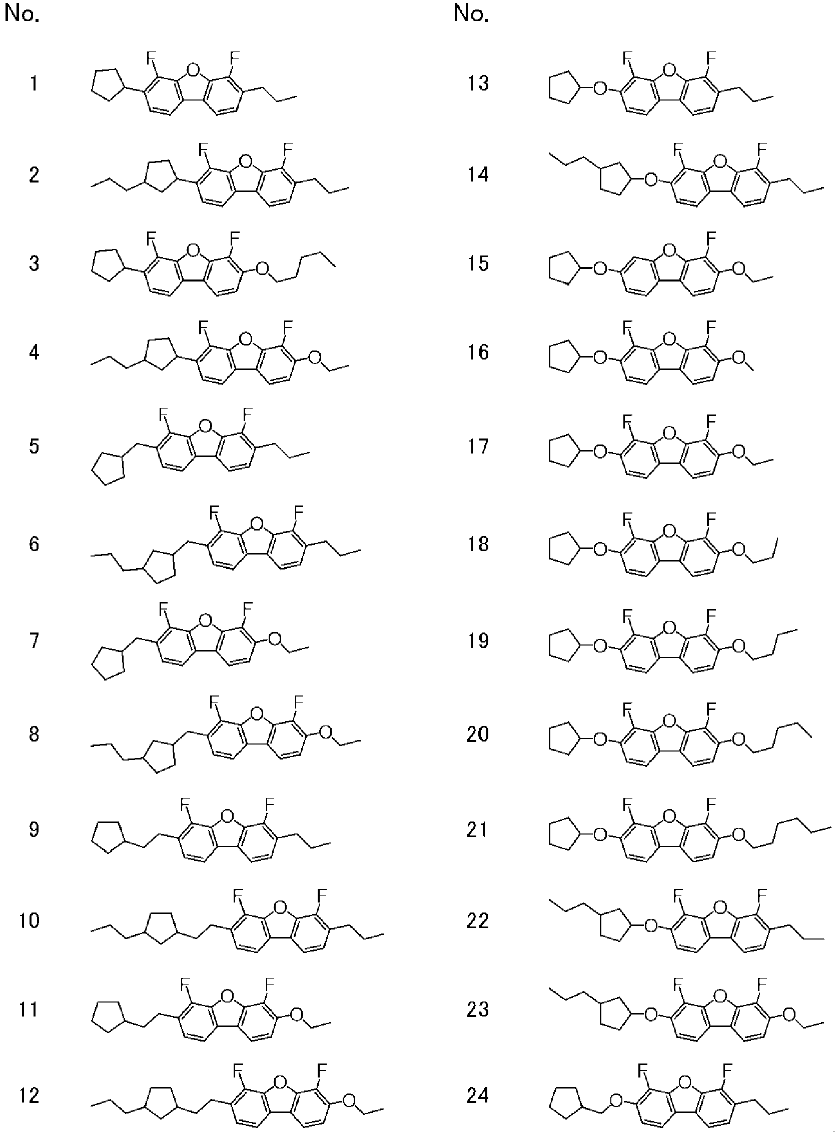

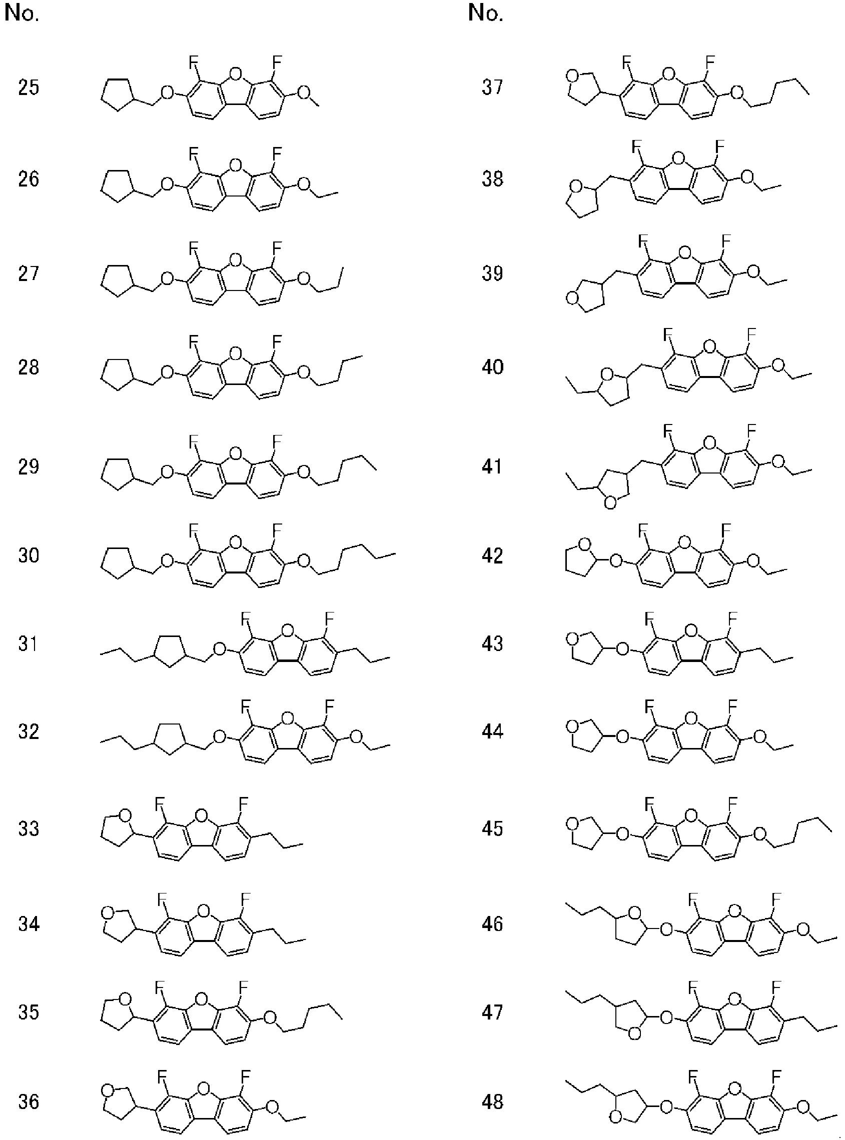

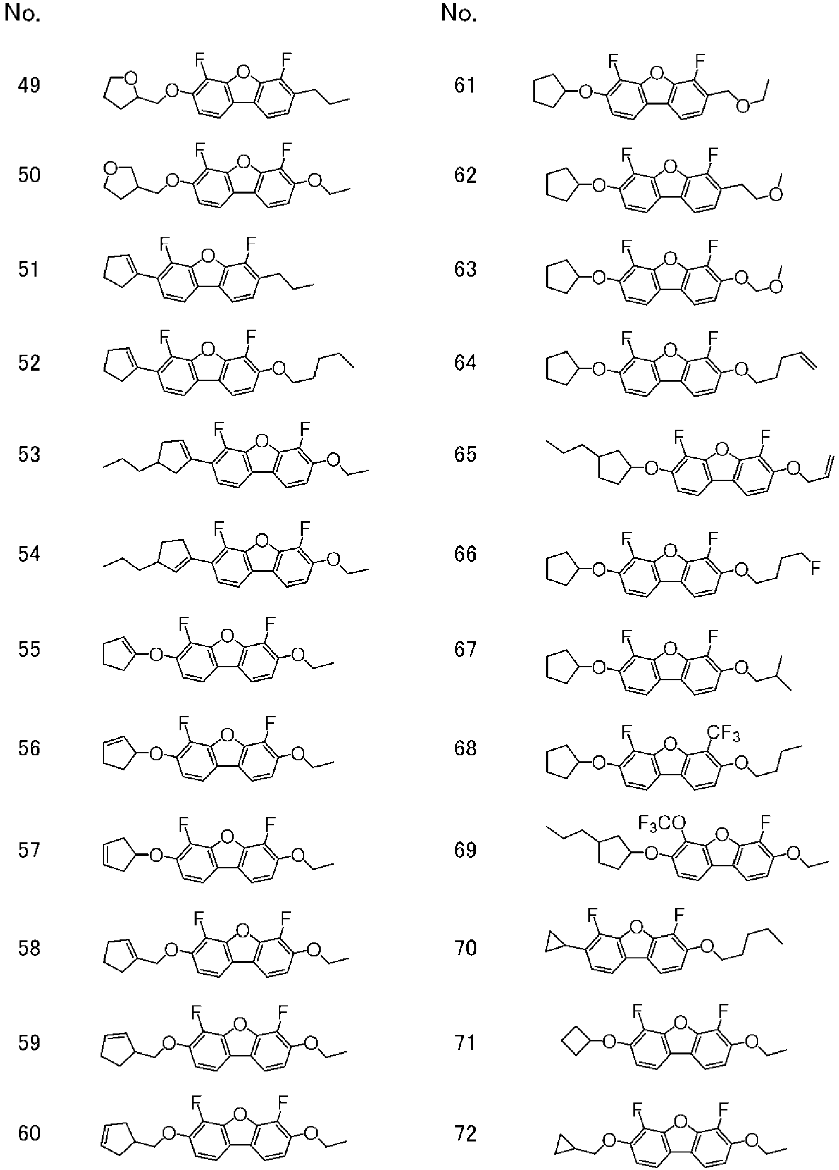

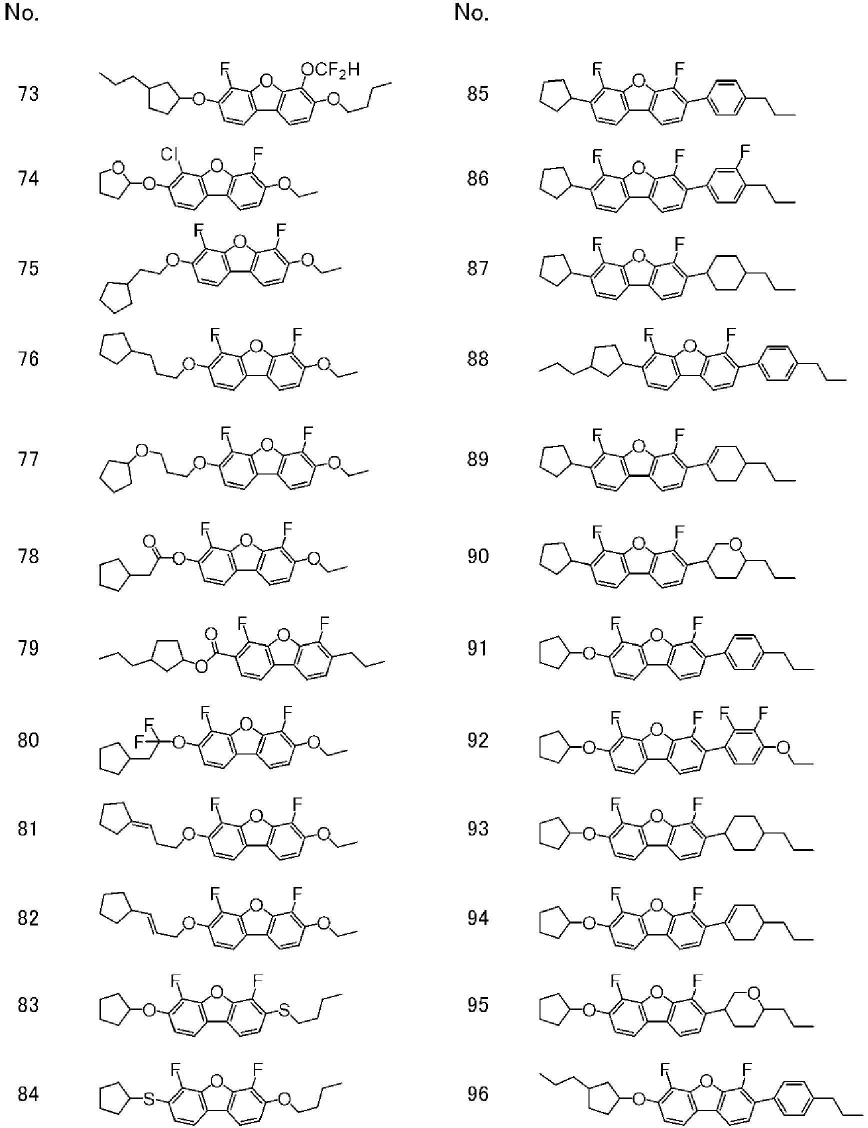

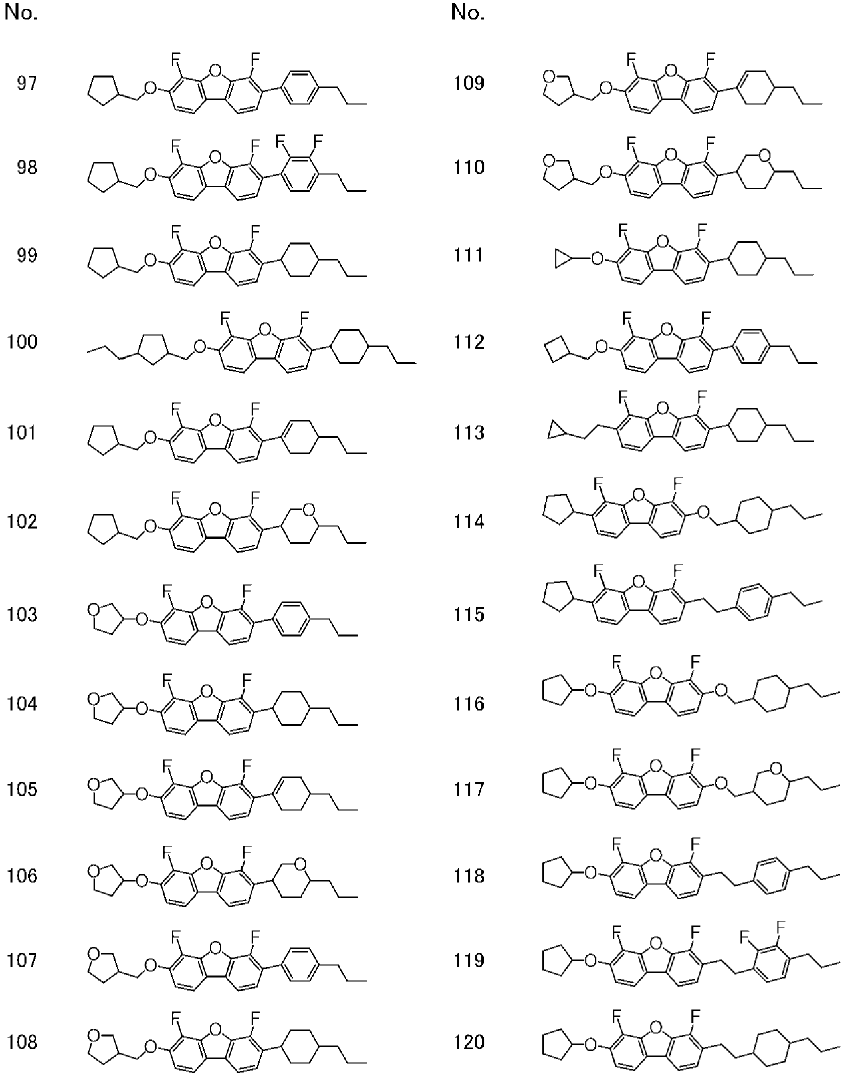

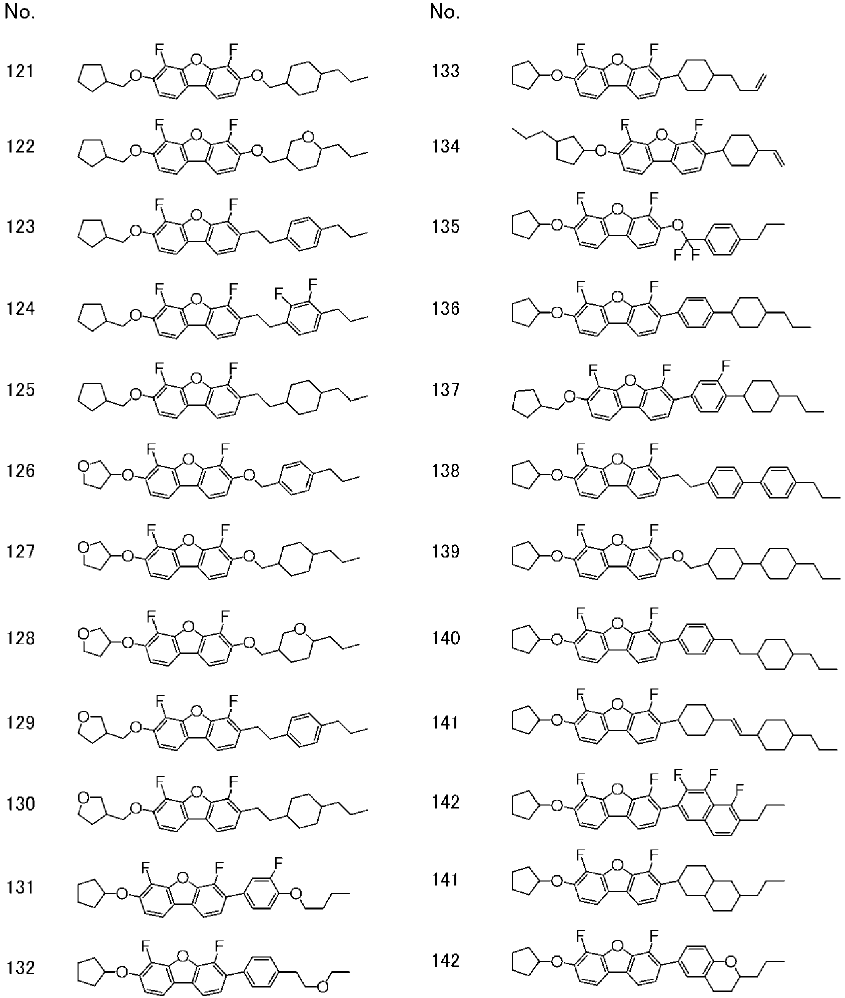

### [Comparative Example 1]

### Comparison of physical properties

The following Compound (S-1) was selected as a comparative compound. This is because this compound is described in Japanese Unexamined Patent Application Publication No. 2015-174864, and is similar to the compound of the present disclosure

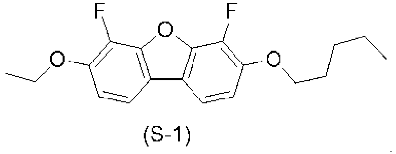

¹H-NMR (CDCl₃; δppm): 7.47-7.45(m, 2H), 6.99-6.95(m, 2H), 4.20(q, J=7.0 Hz, 2H), 4.12(t, J=6.6 Hz, 2H), 1.89-1.83(m, 2H), 1.52-1.47(m, 5H), 1.46-1.37(m, 2H)), 0.95(t, J=7.3 Hz, 3H). ¹⁹F-NMR(δppm; CDCl₃): -158.10--158.11(m, 2F).

Phase transition temperature: C 59.2 I. upper limit temperature (NI)=24.3 °C; dielectric anisotropy (Δε)=-11.5; optical anisotropy (Δn)=0.190; viscosity (η)=62.5 mPa·s.

**Table 2. Compatibilities at a low temperature between Compound (No. 20) and Comparative Compound (S-1)**

| Sample for measurement | Components of sample | | Conditions (-10 °C, 30 days) |
|---|---|---|---|
| Composition (X-1) | 15 weight% of Compound (No. 20) | 85 weight% of mother liquid crystal (B) | Maintain nematic phase |
| Composition (X-2) | 15 weight% of Compound (S-1) | 85 weight% of mother liquid crystal (B) | Precipitate crystal |
| Composition (X-3) | 10 weight% of Compound (S-1) | 90 weight% of mother liquid crystal (B) | Precipitate crystal |
| Composition (X-4) | 5 weight% of Compound (S-1) | 95 weight% of mother liquid crystal (B) | Maintain nematic phase |

Compatibilities at a low temperature between Compound (No. 20) obtained in Synthesis Example 3 and Comparative Compound (S-1) are summarized in Table 2. In Composition (X-1) in which Compound (No. 20) was added at 15 weight% in the mother liquid crystal (B), a nematic phase was maintained after 30 days in a freezer at -10 °C. On the other hand, in Compositions (X-2) and (X-3) in which Comparative Compound (S-1) was added at 15 weight% and 10 weight%, respectively, precipitation of crystals was observed. This indicates that Compound (No. 20) of the present application had superior solubility in mother liquid crystals at a low temperature compared with Comparative Compound (S-1).

### [Comparative Example 2]

### Comparison of physical properties

The following Compound (S-2) was selected as a comparative compound. This is because this compound was similar to the compound of the present invention in that it had a ring structure other than a dibenzofuran ring.

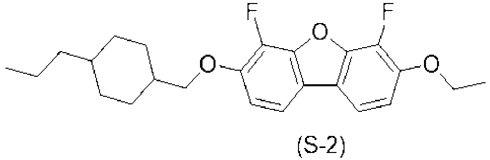

¹H-NMR (CDCl₃; δ ppm): 7.48-7.46(m, 2H), 6.99-6.95(m, 2H), 4.21(q, J=7.0 Hz, 2H), 3.92(d, J=6.4 Hz, 2H), 1.96-1.93(m, 2H), 1.87-1.78(m, 3H), 1.49(t, J=7.1 Hz, 3H), 1.36-1.05(m, 7H)), 0.99-0.87(m, 5H). ¹⁹F-NMR (δ ppm; CDCl₃): -158.08(d, J=7.3 Hz, 1F), -158.17(d, J=7.5 Hz, 1F).

**Table 3. Compatibilities at a low temperature between Compound (No. 20) and Comparative Compound (S-2)**

| Sample for measurement | Components of sample | | Conditions (-10 °C, 30 days) |
|---|---|---|---|
| Composition (X-1) | 15 weight% of Compound (No. 20) | 85 weight% of mother liquid crystal (B) | Maintain nematic phase |
| Composition (X-5) | 15 weight% of Compound (S-2) | 85 weight% of mother liquid crystal (B) | Precipitate crystal |
| Composition (X-6) | 10 weight% of Compound (S-2) | 90 weight% of mother liquid crystal (B) | Precipitate crystal |
| Composition (X-7) | 5 weight% of Compound (S-2) | 95 weight% of mother liquid crystal (B) | Precipitate crystal |
| Composition (X-8) | 3 weight% of Compound (S-2) | 97 weight% of mother liquid crystal (B) | Precipitate crystal |

Compatibilities at a low temperature between Compound (No. 20) obtained in Synthesis Example 3 and Comparative Compound (S-2) are summarized in Table 3. In Composition (X-1) in which Compound (No. 20) was added at 15 weight% in the mother liquid crystal (B), a nematic phase was maintained after 30 days in a freezer at -10 °C. On other hand, in all of Compositions (X-5) to (X-8) in which Comparative Compound (S-2) was added at 15 weight%, 10 weight%, 5 weight%, and 3 weight%, respectively, precipitation of crystals was observed. In addition, also in Composition (X-8) in which Compound (S-2) was added only at 3 weight% in the mother liquid crystal (B), crystals were already precipitated at 25 °C. The above results indicate that Compound (No. 20) of the present application had superior solubility in mother liquid crystals at room temperature and a low temperature compared with Comparative Compound (S-2).

### 2. Examples of composition

The present disclosure will be described in further detail with reference to examples. Since the examples are typical examples, the present disclosure is not limited by the examples. For example, the present disclosure includes a mixture of the composition of Usage Example 1 and the composition of Usage Example 2 in addition to the composition of these usage examples. The present disclosure includes a mixture prepared by mixing at least two compositions of these usage examples. The compounds in the usage examples are indicated by symbols on the basis of definitions in the following Table 4. In Table 4, the configuration related to 1,4-cyclohexylene is trans. In the usage example, a number in parentheses after a symbol indicates a chemical formula to which the compound belongs. The symbol (-) refers to a liquid crystalline compound different from Compounds (1) to (15). A proportion (percentage) of the liquid crystalline compound is a weight percentage (weight%) on the basis of the weight of the liquid crystal composition containing no additives. Finally, values of physical properties of the compositions are summarized. The physical properties were measured according to the methods described above and measured values (without extrapolation) were shown without change.

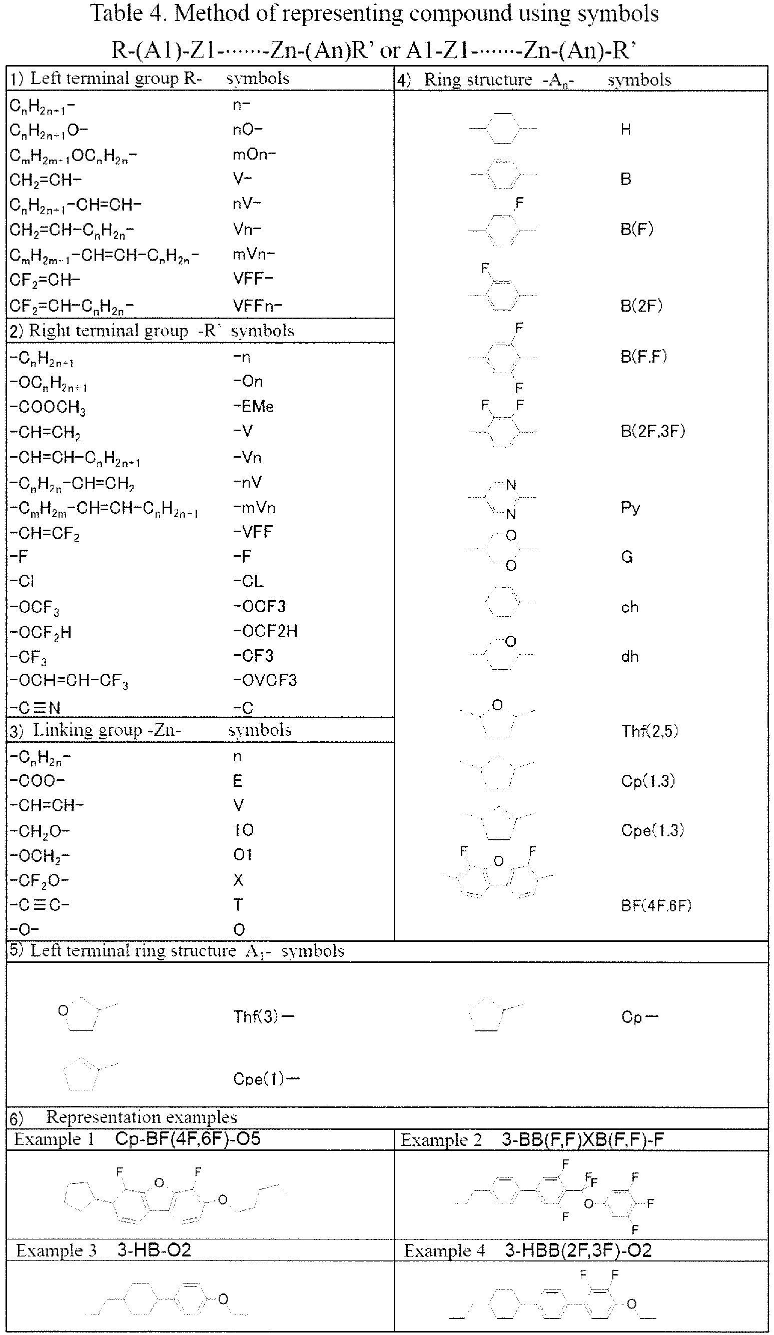

**[Usage Example 1]**

| Cp1O-BF(4F, 6F)-O2 | (No. 26) | 3% |
|---|---|---|
| 3-HB-O2 | (2-5) | 15% |
| 2-BTB-1 | (2-10) | 3% |
| 3-HHB-1 | (3-1) | 8% |
| 3-HHB-O1 | (3-1) | 5% |
| 3-HHB-3 | (3-1) | 14% |
| 3-HHB-F | (13-1) | 4% |
| 2-HHB(F)-F | (13-2) | 6% |
| 3-HHB(F)-F | (13-2) | 6% |
| 5-HHB(F)-F | (13-2) | 6% |
| 3-HHB(F, F)-F | (13-3) | 4% |
| 3-HHEB-F | (13-10) | 5% |
| 5-HHEB-F | (13-10) | 5% |
| 2-HB-C | (15-1) | 4% |
| 3-HB-C | (15-1) | 12% |

NI=102.3 °C; η=20.4 mPa·s; Δn=0.105; Δε=4.0.

**[Usage Example 2]**

| Cp-BF(4F, 6F)-O5 | (No. 3) | 4% |
|---|---|---|
| 3-HH-4 | (2-1) | 10% |
| 3-HB-O2 | (2-5) | 10% |
| 3-HB-CL | (12-2) | 11% |

| 3-HHB(F, F)-F | (13-3) | 4% |
|---|---|---|
| 3-HBB(F, F)-F | (13-24) | 25% |
| 5-HBB(F, F)-F | (13-24) | 25% |
| 5-HBB(F)B-2 | (4-5) | 6% |
| 5-HBB(F)B-3 | (4-5) | 5% |

NI=71.4 °C; η=21.8 mPa·s; Δn=0.121; Δε=4.7.

**[Usage Example 3]**

| CpO-BF(4F, 6F)-O5 | (No. 20) | 7% |
|---|---|---|
| 3-HB-O2 | (2-5) | 7% |
| 7-HB(F, F)-F | (12-4) | 5% |
| 2-HHB(F)-F | (13-2) | 7% |
| 3-HHB(F)-F | (13-2) | 8% |
| 5-HHB(F)-F | (13-2) | 8% |
| 2-HBB-F | (13-22) | 5% |
| 3-HBB-F | (13-22) | 5% |
| 5-HBB-F | (13-22) | 5% |
| 2-HBB(F)-F | (13-23) | 10% |
| 3-HBB(F)-F | (13-23) | 10% |
| 5-HBB(F)-F | (13-23) | 11% |
| 3-HBB(F, F)-F | (13-24) | 6% |
| 5-HBB(F, F)-F | (13-24) | 6% |

NI=76.4 °C; η=28.2 mPa·s; Δn=0.119; Δε=4.1.

**[Usage Example 4]**

| CpO-BF(4F, 6F)-O2 | (No. 17) | 3% |
|---|---|---|
| 3-HH-4 | (2-1) | 10% |
| 3-HH-5 | (2-1) | 6% |
| 1O1-HBBH-5 | (4-1) | 6% |
| 5-HB-CL | (12-2) | 10% |
| 3-HHB-F | (13-1) | 4% |
| 3-HHB-CL | (13-1) | 3% |
| 4-HHB-CL | (13-1) | 4% |
| 3-HHB(F)-F | (13-2) | 10% |
| 4-HHB(F)-F | (13-2) | 9% |
| 5-HHB(F)-F | (13-2) | 9% |
| 7-HHB(F)-F | (13-2) | 9% |
| 5-HBB(F)-F | (13-23) | 3% |
| 3-HHBB(F, F)-F | (14-6) | 2% |
| 4-HHBB(F, F)-F | (14-6) | 3% |
| 5-HHBB(F, F)-F | (14-6) | 3% |
| 3-HH2BB(F, F)-F | (14-15) | 3% |
| 4-HH2BB(F, F)-F | (14-15) | 3% |

NI=122.2 °C; η=23.5 mPa·s; Δn=0.098; Δε=3.1.

**[Usage Example 5]**

| CpO-BF(4F, 6F)-O3 | (No. 18) | 5% |
|---|---|---|
| 1O1-HBBH-4 | (4-1) | 3% |
| 1O1-HBBH-5 | (4-1) | 3% |
| 3-HHB(F, F)-F | (13-3) | 10% |
| 3-H2HB(F, F)-F | (13-15) | 8% |
| 4-H2HB(F, F)-F | (13-15) | 8% |
| 5-H2HB(F, F)-F | (13-15) | 10% |
| 3-HBB(F, F)-F | (13-24) | 18% |
| 5-HBB(F, F)-F | (13-24) | 18% |
| 3-H2BB(F, F)-F | (13-27) | 9% |
| 5-HHBB(F, F)-F | (14-6) | 3% |
| 3-HH2BB(F, F)-F | (14-15) | 3% |
| 5-HHEBB-F | (14-17) | 2% |

NI=92.2 °C; η=36.6 mPa·s; Δn=0.116; Δε=7.9.

**[Usage Example 6]**

| CpO-BF(4F, 6F)-O4 | (No. 19) | 4% |
|---|---|---|
| 5-HBBH-3 | (4-1) | 5% |
| 3-HB(F)BH-3 | (4-2) | 3% |
| 5-HB-F | (12-2) | 12% |
| 6-HB-F | (12-2) | 9% |
| 7-HB-F | (12-2) | 7% |
| 2-HHB-OCF₃ | (13-1) | 8% |
| 3-HHB-OCF₃ | (13-1) | 5% |
| 4-HHB-OCF₃ | (13-1) | 7% |
| 5-HHB-OCF₃ | (13-1) | 5% |
| 3-HHB(F, F)-OCF2H | (13-3) | 6% |
| 3-HHB(F, F)-OCF₃ | (13-3) | 4% |
| 3-HH2B-OCF₃ | (13-4) | 5% |
| 5-HH2B-OCF₃ | (13-4) | 4% |
| 3-HH2B(F)-F | (13-5) | 3% |
| 3-HBB(F)-F | (13-23) | 5% |
| 5-HBB(F)-F | (13-23) | 8% |

**[Usage Example 7]**

| Cp1O-BF(4F, 6F)-O5 | (No. 29) | 3% |
|---|---|---|
| 3-HH-4 | (2-1) | 8% |
| 3-HHB-1 | (3-1) | 6% |
| 5-HB-CL | (12-2) | 8% |
| 3-HHB(F, F)-F | (13-3) | 9% |
| 3-HHEB(F, F)-F | (13-12) | 9% |
| 4-HHEB(F, F)-F | (13-12) | 4% |
| 5-HHEB(F, F)-F | (13-12) | 3% |
| 3-HBB(F, F)-F | (13-24) | 17% |
| 5-HBB(F, F)-F | (13-24) | 14% |
| 2-HBEB(F, F)-F | (13-39) | 4% |
| 3-HBEB(F, F)-F | (13-39) | 5% |
| 5-HBEB(F, F)-F | (13-39) | 4% |
| 3-HHBB(F, F)-F | (14-6) | 6% |

**[Usage Example 8]**

| THF (3)O-BF(4F, 6F)-O5 | (No. 45) | 5% |
|---|---|---|
| 3-HB-CL | (12-2) | 5% |
| 5-HB-CL | (12-2) | 5% |
| 3-HHB-OCF₃ | (13-1) | 5% |
| V-HHB(F)-F | (13-2) | 4% |
| 3-HHB(F)-F | (13-2) | 4% |
| 5-HHB(F)-F | (13-2) | 4% |
| 3-H2HB-OCF₃ | (13-13) | 5% |
| 5-H2HB(F, F)-F | (13-15) | 4% |
| 5-H4HB-OCF₃ | (13-19) | 15% |
| 3-H4HB(F, F)-CF₃ | (13-21) | 8% |
| 5-H4HB(F, F)-CF₃ | (13-21) | 10% |
| 5-H4HB(F, F)-F | (13-21) | 7% |
| 2-H2BB(F)-F | (13-26) | 5% |
| 3-H2BB(F)-F | (13-26) | 8% |
| 3-HBEB(F, F)-F | (13-39) | 6% |

**[Usage Example 9]**

| CpO-BF(4F, 6F)-O3V | (No. 64) | 6% |
|---|---|---|
| 3-HH-4 | (2-1) | 10% |
| 3-HH-5 | (2-1) | 5% |
| 3-HB-O2 | (2-5) | 10% |
| 3-HHB-1 | (3-1) | 10% |
| 3-HHB-O1 | (3-1) | 5% |
| 5-HB-CL | (12-2) | 10% |
| 7-HB(F, F)-F | (12-4) | 7% |
| 2-HHB(F)-F | (13-2) | 7% |
| 3-HHB(F)-F | (13-2) | 7% |
| 5-HHB(F)-F | (13-2) | 7% |
| 3-HHB(F, F)-F | (13-3) | 6% |
| 3-H2HB(F, F)-F | (13-15) | 5% |
| 4-H2HB(F, F)-F | (13-15) | 5% |

**[Usage Example 10]**

| Cpe(1)1O-BF(4F, 6F)-O5 | (No. 60) | 4% |
|---|---|---|
| 3-HH-4 | (2-1) | 9% |
| 3-HH-5 | (2-1) | 9% |
| 3-HB-O2 | (2-5) | 9% |
| 5-HB-CL | (12-2) | 5% |
| 7-HB(F)-F | (12-3) | 5% |
| 2-HHB(F, F)-F | (13-3) | 5% |
| 3-HHB(F, F)-F | (13-3) | 5% |
| 3-HHEB-F | (13-10) | 8% |
| 5-HHEB-F | (13-10) | 8% |
| 3-HHEB(F, F)-F | (13-12) | 10% |
| 4-HHEB(F, F)-F | (13-12) | 5% |
| 3-GHB(F, F)-F | (13-109) | 5% |
| 4-GHB(F, F)-F | (13-109) | 6% |
| 5-GHB(F, F)-F | (13-109) | 7% |

**[Usage Example 11]**

| 3Cp(1, 3)O-BF(4F, 6F)-O2 | (No. 23) | 5% |
|---|---|---|
| 5-HH-VFF | (2-1) | 19% |
| 2-BTB-1 | (2-10) | 12% |
| 3-HHB-1 | (3-1) | 5% |
| VFF-HHB-1 | (3-1) | 5% |
| VFF2-HHB-1 | (3-1) | 12% |
| 3-H2BTB-2 | (3-17) | 5% |
| 3-H2BTB-3 | (3-17) | 5% |
| 3-H2BTB-4 | (3-17) | 5% |
| 3-HB-C | (15-1) | 20% |
| 1V2-BEB(F, F)-C | (15-15) | 7% |

**[Usage Example 12]**

| 3Cp(1, 3)1O-BF(4F, 6F)-O2 | (No. 32) | 3% |
|---|---|---|
| 3-HH-V | (2-1) | 36% |
| 3-HH-V1 | (2-1) | 8% |
| 3-HHB-1 | (3-1) | 5% |
| V-HHB-1 | (3-1) | 6% |
| V2-BB(F)B-1 | (3-6) | 5% |
| 3-HHEH-5 | (3-13) | 5% |
| 1V2-BB-F | (12-1) | 3% |
| 3-BB(F, F)XB(F, F)-F | (13-97) | 10% |
| 3-HHBB(F, F)-F | (14-6) | 3% |
| 5-HB(F)B(F, F)XB(F, F)-F | (14-41) | 4% |
| 3-BB(F)B(F, F)XB(F, F)-F | (14-47) | 4% |
| 4-BB(F)B(F, F)XB(F, F)-F | (14-47) | 5% |
| 5-BB(F)B(F, F)XB(F, F)-F | (14-47) | 3% |

**[Usage Example 13]**

| Cp1O-BF(4F, 6F)-O2 | (No. 26) | 3% |
|---|---|---|
| Cp-BF(4F, 6F)-O5 | (No. 3) | 5% |
| 3-HH-V | (2-1) | 37% |
| 3-HH-V1 | (2-1) | 5% |
| 3-HHB-1 | (3-1) | 4% |
| V-HHB-1 | (3-1) | 4% |
| V2-BB(F)B-1 | (3-6) | 5% |
| 3-HHEH-5 | (3-13) | 3% |
| 1V2-BB-F | (12-1) | 3% |
| 3-BB(F, F)XB(F, F)-F | (13-97) | 5% |
| 3-GB(F, F)XB(F, F)-F | (13-113) | 5% |
| 3-HHBB(F, F)-F | (14-6) | 4% |
| 5-HB(F)B(F, F)XB(F, F)-F | (14-41) | 3% |
| 3-BB(F)B(F, F)XB(F, F)-F | (14-47) | 3% |
| 4-BB(F)B(F, F)XB(F, F)-F | (14-47) | 4% |
| 5-BB(F)B(F, F)XB(F, F)-F | (14-47) | 3% |
| 3-GB(F)B(F, F)XB(F, F)-F | (14-57) | 4% |

NI=78.9 °C; η=19.0 mPa·s; Δn=0.111; Δε=6.0.

**[Usage Example 14]**

| CpO-BF(4F, 6F)-O5 | (No. 20) | 5% |
|---|---|---|
| CpO-BF(4F, 6F)-O3 | (No. 18) | 7% |
| 3-HH-V | (2-1) | 35% |
| 3-HH-V1 | (2-1) | 3% |
| 3-HHB-1 | (3-1) | 3% |
| V-HHB-1 | (3-1) | 5% |
| V2-BB(F)B-1 | (3-6) | 3% |
| 3-HHEH-5 | (3-13) | 3% |
| 1V2-BB-F | (12-1) | 3% |
| 3-BB(F, F)XB(F, F)-F | (13-97) | 3% |
| 3-GB(F, F)XB(F, F)-F | (13-113) | 3% |
| 3-HHBB(F, F)-F | (14-6) | 3% |
| 3-BB(F)B(F, F)XB(F, F)-F | (14-47) | 3% |
| 4-BB(F)B(F, F)XB(F, F)-F | (14-47) | 3% |
| 5-BB(F)B(F, F)XB(F, F)-F | (14-47) | 3% |
| 3-GB(F)B(F, F)XB(F, F)-F | (14-57) | 3% |
| 4-GB(F)B(F, F)XB(F, F)-F | (14-57) | 4% |
| 5-GB(F)B(F, F)XB(F, F)-F | (14-57) | 5% |
| 3-GB(F)B(F, F)XB(F, F)-F | (14-57) | 3% |

NI=76.4 °C; η=28.2 mPa·s; Δn=0.113; Δε=7.2.

**[Usage Example 15]**

| CpO-BF(4F, 6F)-O2 | (No. 17) | 4% |
|---|---|---|
| 3-HH-V | (2-1) | 37% |
| 3-HH-V1 | (2-1) | 4% |
| 3-HHB-1 | (3-1) | 4% |
| V-HHB-1 | (3-1) | 4% |
| V2-BB(F)B-1 | (3-6) | 4% |
| 3-HHEH-5 | (3-13) | 4% |
| 1V2-BB-F | (12-1) | 4% |
| 3-BB(F, F)XB(F, F)-F | (13-97) | 7% |
| 3-HHBB(F, F)-F | (14-6) | 4% |
| 5-HB(F)B(F, F)XB(F, F)-F | (14-41) | 4% |
| 3-BB(F)B(F, F)XB(F, F)-F | (14-47) | 4% |
| 4-BB(F)B(F, F)XB(F, F)-F | (14-47) | 4% |
| 5-BB(F)B(F, F)XB(F, F)-F | (14-47) | 4% |
| 2-dhBB(F, F)XB(F, F)-F | (14-50) | 4% |
| 3-dhBB(F, F)XB(F, F)-F | (14-50) | 4% |

NI=84.3 °C; η=20.9 mPa·s; Δn=0.113; Δε=6.7.

**[Usage Example 16]**

| CpO-BF(4F, 6F)-O4 | (No. 19) | 5% |
|---|---|---|
| 3-HH-V | (2-1) | 35% |
| 3-HH-V1 | (2-1) | 5% |
| 3-HHB-1 | (3-1) | 5% |
| V-HHB-1 | (3-1) | 3% |
| V2-BB(F)B-1 | (3-6) | 3% |
| 3-HHEH-5 | (3-13) | 3% |
| 1V2-BB-F | (12-1) | 3% |
| 3-BB(F, F)XB(F, F)-F | (13-97) | 3% |
| 3-GB(F, F)XB(F, F)-F | (13-113) | 3% |
| 3-HHBB(F, F)-F | (14-6) | 3% |
| 5-HB(F)B(F, F)XB(F, F)-F | (14-41) | 3% |
| 3-BB(F)B(F, F)XB(F, F)-F | (14-47) | 3% |
| 4-BB(F)B(F, F)XB(F, F)-F | (14-47) | 3% |
| 5-BB(F)B(F, F)XB(F, F)-F | (14-47) | 3% |
| 3-GB(F)B(F, F)XB(F, F)-F | (14-57) | 3% |
| 3-GBB(F, F)XB(F, F)-F | (14-58) | 5% |
| 4-GBB(F, F)XB(F, F)-F | (14-58) | 4% |
| 5-GBB(F, F)XB(F, F)-F | (14-58) | 5% |

### [Industrial Applicability]

The liquid crystalline compound of the present disclosure has favorable physical properties. The liquid crystal composition including this compound can be widely used for liquid crystal display elements such as personal computers and televisions.

## Claims

1. A compound, **characterized in that** the compound represented by Formula (1): 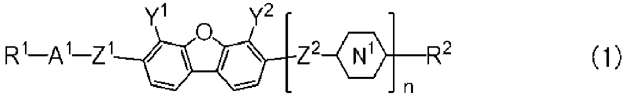 in Formula (1),
R¹ and R² independently represent a hydrogen atom or an alkyl group having 1 to 10 carbon atoms, and in the alkyl group, at least one -CH₂- is optionally substituted with -O-, -S-, -CO-, or -SiH₂-, at least one -CH₂CH₂- is optionally substituted with -CH=CH- or -C≡C-, and in these groups, at least one hydrogen atom is optionally substituted with a fluorine atom;
the ring A¹ represents 1,2-cyclopropylene, 1,3-cyclobutylene, 1,3-cyclopentylene, 1,3-cyclopentenylene, or 1,4-cyclopentenylene, and in these groups, at least one -CH₂- is optionally substituted with -O-;
the ring N¹ represents 1,4-cyclohexylene, decahydronaphthalene-2,6-diyl, or 1,4-phenylene, and in these groups, at least one -CH₂- is optionally substituted with -O-, -S-, -CO-, or -SiH₂-, at least one -CH₂CH₂- is optionally substituted with -CH=CH- or -CH=N-, and in these divalent groups, at least one hydrogen atom is optionally substituted with a fluorine atom, a chlorine atom, -C≡N, -CF₃, -CHF₂, -CH₂F, -OCF₃, -OCHF₂, or -OCH₂F;
Y¹ and Y² independently represent a hydrogen atom, a fluorine atom, a chlorine atom, -CF₃, -CHF₂, -CH₂F, -OCF₃, -OCHF₂, or -OCH₂F;
n is 0, 1 or 2;
Z¹ and Z² independently represent a single bond or an alkylene group having 1 to 6 carbon atoms, and in the alkylene group, at least one -CH₂- is optionally substituted with -O-, -S-, or -CO-, one or two -CH₂CH₂-'s are optionally substituted with -CH=CH- or -C≡C-, and in these divalent groups, at least one hydrogen atom is optionally substituted with a fluorine atom.

2. The compound according to claim 1,
wherein, in Formula (1),
R¹ and R² independently represent a hydrogen atom, an alkyl group having 1 to 10 carbon atoms, an alkoxy group having 1 to 9 carbon atoms, an alkoxyalkyl group having 2 to 9 carbon atoms, an alkenyl group having 2 to 10 carbon atoms, or an alkenyloxy group having 2 to 9 carbon atoms;
the ring N¹ represents 1,4-cyclohexylene or 1,4-phenylene, and in these groups, at least one -CH₂- is optionally substituted with -O-, at least one -CH₂CH₂- is optionally substituted with -CH=CH-, and in these divalent groups, at least one hydrogen atom is optionally substituted with a fluorine atom;
Y¹ and Y² independently represent a hydrogen atom or a fluorine atom; and
Z¹ and Z² independently represent a single bond or an alkylene group having 1 to 6 carbon atoms, and at least one -CH₂- is optionally substituted with -O-.

3. The compound according to claim 1, which is represented by Formulae (1-1) to (1-3): 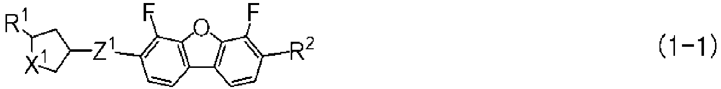 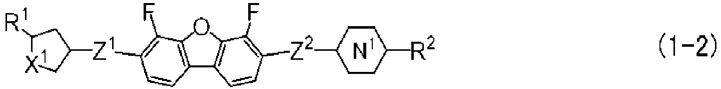 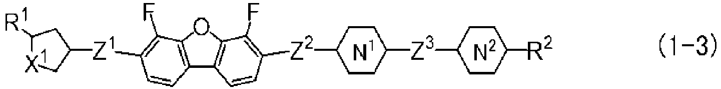 in Formulae (1-1) to (1-3),
R¹ and R² independently represent a hydrogen atom, an alkyl group having 1 to 10 carbon atoms, an alkoxy group having 1 to 9 carbon atoms, an alkoxyalkyl group having 2 to 9 carbon atoms, an alkenyl group having 2 to 10 carbon atoms, or an alkenyloxy group having 2 to 9 carbon atoms;
X¹ represents -CH₂- or -O-;
the ring N¹ and the ring N² represent 1,4-cyclohexylene, 1,4-cyclohexenylene, 1,4-phenylene, 1,4-phenylene in which at least one hydrogen atom is substituted with a halogen atom or tetrahydropyran-2,5-diyl; and
Z¹, Z² and Z³ independently represent a single bond or an alkylene group having 1 to 6 carbon atoms, and at least one -CH₂- is optionally substituted with -O-.

4. The compound according to claim 3,
wherein, in Formulae (1-1) to (1-3),
Z¹ represents a single bond, -O-, -CH₂-, -CH₂O-, -OCH₂- or -CH₂CH₂-;
X¹ represents -CH₂-; and
Z² and Z³ represent a single bond.

5. The compound according to claim 1, which is represented by any one of Formulae (1-4) to (1-14):
| | | | |
|---|---|---|---|
| 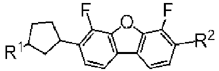 | (1-4) | 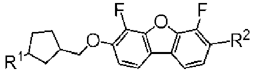 | (1-10) |
| 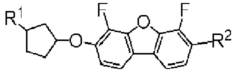 | (1-5) | 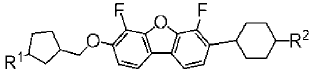 | (1-11) |
| 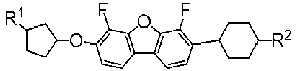 | (1-6) | 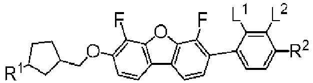 | (1-12) |
| 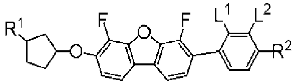 | (1-7) | 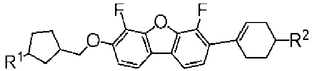 | (1-13) |
| 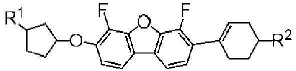 | (1-8) | 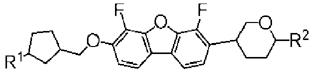 | (1-14) |
| 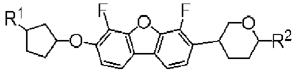 | (1-9) | | |
in Formulae (1-4) to (1-14),
R¹ and R² independently represent a hydrogen atom, an alkyl group having 1 to 10 carbon atoms, an alkoxy group having 1 to 9 carbon atoms, an alkoxyalkyl group having 2 to 9 carbon atoms, an alkenyl group having 2 to 10 carbon atoms, or an alkenyloxy group having 2 to 9 carbon atoms; and L¹ and L² independently represent a hydrogen atom or a fluorine atom.

6. The compound according to claim 5,
wherein, in Formulae (1-4) to (1-14),
R¹ represents a hydrogen atom, and R² represents an alkyl group having 1 to 10 carbon atoms, an alkoxy group having 2 to 9 carbon atoms, or an alkenyl group having 2 to 10 carbon atoms.

7. The compound according to claim 1, which is represented by Formula (1-15) or (1-16): 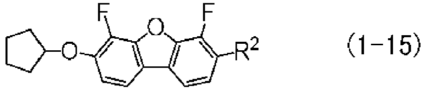 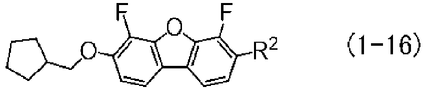 in Formulae (1-15) and (1-16), R² represents an alkoxy group having 1 to 9 carbon atoms.

8. A liquid crystal composition, **characterized in that** the liquid crystal composition comprising at least one compound selected from the group consisting of compounds represented by Formula (1) and at least one compound selected from the group consisting of compounds represented by Formulae (2) to (4): 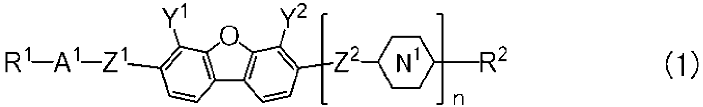 in Formula (1),
R¹ and R² independently represent a hydrogen atom or an alkyl group having 1 to 10 carbon atoms, and in the alkyl group, at least one -CH₂- is optionally substituted with -O-, -S-, -CO-, or -SiH₂-, at least one -CH₂CH₂- is optionally substituted with -CH=CH- or -C≡C-, and in these groups, at least one hydrogen atom is optionally substituted with a fluorine atom;
the ring A¹ represents 1,2-cyclopropylene, 1,3-cyclobutylene, 1,3-cyclopentylene, 1,3-cyclopentenylene, or 1,4-cyclopentenylene, and in these groups, at least one -CH₂- is optionally substituted with -O-;
the ring N¹ represents 1,4-cyclohexylene, decahydronaphthalene-2,6-diyl, or 1,4-phenylene, and in these groups, at least one -CH₂- is optionally substituted with -O-, -S-, -CO-, or -SiH₂-, at least one -CH₂CH₂- is optionally substituted with -CH=CH- or -CH=N-, and in these divalent groups, at least one hydrogen atom is optionally substituted with a fluorine atom, a chlorine atom, -C≡N, -CF₃, -CHF₂, -CH₂F, -OCF₃, -OCHF₂, or -OCH₂F;
Y¹ and Y² independently represent a hydrogen atom, a fluorine atom, a chlorine atom, -CF₃, -CHF₂, -CH₂F, -OCF₃, -OCHF₂, or -OCH₂F;
n is 0, 1 or 2; and
Z¹ and Z² independently represent a single bond or an alkylene group having 1 to 6 carbon atoms, and in the alkylene group, at least one -CH₂- is optionally substituted with -O-, -S-, or -CO-, one or two -CH₂CH₂-'s are optionally substituted with -CH=CH- or -C≡C-, and in these divalent groups, at least one hydrogen atom is optionally substituted with a fluorine atom.
; and 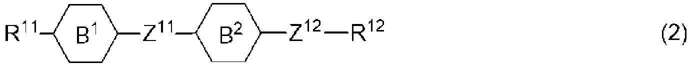 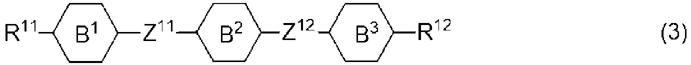 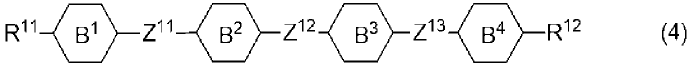
in Formulae (2) to (4),
R¹¹ and R¹² independently represent an alkyl group having 1 to 10 carbon atoms or an alkenyl group having 2 to 10 carbon atoms, and in the alkyl and alkenyl groups, at least one -CH₂- is optionally substituted with -O-, and in these groups, at least one hydrogen atom is optionally substituted with a fluorine atom;
the ring B¹, the ring B², the ring B³, and the ring B⁴ independently represent 1,4-cyclohexylene, 1,4-phenylene, 2-fluoro-1,4-phenylene, 2,5-difluoro-1,4-phenylene, or pyrimidin-2,5-diyl; and
Z¹¹, Z¹², and Z¹³ independently represent a single bond, -COO-, -CH₂CH₂-, -CH=CH-, or -C≡C-.

9. The liquid crystal composition according to claim 8, further comprising at least one compound selected from the group consisting of compounds represented by Formulae (5) to (11): 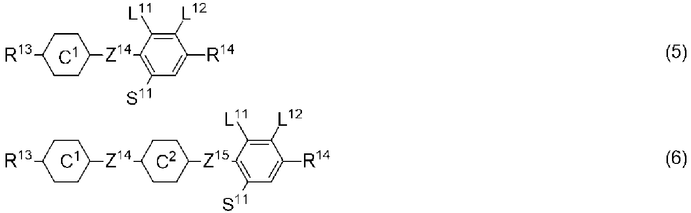 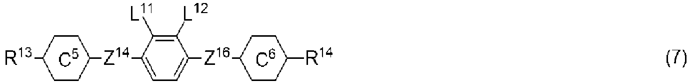 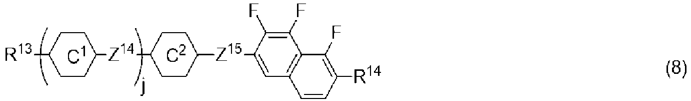 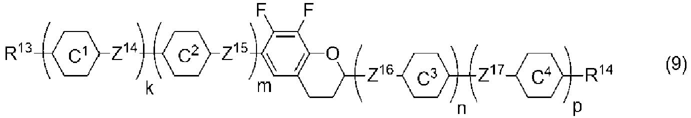 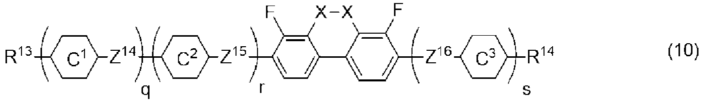 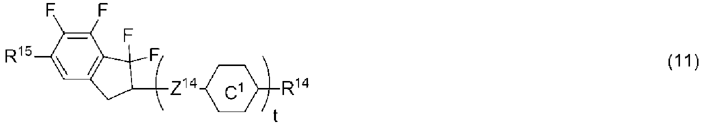 in Formulae (5) to (11),
R¹³, R¹⁴ and R¹⁵ independently represent an alkyl group having 1 to 10 carbon atoms or an alkenyl group having 2 to 10 carbon atoms, and in the alkyl and alkenyl groups, at least one -CH₂- is optionally substituted with -O-, and in these groups, at least one hydrogen atom is optionally substituted with a fluorine atom, and R¹⁵ may be a hydrogen atom or a fluorine atom;
the ring C¹, the ring C², the ring C³, and the ring C⁴ independently represent 1,4-cyclohexylene, 1,4-cyclohexenylene, 1,4-phenylene in which at least one hydrogen atom is optionally substituted with a fluorine atom, tetrahydropyran-2,5-diyl, or decahydronaphthalene-2,6-diyl;
the ring C⁵ and the ring C⁶ independently represent 1,4-cyclohexylene, 1,4-cyclohexenylene, 1,4-phenylene, tetrahydropyran-2,5-diyl, or decahydronaphthalene-2,6-diyl;
Z¹⁴, Z¹⁵, Z¹⁶, and Z¹⁷ independently represent a single bond, -COO-, -CH₂O-, -OCF₂-, -CH₂CH₂-, or -OCF₂CH₂CH₂-;
L¹¹ and L¹² independently represent a fluorine atom or a chlorine atom;
S¹¹ represents a hydrogen atom or a methyl group;
X represents -CHF- or -CF₂-; and
j, k, m, n, p, q, r, and s independently represent 0 or 1, a sum of k, m, n, and p is 1 or 2, a sum of q, r, and s is 0, 1, 2, or 3, and t is 1, 2, or 3.

10. The liquid crystal composition according to claim 8 or 9, further comprising at least one compound selected from the group consisting of compounds represented by Formulae (12) to (14): 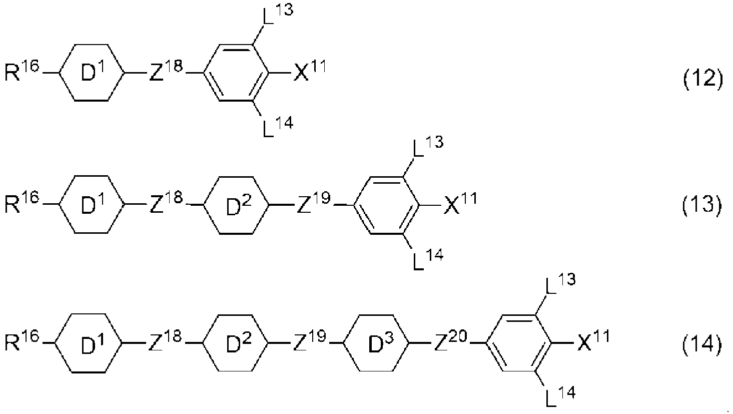 in Formulae (12) to (14),
R¹⁶ represents an alkyl group having 1 to 10 carbon atoms or an alkenyl group having 2 to 10 carbon atoms, and in the alkyl and alkenyl groups, at least one -CH₂- is optionally substituted with -O-, and in these groups, at least one hydrogen atom is optionally substituted with a fluorine atom;
X¹¹ represents a fluorine atom, a chlorine atom, -CF₃, -CHF₂, -CH₂F, -OCF₃, -OCHF₂, -OCF₂CHF₂, or -OCF₂CHFCF₃;
the ring D¹, the ring D², and the ring D³ independently represent 1,4-cyclohexylene, 1,4-phenylene in which at least one hydrogen atom is optionally substituted with a fluorine atom, tetrahydropyran-2,5-diyl, 1,3-dioxane-2,5-diyl, or pyrimidin-2,5-diyl;
Z¹⁸, Z¹⁹, and Z²⁰ independently represent a single bond, -COO-, -CH₂O-, -CF₂O-, -OCF₂-, -CH₂CH₂-, -CH=CH-, -C≡C-, or -(CH₂)₄-; and
L¹³ and L¹⁴ independently represent a hydrogen atom or a fluorine atom.

11. The liquid crystal composition according to any one of claims 8 to 10, further comprising at least one compound selected from the group consisting of compounds represented by Formula (15): 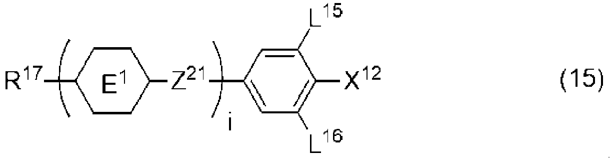 in Formula (15),
R¹⁷ represents an alkyl group having 1 to 10 carbon atoms or an alkenyl group having 2 to 10 carbon atoms, and in the alkyl and alkenyl groups, at least one -CH₂- is optionally substituted with -O-, and in these groups, at least one hydrogen atom is optionally substituted with a fluorine atom;
X¹² represents -C≡N or -C≡C-C≡N;
the ring E¹ represents 1,4-cyclohexylene, 1,4-phenylene in which at least one hydrogen atom is optionally substituted with a fluorine atom, tetrahydropyran-2,5-diyl, 1,3-dioxane-2,5-diyl, or pyrimidin-2,5-diyl;
Z²¹ represents a single bond, -COO-, -CH₂O-, -CF₂O-, -OCF₂-, -CH₂CH₂-, or -C≡C-;
L¹⁵ and L¹⁶ independently represent a hydrogen atom or a fluorine atom; and
i is 1, 2, 3, or 4.

12. A liquid crystal display element comprising the liquid crystal composition according to any one of claims 8 to 11.
